# EUROPEAN PATENT APPLICATION

(11) **EP 4 534 559 A1**
(43) Date of publication of application: **09.04.2025**
(21) Application number: 23815215.1
(22) Date of filing: 30.05.2023
(51) Int. Cl.: C07K 16/28, C07K 16/46, C12N 15/13, C12N 15/63, C12N 5/10, A61K 39/395, A61P 35/00, G01N 33/53

(54) **ANTI-CD40 ANTIBODY, ANTI-PD-L1×CD40 BISPECIFIC ANTIBODY, AND USE THEREOF**

(30) Priority: 31.05.2022 CN 202210613702; 31.05.2022 CN 202210616280; 19.05.2023 CN 202310573344
(71) Applicant: Futuregen Biopharmaceutical (Beijing) Co., Ltd., Beijing 102629 (CN)
(72) Inventor: YANG, Yaping, Beijing 102629 (CN); ZHAO, An, Beijing 102629 (CN); ZHANG, Li'na, Beijing 102629 (CN); LI, Yun, Beijing 102629 (CN); JIN, Zhaoyu, Beijing 102629 (CN); HUO, Naifan, Beijing 102629 (CN); YANG, Xi, Beijing 102629 (CN)
(74) Representative: Murgitroyd & Company
(86) International application number: PCT/CN2023/097156
(87) International publication number: WO 2023/232036

(57) **Abstract**

Disclosed are an anti-CD40 antibody, an anti-PD-L1×CD40 bispecific antibody, and use thereof. The anti-CD40 antibody comprises a heavy chain variable region and a light chain variable region. The heavy chain variable region comprises the HCDR1, HCDR2 and HCDR3 in SEQ ID NO: 64. The light chain variable region comprises the LCDR1, LCDR2 and LCDR3 in SEQ ID NO: 65. The novel anti-CD40 agonistic antibody of the present invention can effectively regulate and control the activation of DC cells, has a stronger T cell activation effect while having low toxic and side effects, and is suitable for immunotherapy of tumors. The bispecific antibody of the present invention can maximize the anti-tumor drug effect, improve the selectivity of CD40 activation by means of PD-L1-dependent CD40 activation, and lower the toxic and side effects of the CD40 agonistic antibody.

## Description

The present application claims the right of the priority of Chinese patent application 2022106137021 filed on May 31, 2022, Chinese patent application 2022106162803 filed on May 31, 2022, and Chinese patent application 2023105733440 filed on May 19, 2023. The contents of the above Chinese patent applications are incorporated herein by reference in their entirety.

### TECHNICAL FIELD

The present disclosure belongs to the field of biotechnology, and specifically relates to an anti-CD40 antibody, an anti-PD-L1×CD40 bispecific antibody, and a use thereof.

### BACKGROUND

In recent years, the field of tumor therapy has developed rapidly, evolving from classic treatment methods based on surgery, radiotherapy, and chemotherapy to more advanced treatment options such as targeted therapy and immunotherapy. Notably, tumor immunotherapy, represented by PD-L1/PD-1 inhibitors, has significantly prolonged the survival of cancer patients for many tumor indications and has also been positioned as first-line therapy for many tumor indications (Nat Rev Immunol. 2020 Nov; 20(11): 651-668). As the most promising approach for curing tumors, tumor immunotherapy has become a central focus in the field of research and development of novel anti-tumor drugs.

One of the key challenges in tumor immunology is to increase the quantity and quality of infiltrating T cells in cold tumors. In many cancer patients, insufficient T cell activation contributes to the lack of T cells in the tumor microenvironment. Antigen-presenting cells, especially DC cells, which loaded with tumor antigens, play a decisive role in activating T cell responses. CD40, a type I transmembrane protein, is a member of the tumor necrosis factor receptor (TNFR) superfamily and is widely expressed in antigen-presenting cells (DC cells, macrophages, and B cells), platelets, some non-hematopoietic cells, and various types of tumor cells. It plays an important role in both innate and adaptive immunity, and plays a key role in the activation of DC cells (Expert Opin Biol Ther. 2021 Dec; 21(12): 1635-1646; Annu Rev Med. 2020 Jan 27; 71: 47-58; Expert Rev Anticancer Ther. 2017 Feb; 17(2): 175-186; Hum Vaccin Immunother. 2020; 16(2): 377-387). Upon activation of CD40, by upregulating co-stimulatory molecules and MHC on DC cells and inducing pro-inflammatory cytokines, DC cells are activated and empowered to promote the activation of anti-tumor-specific T cells, which have the potential to eliminate tumor cells completely.

In multiple tumor models in mice, CD40 agonistic antibodies can achieve T cell activation and exhibit robust anti-tumor efficacy (Science. 2011 Aug 19; 333(6045): 1030-4; Clin Cancer Res. 2015 Mar 1; 21(5): 1115-26; Int J Cancer. 2019 Sep 1; 145(5): 1189-1199; J Immunother Cancer. 2020 May; 8(1): e000624.). CD40 agonistic antibodies can also exert synergistic anti-tumor effects with immune checkpoint antibodies, such as PD-L1/PD-1, through a mechanism of cross-talk between DC cells and T cells, where DC cells stimulate the activation of tumor-specific T cells by upregulating co-stimulatory molecules and secreting IL-12, while activated T cells, in turn, activate DC cells by secreting IFN-γ. CD40 antibodies and PD-L1/PD-1 antibodies act on different aspects of cross-talk, enhancing positive feedback and maximizing anti-tumor efficacy (Cancer Res. 2016 Nov 1; 76(21): 6266-6277; Immunity. 2018 Dec 18; 49(6): 1148-1161.e7).

Currently, several biopharmaceutical companies are developing agonistic monoclonal antibodies against CD40, with relevant patents such as WO2003040170, WO2014070934A1, US20180066053, US20140348836, WO2020108611, and CN111763259, and many antibodies have entered clinical trials. Selicrelumab, a CD40 monoclonal antibody developed by Pfizer, produced an objective partial response (PR) in 4 of 15 patients with advanced melanoma in a first-in-human single-dose study. One of these patients subsequently received a repeat dose of Selicrelumab over one year and remained in complete remission (CR) after 15 years. However, Selicrelumab showed poor efficacy in subsequent clinical trials, while other CD40 monoclonal antibodies such as APX005M and SEA-CD40 showed very low objective response rates (ORR). CD40 agonistic antibodies have shown many adverse effects in clinical practice, including cytokine release syndrome (CRS), liver injury, and thrombocytopenia, with maximum tolerated doses (MTD) of 0.2, 0.3, and 0.06 mg/kg for Selicrelumab, APX005M, and SEA-CD40, respectively (J Clin Oncol. 2007 Mar 1; 25(7): 876-83; Cancer Biol Ther. 2010 Nov 15; 10(10): 983-93; Lancet Oncol. 2021 Jan; 22(1): 118-131; Oncol Lett. 2020 Nov; 20(5): 176; Annu Rev Med. 2020 Jan 27; 71: 47-58). Therefore, there is an urgent need for a CD40-targeting antibody with high safety and good efficacy in the art.

### CONTENT OF THE PRESENT INVENTION

In order to solve the above technical problems, in view of the current status of research and development of CD40 antibodies and/or PD-L1 antibodies, the present disclosure provides an anti-CD40 antibody and an anti-PD-L1×CD40 bispecific antibody. The novel anti-CD40 agonistic antibody of the present disclosure can effectively regulate the activation of DC cells, with stronger T cell activation but with low toxic and side effects, making it suitable for tumor immunotherapy. The anti-PD-L1×CD40 bispecific antibody of the present disclosure acts in a positive feedback loop involving cross-talk between DC cells and T cells by simultaneously activating CD40 and blocking PD-L1/PD-1, thereby maximizing anti-tumor efficacy; it also improves the selectivity of CD40 activation by means of PD-L1-dependent CD40 activation, thereby reducing the toxic and side effects of the CD40 agonistic antibody.

A first aspect of the present disclosure provides an anti-CD40 antibody comprising a heavy chain variable region and a light chain variable region, wherein the heavy chain variable region comprises HCDR1, HCDR2, and HCDR3 of SEQ ID NO: 64, and the light chain variable region comprises LCDR1, LCDR2, and LCDR3 of SEQ ID NO: 65.

The term "CD40" includes any variant or isoform of CD40 that is naturally expressed by a cell. The antibody of the present disclosure can specifically bind to human CD40 and monkey CD40 (*e.g.,* cynomolgus monkey). Alternatively, the antibody may also be specific for human CD40 and may not exhibit cross-reactivity with other species. CD40 or any variant or isoform thereof can be isolated from cells or tissues in which they are naturally expressed, or produced by recombinant techniques using common techniques in the art and those described herein.

In some embodiments, the light chain variable region comprises LCDR1, LCDR2, and LCDR3 of SEQ ID NO: 38, and the heavy chain variable region comprises HCDR1, HCDR2, and HCDR3 of SEQ ID NO: 40;
or, the light chain variable region comprises LCDR1, LCDR2, and LCDR3 of SEQ ID NO: 38, and the heavy chain variable region comprises HCDR1, HCDR2, and HCDR3 of SEQ ID NO: 39;
or, the light chain variable region comprises LCDR1, LCDR2, and LCDR3 of SEQ ID NO: 30, and the heavy chain variable region comprises HCDR1, HCDR2, and HCDR3 of SEQ ID NO: 31;
or, the light chain variable region comprises LCDR1, LCDR2, and LCDR3 of SEQ ID NO: 32, and the heavy chain variable region comprises HCDR1, HCDR2, and HCDR3 of SEQ ID NO: 33;
or, the light chain variable region comprises LCDR1, LCDR2, and LCDR3 of SEQ ID NO: 32, and the heavy chain variable region comprises HCDR1, HCDR2, and HCDR3 of SEQ ID NO: 34;
or, the light chain variable region comprises LCDR1, LCDR2, and LCDR3 of SEQ ID NO: 35, and the heavy chain variable region comprises HCDR1, HCDR2, and HCDR3 of SEQ ID NO: 36;
or, the light chain variable region comprises LCDR1, LCDR2, and LCDR3 of SEQ ID NO: 35, and the heavy chain variable region comprises HCDR1, HCDR2, and HCDR3 of SEQ ID NO: 37;
or, the light chain variable region comprises LCDR1, LCDR2, and LCDR3 of SEQ ID NO: 41, and the heavy chain variable region comprises HCDR1, HCDR2, and HCDR3 of SEQ ID NO: 42;
or, the light chain variable region comprises LCDR1, LCDR2, and LCDR3 of SEQ ID NO: 41, and the heavy chain variable region comprises HCDR1, HCDR2, and HCDR3 of SEQ ID NO: 43.

In some embodiments, the CDR is defined according to the Kabat, IMGT, Chothia, AbM, or Contact numbering system, and in some specific embodiments, the CDR is determined according to the Kabat numbering rule.

In some embodiments, in the antibody according to the first aspect of the present disclosure, the LCDR1 comprises an amino acid sequence as shown in SEQ ID NO: 1; the LCDR2 comprises an amino acid sequence as shown in X₅X₆SX₇X₈X₉S, wherein X₅ is Y or A; X₆ is T or A; X₇ is S, R, or T; X₈ is L or R; X₉ is Q or D; the LCDR3 comprises an amino acid sequence as shown in SEQ ID NO: 3; the HCDR1 comprises an amino acid sequence as shown in SEQ ID NO: 4; the HCDR2 comprises an amino acid sequence as shown in SEQ ID NO: 5; and the HCDR3 comprises an amino acid sequence as shown in SEQ ID NO: 6.

In a preferred embodiment of the present disclosure, the amino acid sequence of the LCDR1 is as shown in SEQ ID NO: 1; the amino acid sequence of the LCDR2 is as shown in X₅X₆SX₇X₈X₉S, wherein X₅ is Y or A; X₆ is T or A; X₇ is S, R, or T; X₈ is L or R; X₉ is Q or D; the amino acid sequence of the LCDR3 is as shown in SEQ ID NO: 3; the amino acid sequence of the HCDR1 is as shown in SEQ ID NO: 4; the amino acid sequence of the HCDR2 is as shown in SEQ ID NO: 5; and the amino acid sequence of the HCDR3 is as shown in SEQ ID NO: 6.

In the antibody according to the first aspect of the present disclosure, the LCDR1 comprises an amino acid sequence as shown in SEQ ID NO: 9, SEQ ID NO: 8, SEQ ID NO: 7, or SEQ ID NO: 10; the LCDR2 comprises an amino acid sequence as shown in SEQ ID NO: 14, SEQ ID NO: 12, SEQ ID NO: 13, SEQ ID NO: 11, or SEQ ID NO: 15; the LCDR3 comprises an amino acid sequence as shown in SEQ ID NO: 19, SEQ ID NO: 17, SEQ ID NO: 18, SEQ ID NO: 16, or SEQ ID NO: 20; the HCDR1 comprises an amino acid sequence as shown in SEQ ID NO: 21 or SEQ ID NO: 22; the HCDR2 comprises an amino acid sequence as shown in SEQ ID NO: 23 or SEQ ID NO: 24; and the HCDR3 comprises an amino acid sequence as shown in SEQ ID NO: 28, SEQ ID NO: 26, SEQ ID NO: 27, SEQ ID NO: 25, or SEQ ID NO: 29.

In a preferred embodiment of the present disclosure, the antibody comprises the amino acid sequence of the LCDR1 as shown in SEQ ID NO: 9, SEQ ID NO: 8, SEQ ID NO: 7, or SEQ ID NO: 10, the amino acid sequence of the LCDR2 as shown in SEQ ID NO: 14, SEQ ID NO: 12, SEQ ID NO: 13, SEQ ID NO: 11, or SEQ ID NO: 15, the amino acid sequence of the LCDR3 as shown in SEQ ID NO: 19, SEQ ID NO: 17, SEQ ID NO: 18, SEQ ID NO: 16, or SEQ ID NO: 20, the amino acid sequence of the HCDR1 as shown in SEQ ID NO: 21 or SEQ ID NO: 22, the amino acid sequence of the HCDR2 as shown in SEQ ID NO: 23 or SEQ ID NO: 24, and the amino acid sequence of the HCDR3 as shown in SEQ ID NO: 28, SEQ ID NO: 26, SEQ ID NO: 27, SEQ ID NO: 25, or SEQ ID NO: 29.

In a preferred embodiment of the present disclosure, the amino acid sequence of the LCDR1 is as shown in SEQ ID NO: 9, SEQ ID NO: 8, SEQ ID NO: 7, or SEQ ID NO: 10; the amino acid sequence of the LCDR2 is as shown in SEQ ID NO: 14, SEQ ID NO: 12, SEQ ID NO: 13, SEQ ID NO: 11, or SEQ ID NO: 15; the amino acid sequence of the LCDR3 is as shown in SEQ ID NO: 19, SEQ ID NO: 17, SEQ ID NO: 18, SEQ ID NO: 16, or SEQ ID NO: 20; the amino acid sequence of the HCDR1 is as shown in SEQ ID NO: 21 or SEQ ID NO: 22; the amino acid sequence of the HCDR2 is as shown in SEQ ID NO: 23 or SEQ ID NO: 24; and the amino acid sequence of the HCDR3 is as shown in SEQ ID NO: 28, SEQ ID NO: 26, SEQ ID NO: 27, SEQ ID NO: 25, or SEQ ID NO: 29.

In the antibody according to the first aspect of the present disclosure, in a preferred embodiment of the present disclosure:
A) the LCDR1 comprises the amino acid sequence as shown in SEQ ID NO: 9; the LCDR2 comprises the amino acid sequence as shown in SEQ ID NO: 14; the LCDR3 comprises the amino acid sequence as shown in SEQ ID NO: 19; the HCDR1 comprises the amino acid sequence as shown in SEQ ID NO: 21; the HCDR2 comprises the amino acid sequence as shown in SEQ ID NO: 23; and the HCDR3 comprises the amino acid sequence as shown in SEQ ID NO: 28;
B) the LCDR1 comprises the amino acid sequence as shown in SEQ ID NO: 9; the LCDR2 comprises the amino acid sequence as shown in SEQ ID NO: 14; the LCDR3 comprises the amino acid sequence as shown in SEQ ID NO: 19; the HCDR1 comprises the amino acid sequence as shown in SEQ ID NO: 21; the HCDR2 comprises the amino acid sequence as shown in SEQ ID NO: 24; and the HCDR3 comprises the amino acid sequence as shown in SEQ ID NO: 28;
C) the LCDR1 comprises the amino acid sequence as shown in SEQ ID NO: 7; the LCDR2 comprises the amino acid sequence as shown in SEQ ID NO: 11; the LCDR3 comprises the amino acid sequence as shown in SEQ ID NO: 16; the HCDR1 comprises the amino acid sequence as shown in SEQ ID NO: 21; the HCDR2 comprises the amino acid sequence as shown in SEQ ID NO: 23; and the HCDR3 comprises the amino acid sequence as shown in SEQ ID NO: 25;
D) the LCDR1 comprises the amino acid sequence as shown in SEQ ID NO: 7; the LCDR2 comprises the amino acid sequence as shown in SEQ ID NO: 12; the LCDR3 comprises the amino acid sequence as shown in SEQ ID NO: 17; the HCDR1 comprises the amino acid sequence as shown in SEQ ID NO: 22; the HCDR2 comprises the amino acid sequence as shown in SEQ ID NO: 24; and the HCDR3 comprises the amino acid sequence as shown in SEQ ID NO: 26;
E) the LCDR1 comprises the amino acid sequence as shown in SEQ ID NO: 7; the LCDR2 comprises the amino acid sequence as shown in SEQ ID NO: 12; the LCDR3 comprises the amino acid sequence as shown in SEQ ID NO: 17; the HCDR1 comprises the amino acid sequence as shown in SEQ ID NO: 22; the HCDR2 comprises the amino acid sequence as shown in SEQ ID NO: 23; and the HCDR3 comprises the amino acid sequence as shown in SEQ ID NO: 26;
F) the LCDR1 comprises the amino acid sequence as shown in SEQ ID NO: 8; the LCDR2 comprises the amino acid sequence as shown in SEQ ID NO: 13; the LCDR3 comprises the amino acid sequence as shown in SEQ ID NO: 18; the HCDR1 comprises the amino acid sequence as shown in SEQ ID NO: 22; the HCDR2 comprises the amino acid sequence as shown in SEQ ID NO: 24; and the HCDR3 comprises the amino acid sequence as shown in SEQ ID NO: 27;
G) the LCDR1 comprises the amino acid sequence as shown in SEQ ID NO: 8; the LCDR2 comprises the amino acid sequence as shown in SEQ ID NO: 13; the LCDR3 comprises the amino acid sequence as shown in SEQ ID NO: 18; the HCDR1 comprises the amino acid sequence as shown in SEQ ID NO: 22; the HCDR2 comprises the amino acid sequence as shown in SEQ ID NO: 23; and the HCDR3 comprises the amino acid sequence as shown in SEQ ID NO: 27;
H) the LCDR1 comprises the amino acid sequence as shown in SEQ ID NO: 10; the LCDR2 comprises the amino acid sequence as shown in SEQ ID NO: 15; the LCDR3 comprises the amino acid sequence as shown in SEQ ID NO: 20; the HCDR1 comprises the amino acid sequence as shown in SEQ ID NO: 21; the HCDR2 comprises the amino acid sequence as shown in SEQ ID NO: 24; and the HCDR3 comprises the amino acid sequence as shown in SEQ ID NO: 29; or
I) the LCDR1 comprises the amino acid sequence as shown in SEQ ID NO: 10; the LCDR2 comprises the amino acid sequence as shown in SEQ ID NO: 15; the LCDR3 comprises the amino acid sequence as shown in SEQ ID NO: 20; the HCDR1 comprises the amino acid sequence as shown in SEQ ID NO: 21; the HCDR2 comprises the amino acid sequence as shown in SEQ ID NO: 23; and the HCDR3 comprises the amino acid sequence as shown in SEQ ID NO: 29.

In a more preferred embodiment of the present disclosure:
A) the light chain variable region comprises LCDR1, LCDR2, and LCDR3 as shown in SEQ ID NO: 9, SEQ ID NO: 14, and SEQ ID NO: 19, respectively, and the heavy chain variable region comprises HCDR1, HCDR2, and HCDR3 as shown in SEQ ID NO: 21, SEQ ID NO: 23, and SEQ ID NO: 28, respectively;
B) the light chain variable region comprises LCDR1, LCDR2, and LCDR3 as shown in SEQ ID NO: 9, SEQ ID NO: 14, and SEQ ID NO: 19, respectively, and the heavy chain variable region comprises HCDR1, HCDR2, and HCDR3 as shown in SEQ ID NO: 21, SEQ ID NO: 24, and SEQ ID NO: 28, respectively;
C) the light chain variable region comprises LCDR1, LCDR2, and LCDR3 as shown in SEQ ID NO: 7, SEQ ID NO: 11, and SEQ ID NO: 16, respectively, and the heavy chain variable region comprises HCDR1, HCDR2, and HCDR3 as shown in SEQ ID NO: 21, SEQ ID NO: 23, and SEQ ID NO: 25, respectively;
D) the light chain variable region comprises LCDR1, LCDR2, and LCDR3 as shown in SEQ ID NO: 7, SEQ ID NO: 12, and SEQ ID NO: 17, respectively, and the heavy chain variable region comprises HCDR1, HCDR2, and HCDR3 as shown in SEQ ID NO: 22, SEQ ID NO: 24, and SEQ ID NO: 26, respectively;
E) the light chain variable region comprises LCDR1, LCDR2, and LCDR3 as shown in SEQ ID NO: 7, SEQ ID NO: 12, and SEQ ID NO: 17, respectively, and the heavy chain variable region comprises HCDR1, HCDR2, and HCDR3 as shown in SEQ ID NO: 22, SEQ ID NO: 23, and SEQ ID NO: 26, respectively;
F) the light chain variable region comprises LCDR1, LCDR2, and LCDR3 as shown in SEQ ID NO: 8, SEQ ID NO: 13, and SEQ ID NO: 18, respectively, and the heavy chain variable region comprises HCDR1, HCDR2, and HCDR3 as shown in SEQ ID NO: 22, SEQ ID NO: 24, and SEQ ID NO: 27, respectively;
G) the light chain variable region comprises LCDR1, LCDR2, and LCDR3 as shown in SEQ ID NO: 8, SEQ ID NO: 13, and SEQ ID NO: 18, respectively, and the heavy chain variable region comprises HCDR1, HCDR2, and HCDR3 as shown in SEQ ID NO: 22, SEQ ID NO: 23, and SEQ ID NO: 27, respectively;
H) the light chain variable region comprises LCDR1, LCDR2, and LCDR3 as shown in SEQ ID NO: 10, SEQ ID NO: 15, and SEQ ID NO: 20, respectively, and the heavy chain variable region comprises HCDR1, HCDR2, and HCDR3 as shown in SEQ ID NO: 21, SEQ ID NO: 24, and SEQ ID NO: 29, respectively; or
I) the light chain variable region comprises LCDR1, LCDR2, and LCDR3 as shown in SEQ ID NO: 10, SEQ ID NO: 15, and SEQ ID NO: 20, respectively, and the heavy chain variable region comprises HCDR1, HCDR2, and HCDR3 as shown in SEQ ID NO: 21, SEQ ID NO: 23, and SEQ ID NO: 29, respectively.

In a preferred embodiment of the present disclosure:
In some embodiments, in the antibody, the amino acid sequence of the LCDR1 is as shown in SEQ ID NO: 9; the amino acid sequence of the LCDR2 is as shown in SEQ ID NO: 14; the amino acid sequence of the LCDR3 is as shown in SEQ ID NO: 19; the amino acid sequence of the HCDR1 is as shown in SEQ ID NO: 21; the amino acid sequence of the HCDR2 is as shown in SEQ ID NO: 23; and the amino acid sequence of the HCDR3 is as shown in SEQ ID NO: 28.

In some embodiments, in the antibody, the amino acid sequence of the LCDR1 is as shown in SEQ ID NO: 9; the amino acid sequence of the LCDR2 is as shown in SEQ ID NO: 14; the amino acid sequence of the LCDR3 is as shown in SEQ ID NO: 19; the amino acid sequence of the HCDR1 is as shown in SEQ ID NO: 21; the amino acid sequence of the HCDR2 is as shown in SEQ ID NO: 24; and the amino acid sequence of the HCDR3 is as shown in SEQ ID NO: 28.

In some embodiments, in the antibody, the amino acid sequence of the LCDR1 is as shown in SEQ ID NO: 7; the amino acid sequence of the LCDR2 is as shown in SEQ ID NO: 11; the amino acid sequence of the LCDR3 is as shown in SEQ ID NO: 16; the amino acid sequence of the HCDR1 is as shown in SEQ ID NO: 21; the amino acid sequence of the HCDR2 is as shown in SEQ ID NO: 23; and the amino acid sequence of the HCDR3 is as shown in SEQ ID NO: 25.

In some embodiments, in the antibody, the amino acid sequence of the LCDR1 is as shown in SEQ ID NO: 7; the amino acid sequence of the LCDR2 is as shown in SEQ ID NO: 12; the amino acid sequence of the LCDR3 is as shown in SEQ ID NO: 17; the amino acid sequence of the HCDR1 is as shown in SEQ ID NO: 22; the amino acid sequence of the HCDR2 is as shown in SEQ ID NO: 24; and the amino acid sequence of the HCDR3 is as shown in SEQ ID NO: 26.

In some embodiments, in the antibody, the amino acid sequence of the LCDR1 is as shown in SEQ ID NO: 7; the amino acid sequence of the LCDR2 is as shown in SEQ ID NO: 12; the amino acid sequence of the LCDR3 is as shown in SEQ ID NO: 17; the amino acid sequence of the HCDR1 is as shown in SEQ ID NO: 22; the amino acid sequence of the HCDR2 is as shown in SEQ ID NO: 23; and the amino acid sequence of the HCDR3 is as shown in SEQ ID NO: 26.

In some embodiments, in the antibody, the amino acid sequence of the LCDR1 is as shown in SEQ ID NO: 8; the amino acid sequence of the LCDR2 is as shown in SEQ ID NO: 13; the amino acid sequence of the LCDR3 is as shown in SEQ ID NO: 18; the amino acid sequence of the HCDR1 is as shown in SEQ ID NO: 22; the amino acid sequence of the HCDR2 is as shown in SEQ ID NO: 24; and the amino acid sequence of the HCDR3 is as shown in SEQ ID NO: 27.

In some embodiments, in the antibody, the amino acid sequence of the LCDR1 is as shown in SEQ ID NO: 8; the amino acid sequence of the LCDR2 is as shown in SEQ ID NO: 13; the amino acid sequence of the LCDR3 is as shown in SEQ ID NO: 18; the amino acid sequence of the HCDR1 is as shown in SEQ ID NO: 22; the amino acid sequence of the HCDR2 is as shown in SEQ ID NO: 23; and the amino acid sequence of the HCDR3 is as shown in SEQ ID NO: 27.

In some embodiments, in the antibody, the amino acid sequence of the LCDR1 is as shown in SEQ ID NO: 10; the amino acid sequence of the LCDR2 is as shown in SEQ ID NO: 15; the amino acid sequence of the LCDR3 is as shown in SEQ ID NO: 20; the amino acid sequence of the HCDR1 is as shown in SEQ ID NO: 21; the amino acid sequence of the HCDR2 is as shown in SEQ ID NO: 24; and the amino acid sequence of the HCDR3 is as shown in SEQ ID NO: 29.

In some embodiments, in the antibody, the amino acid sequence of the LCDR1 is as shown in SEQ ID NO: 10; the amino acid sequence of the LCDR2 is as shown in SEQ ID NO: 15; the amino acid sequence of the LCDR3 is as shown in SEQ ID NO: 20; the amino acid sequence of the HCDR1 is as shown in SEQ ID NO: 21; the amino acid sequence of the HCDR2 is as shown in SEQ ID NO: 23; and the amino acid sequence of the HCDR3 is as shown in SEQ ID NO: 29.

In some of the above embodiments, the amino acid sequences of the listed CDRs are determined according to the Kabat definition rule. However, it is well known to those skilled in the art that the CDR of an antibody can be defined by a variety of methods in the art, such as Chothia based on the three-dimensional structure of the antibody and the topology of the CDR loops (Chothia et al. (1989), Nature, 342: 877-883; Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures. It should be understood by those skilled in the art that, unless otherwise specified, the terms "CDR" and "complementary determining region" for a given antibody or region thereof (*e.g.,* variable region) should be interpreted to encompass complementary determining regions defined by any one of the known schemes as described by the present disclosure. Various numbering systems and their corresponding CDRs are well known to those skilled in the art, as shown in Table 1:

**Table 1: Antibody CDR definition method**

| | Kabat | AbM | Chothia | Contact | IMGT |
|---|---|---|---|---|---|
| LCDR1 | L24-L34 | L24-L34 | L24-L34 | L30-L36 | L27-L32 |
| LCDR2 | L50-L56 | L50-L56 | L50-L56 | L46-L55 | L50-L52 |
| LCDR3 | L89-L97 | L89-L97 | L89-L97 | L89-L96 | L89-L96 |
| HCDR1 | H31-H35 | H26-H35 | H26-H32 | H30-H35 | H26-H35 |
| HCDR2 | H50-H65 | H50-H58 | H52-H56 | H47-H58 | H51-H57 |
| HCDR3 | H95-H102 | H95-H102 | H95-H102 | H93-H101 | H93-HI02 |

Note: In Table 1, Laa-Lbb refers to the amino acid sequence from position aa to position bb starting from the N-terminus of the light chain of the antibody according to the corresponding numbering rule; Haa-Hbb refers to the amino acid sequence from position aa to position bb starting from the N-terminus of the heavy chain of the antibody according to the corresponding numbering rule. For example, L24-L34 in the second row and second column of Table 1 refers to the amino acid sequence from residue 24 to residue 34 starting from the N-terminus of the light chain variable region of the antibody according to the Kabat numbering rule; and so on.

In the antibody according to the first aspect of the present disclosure, the framework region of the light chain variable region is a human framework region, and the framework region of the heavy chain variable region is a human framework region.

In the antibody according to the first aspect of the present disclosure, in a preferred embodiment of the present disclosure:
a) the light chain variable region comprises an amino acid sequence having at least 90%, at least 95%, or at least 99% sequence identity with SEQ ID NO: 38, and the heavy chain variable region comprises an amino acid sequence having at least 90%, at least 95%, or at least 99% sequence identity with SEQ ID NO: 40 or SEQ ID NO: 39;
b) the light chain variable region comprises an amino acid sequence having at least 90%, at least 95%, or at least 99% sequence identity with SEQ ID NO: 30, and the heavy chain variable region comprises an amino acid sequence having at least 90%, at least 95%, or at least 99% sequence identity with SEQ ID NO: 31;
c) the light chain variable region comprises an amino acid sequence having at least 90%, at least 95%, or at least 99% sequence identity with SEQ ID NO: 32, and the heavy chain variable region comprises an amino acid sequence having at least 90%, at least 95%, or at least 99% sequence identity with SEQ ID NO: 33 or SEQ ID NO: 34;
d) the light chain variable region comprises an amino acid sequence having at least 90%, at least 95%, or at least 99% sequence identity with SEQ ID NO: 35, and the heavy chain variable region comprises an amino acid sequence having at least 90%, at least 95%, or at least 99% sequence identity with SEQ ID NO: 36 or SEQ ID NO: 37; or
e) the light chain variable region comprises an amino acid sequence having at least 90%, at least 95%, or at least 99% sequence identity with SEQ ID NO: 41, and the heavy chain variable region comprises an amino acid sequence having at least 90%, at least 95%, or at least 99% sequence identity with SEQ ID NO: 42 or SEQ ID NO: 43.

In a preferred embodiment of the present disclosure, the variable region comprising the amino acid sequence having at least 90%, at least 95%, or at least 99% sequence identity with an original sequence maintains the function of binding to an antigen (*e.g.,* human CD40) that is identical to the original sequence.

The calculation of sequence identity between sequences is conducted as follows. To determine the percentage of identity between two amino acid sequences, the sequences are aligned for optimal comparison purposes (*e.g.,* gaps may be introduced in the first and second amino acid sequences for optimal alignment, or non-homologous sequences may be discarded for comparison purposes).In a preferred embodiment, for comparison purposes, the aligned length of the reference sequence is at least 30%, preferably at least 40%, more preferably at least 50% or 60%, and even more preferably at least 70%, 80%, 90%, or 100% of the length of the reference sequence. The amino acid residues at corresponding amino acid positions are then compared. When a position in a first sequence is occupied by the same amino acid residue at the corresponding position in a second sequence, the molecules are identical at the position. A mathematical algorithm can be used to perform sequence comparison and calculation of the percentage of identity between two sequences. In a preferred embodiment, the percentage of identity between two amino acid sequences is determined with the Needlema and Wunsch ((1970) J. Mol. Biol., 48: 444-453) algorithm (available at http://www.gcg.com) which has been integrated into the GAP program of the GCG software package, using the Blossom 62 matrix or PAM250 matrix with a gap weight of 16, 14, 12, 10, 8, 6, or 4 and a length weight of 1, 2, 3, 4, 5, or 6. A particularly preferred parameter set (and one that should be used unless otherwise stated) is a Blossom 62 scoring matrix with a gap penalty of 12, a gap extension penalty of 4, and a frameshift gap penalty of 5. The percentage of identity between two amino acid sequences can also be determined with the PAM120 weighted remainder table with a gap length penalty of 12 and a gap penalty of 4, using the E. Meyers and W. Miller algorithm ((1989) CABIOS, 4: 11-17) which has been incorporated into the ALIGN program (version 2.0). Additionally or alternatively, the protein sequences described in the present disclosure may be further used as "query sequences" to perform searches against public databases to, for example, identify sequences of other family members or related sequences.

In the antibody according to the first aspect of the present disclosure, in a more preferred embodiment of the present disclosure:
a) the light chain variable region comprises an amino acid sequence as shown in SEQ ID NO: 38, and the heavy chain variable region comprises an amino acid sequence as shown in SEQ ID NO: 40 or SEQ ID NO: 39;
b) the light chain variable region comprises an amino acid sequence as shown in SEQ ID NO: 30, and the heavy chain variable region comprises an amino acid sequence as shown in SEQ ID NO: 31;
c) the light chain variable region comprises an amino acid sequence as shown in SEQ ID NO: 32, and the heavy chain variable region comprises an amino acid sequence as shown in SEQ ID NO: 33 or SEQ ID NO: 34;
d) the light chain variable region comprises an amino acid sequence as shown in SEQ ID NO: 35, and the heavy chain variable region comprises an amino acid sequence as shown in SEQ ID NO: 36 or SEQ ID NO: 37; or
e) the light chain variable region comprises an amino acid sequence as shown in SEQ ID NO: 41, and the heavy chain variable region comprises an amino acid sequence as shown in SEQ ID NO: 42 or SEQ ID NO: 43.

In the antibody according to the first aspect of the present disclosure, in a further preferred embodiment of the present disclosure:

In some embodiments, the antibody comprises a light chain variable region as shown in SEQ ID NO: 38 and a heavy chain variable region as shown in SEQ ID NO: 40.

In some embodiments, the antibody comprises a light chain variable region as shown in SEQ ID NO: 38 and a heavy chain variable region as shown in SEQ ID NO: 39.

In some embodiments, the antibody comprises a light chain variable region as shown in SEQ ID NO: 30 and a heavy chain variable region as shown in SEQ ID NO: 31.

In some embodiments, the antibody comprises a light chain variable region as shown in SEQ ID NO: 32 and a heavy chain variable region as shown in SEQ ID NO: 33.

In some embodiments, the antibody comprises a light chain variable region as shown in SEQ ID NO: 32 and a heavy chain variable region as shown in SEQ ID NO: 34.

In some embodiments, the antibody comprises a light chain variable region as shown in SEQ ID NO: 35 and a heavy chain variable region as shown in SEQ ID NO: 36.

In some embodiments, the antibody comprises a light chain variable region as shown in SEQ ID NO: 35 and a heavy chain variable region as shown in SEQ ID NO: 37.

In some embodiments, the antibody comprises a light chain variable region as shown in SEQ ID NO: 41 and a heavy chain variable region as shown in SEQ ID NO: 42.

In some embodiments, the antibody comprises a light chain variable region as shown in SEQ ID NO: 41 and a heavy chain variable region as shown in SEQ ID NO: 43.

In a preferred embodiment of the present disclosure:

In some embodiments, in the antibody, the amino acid sequence of the light chain variable region is as shown in SEQ ID NO: 38, and the amino acid sequence of the heavy chain variable region is as shown in SEQ ID NO: 40 or SEQ ID NO: 39.

In some embodiments, in the antibody, the amino acid sequence of the light chain variable region is as shown in SEQ ID NO: 30, and the amino acid sequence of the heavy chain variable region is as shown in SEQ ID NO: 31.

In some embodiments, in the antibody, the amino acid sequence of the light chain variable region is as shown in SEQ ID NO: 32, and the amino acid sequence of the heavy chain variable region is as shown in SEQ ID NO: 33 or SEQ ID NO: 34.

In some embodiments, in the antibody, the amino acid sequence of the light chain variable region is as shown in SEQ ID NO: 35, and the amino acid sequence of the heavy chain variable region is as shown in SEQ ID NO: 36 or SEQ ID NO: 37.

In some embodiments, in the antibody, the amino acid sequence of the light chain variable region is as shown in SEQ ID NO: 41, and the amino acid sequence of the heavy chain variable region is as shown in SEQ ID NO: 42 or SEQ ID NO: 43.

In a more preferred embodiment of the present disclosure:

In some embodiments, in the antibody, the amino acid sequence of the light chain variable region is as shown in SEQ ID NO: 38, and the amino acid sequence of the heavy chain variable region is as shown in SEQ ID NO: 40.

In some embodiments, in the antibody, the amino acid sequence of the light chain variable region is as shown in SEQ ID NO: 38, and the amino acid sequence of the heavy chain variable region is as shown in SEQ ID NO: 39.

In some embodiments, in the antibody, the amino acid sequence of the light chain variable region is as shown in SEQ ID NO: 30, and the amino acid sequence of the heavy chain variable region is as shown in SEQ ID NO: 31.

In some embodiments, in the antibody, the amino acid sequence of the light chain variable region is as shown in SEQ ID NO: 32, and the amino acid sequence of the heavy chain variable region is as shown in SEQ ID NO: 33.

In some embodiments, in the antibody, the amino acid sequence of the light chain variable region is as shown in SEQ ID NO: 32, and the amino acid sequence of the heavy chain variable region is as shown in SEQ ID NO: 34.

In some embodiments, in the antibody, the amino acid sequence of the light chain variable region is as shown in SEQ ID NO: 35, and the amino acid sequence of the heavy chain variable region is as shown in SEQ ID NO: 36.

In some embodiments, in the antibody, the amino acid sequence of the light chain variable region is as shown in SEQ ID NO: 35, and the amino acid sequence of the heavy chain variable region is as shown in SEQ ID NO: 37.

In some embodiments, in the antibody, the amino acid sequence of the light chain variable region is as shown in SEQ ID NO: 41, and the amino acid sequence of the heavy chain variable region is as shown in SEQ ID NO: 42.

In some embodiments, in the antibody, the amino acid sequence of the light chain variable region is as shown in SEQ ID NO: 41, and the amino acid sequence of the heavy chain variable region is as shown in SEQ ID NO: 43.

In the antibody according to the first aspect of the present disclosure, the antibody satisfies one or more of three criteria as follows: (1) the antibody is a full-length antibody, Fab, Fab', F(ab')₂, or Fv, and the Fv is preferably scFv; (2) the antibody is a monospecific antibody or a multispecific antibody; (3) the antibody is a monoclonal antibody or a polyclonal antibody derived from the above antibodies.

The antibody of the present disclosure includes a monoclonal antibody (abbreviated as mAb or Ab), which refers to an antibody obtained from a single clonal cell line, and the cell line is not limited to an eukaryotic, prokaryotic, or phage-derived clonal cell line.

In the antibody according to the first aspect of the present disclosure, the antibody comprises a heavy chain constant region and/or a light chain constant region.

In some embodiments, the heavy chain constant region of the antibody is derived from heavy chain constant region of human antibody IgG1, IgG2, IgG3, or IgG4, and/or the light chain constant region of the antibody is derived from κ chain of a human antibody.

In some embodiments, the constant region comprises a constant region variant that does not alter the structure and function of the variable region of the antibody. A variety of such constant region variants have been disclosed in the prior art. For example, Fc of the heavy chain constant region of an antibody has substitutions of one or more amino acids at positions 238, 265, 269, 270, 297, 327, and 329 (using the EU numbering system) (see U.S. Patent No. 6,737,056), or Fc of the heavy chain constant region of an antibody has substitutions of one or more amino acids at positions 234, 235, 265, and 329, or Fc of the heavy chain constant region of an antibody has substitutions of one or more amino acids at positions 238, 252, 254, 256, 265, 272, 286, 303, 305, 307, 311, 312, 317, 340, 356, 360, 362, 376, 378, 380, 382, 413, 424, or 434 (using the EU numbering system) (see U.S. Patent No. 7,371,826), among others. These mutations have been shown to confer new properties to an antibody without altering the function of the variable region of the antibody.

In a preferred embodiment of the present disclosure, the heavy chain constant region comprises an amino acid sequence as shown in SEQ ID NO: 45, and the light chain constant region comprises an amino acid sequence as shown in SEQ ID NO: 44.

In a more preferred embodiment of the present disclosure, a heavy chain of the antibody comprises an amino acid sequence as shown in SEQ ID NO: 94, and a light chain of the antibody comprises an amino acid sequence as shown in SEQ ID NO: 95.

In a more preferred embodiment of the present disclosure, the heavy chain of the antibody comprises an amino acid sequence as shown in SEQ ID NO: 96, and the light chain of the antibody comprises an amino acid sequence as shown in SEQ ID NO: 81.

In a more preferred embodiment of the present disclosure, the heavy chain of the antibody comprises an amino acid sequence as shown in SEQ ID NO: 87, and the light chain of the antibody comprises an amino acid sequence as shown in SEQ ID NO: 81.

In a preferred embodiment of the present disclosure, the amino acid sequence of the heavy chain constant region is as shown in SEQ ID NO: 45, and the amino acid sequence of the light chain constant region is as shown in SEQ ID NO: 44.

In a preferred embodiment of the present disclosure, the amino acid sequence of the heavy chain of the antibody is as shown in SEQ ID NO: 94, and the amino acid sequence of the light chain of the antibody is as shown in SEQ ID NO: 95.

In a preferred embodiment of the present disclosure, the amino acid sequence of the heavy chain of the antibody is as shown in SEQ ID NO: 96, and the amino acid sequence of the light chain of the antibody is as shown in SEQ ID NO: 81.

In a preferred embodiment of the present disclosure, the amino acid sequence of the heavy chain of the antibody is as shown in SEQ ID NO: 87, and the amino acid sequence of the light chain of the antibody is as shown in SEQ ID NO: 81.

A second aspect of the present disclosure provides a bispecific antibody comprising a first antigen-binding domain that specifically binds to human CD40 and a second antigen-binding domain that specifically binds to human PD-L1; wherein the first antigen-binding domain that specifically binds to human CD40 is as defined in the anti-CD40 antibody according to the first aspect of the present disclosure.

The term "antibody" according to the present disclosure is used in its broadest sense, encompassing monoclonal antibodies, polyclonal antibodies, monospecific antibodies, and multispecific antibodies (*e.g.,* bispecific antibodies, diabodies, triabodies, tetrabodies, tandem bi-scFv, and tandem tri-scFv). It also encompasses traditional antibodies (antibodies with a tetrapeptide chain structure consisting of two identical heavy chains and two identical light chains linked by an inter-chain disulfide bond), as well as those with antigen-binding activity such as Fab, Fab', F(ab')₂, Fv, linear antibodies, single-chain antibodies, scFv, sdAb, sdFv, nanobodies, peptibodies, and domain antibodies (heavy chain (VH) antibodies and light chain (VL) antibodies). The traditional antibody (also referred to as "full-length antibody" or "complete antibody") is typically a heterotetrameric protein of about 150,000 daltons, which is a tetrapeptide chain structure consisting of two identical light chains (L) and two identical heavy chains (H) linked by an inter-chain disulfide bond. Each heavy chain of a full-length antibody consists of a heavy chain variable region (abbreviated as VH in the present disclosure) and a heavy chain constant region. The heavy chain constant region consists of three domains: CH1, CH2, and CH3. Each light chain consists of a light chain variable region (abbreviated as VL in the present disclosure) and a light chain constant region (abbreviated as CL in the present disclosure). The light chain constant region consists of one domain: CL. Mammalian heavy chains are classified as α, δ, ε, γ, and µ heavy chains. Mammalian light chains are classified as λ or κ light chains. Immunoglobulins containing α, δ, ε, γ, and µ heavy chains are immunoglobulin (Ig) A, IgD, IgE, IgG, and IgM. Complete antibodies form a "Y" shape. The stem of Y consists of a second constant region and a third constant region (and optionally a fourth constant region for IgE and IgM) of two heavy chains bound together via a disulfide bond (inter-chain) formed in a hinge region. γ, α, and δ heavy chains have a constant region consisting of three tandem (linear) Ig domains along with a hinge region for increased flexibility; µ and ε heavy chains have a constant region consisting of four immunoglobulin domains. The second and third constant regions are referred to as "CH2 domain" and "CH3 domain", respectively. Each arm of Y includes a variable region and a first constant region (CH1) of a single heavy chain bound to a single light chain. The "Fc" region refers to the two heavy chain fragments containing the CH2 and CH3 domains of an antibody, which are held together by two or more disulfide bonds and through the hydrophobic interaction of the CH3 domain. A variety of Fc constant region variants have been disclosed in the prior art. For example, Fc of the heavy chain constant region of an antibody has substitutions of one or more amino acids at positions 238, 265, 269, 270, 297, 327, and 329 (using the EU numbering system) (see U.S. Patent No. 6,737,056), or Fc of the heavy chain constant region of an antibody has substitutions of one or more amino acids at positions 234, 235, 265, and 329, or Fc of the heavy chain constant region of an antibody has substitutions of one or more amino acids at positions 238, 252, 254, 256, 265, 272, 286, 303, 305, 307, 311, 312, 317, 340, 356, 360, 362, 376, 378, 380, 382, 413, 424, or 434 (using the EU numbering system) (see U.S. Patent No. 7,371,826), among others. These mutations have been shown to confer new properties to an antibody without altering the function of the variable region of the antibody.

The term "variable region" or "variable domain" refers to a domain in the heavy or light chain of an antibody that is involved in the binding of the antibody to an antigen. VHH, VH, and VL each contain four conserved framework regions (FRs) and three complementary determining regions (CDRs). The term "complementary determining region" or "CDR" refers to a region within the variable domain that primarily contributes to antigen binding; "framework" or "FR" refers to variable domain residues other than CDR residues. VH or VHH contains three CDRs: for ease of differentiation, the three CDRs of VH are identified as HCDR1, HCDR2, and HCDR3, while the three CDRs of VHH are identified as VHH-CDR1, VHH-CDR2, and VHH-CDR3; VL contains three CDRs: LCDR1, LCDR2, and LCDR3. Each VH and VL consists of three CDRs and four FRs arranged from the amino terminus to the carboxy terminus in the following order: FR1, CDR1, FR2, CDR2, FR3, CDR3, FR4. A single VH or VL may be sufficient to confer specificity for antigen binding. As used herein, the terms "VHH" and "nanobody" have the same meaning and are used interchangeably, and refer to a variable region of the heavy chain of a cloned antibody, and a nanobody consisting of only one heavy chain variable region, which has a complete antigen-binding function. VHH-specific binding epitopes are not required to be recognized by additional antigen-binding domains (unlike conventional antibodies with a tetrapeptide chain structure, where epitopes are recognized by a structural pair formed by both VL and VH). VHH is a small, stable, and efficient antigen recognition unit formed by a single heavy chain variable domain. Nanobody has excellent biological properties, with a molecular weight of 12 to 15 kDa, which is one-tenth that of an intact antibody. It has good tissue penetration, high specificity, and good water solubility. Due to their special structural properties, nanobodies combine the advantages of traditional antibodies and small molecule drugs, essentially overcoming the shortcomings of traditional antibodies such as long development cycle, low stability, and harsh storage conditions. Consequently, they have gradually emerged as a new force in the next generation of antibody therapies, showing a broad application prospect in immune diagnosis and treatment. VHH includes, but is not limited to, natural antibodies produced by camelids, antibodies produced by camelids followed by humanization, or antibodies obtained through screening by phage display technology. Methods for obtaining VHHs that bind to specific antigens or epitopes have been previously disclosed, for example, in the following references: R. van der Linden et al., Journal of Immunological Methods, 240 (2000) 185-195; Li et al., J Biol Chem., 287 (2012) 13713-13721; Deffar et al., African Journal of Biotechnology, Vol. 8 (12), pp. 2645-2652, June 17, 2009; and WO94/04678.

The term "PD-L1" includes any variant or isoform of PD-L1 that is naturally expressed by a cell. The antibody of the present disclosure can cross-react with PD-L1 from non-human species (*e.g.,* cynomolgus monkey). Alternatively, the antibody may also be specific for human PD-L1 and may not exhibit cross-reactivity with other species. PD-L1 or any variant or isoform thereof can be isolated from cells or tissues in which they are naturally expressed, or produced by recombinant techniques using common techniques in the art and those described herein.

In the bispecific antibody according to the present disclosure, preferably, the second antigen-binding domain comprises at least one VHH, and the VHH comprises VHH-CDR1, VHH-CDR2, and VHH-CDR3 of SEQ ID NO: 66. In some embodiments, the VHH-CDR1 comprises an amino acid sequence as shown in X₄₁YYX₄₂X₄₃C, wherein X₄₁ is D or E, X₄₂ is S or T, and X₄₃ is K or Q; the VHH-CDR2 comprises an amino acid sequence as shown in SEQ ID NO: 76; and the VHH-CDR3 comprises an amino acid sequence as shown in SEQ ID NO: 77.

In some embodiments, the VHH comprises VHH-CDR1, VHH-CDR2, and VHH-CDR3 of SEQ ID NO: 49, SEQ ID NO: 74, SEQ ID NO: 72, or SEQ ID NO: 73.

In some embodiments, the VHH-CDR1, VHH-CDR2, and VHH-CDR3 are defined according to the Kabat, IMGT, Chothia, AbM, or Contact numbering system, and in some specific embodiments, the VHH-CDR1, VHH-CDR2, and VHH-CDR3 are determined according to the Kabat numbering rule. In some embodiments, the VHH-CDR1 comprises an amino acid sequence as shown in SEQ ID NO: 46, SEQ ID NO: 67, or SEQ ID NO: 70; the VHH-CDR2 comprises an amino acid sequence as shown in SEQ ID NO: 47, SEQ ID NO: 68, or SEQ ID NO: 71; and the VHH-CDR3 comprises an amino acid sequence as shown in SEQ ID NO: 48 or SEQ ID NO: 69.

In some embodiments, the VHH comprises VHH-CDR1 as shown in SEQ ID NO: 46, SEQ ID NO: 67, or SEQ ID NO: 70, VHH-CDR2 as shown in SEQ ID NO: 47, SEQ ID NO: 68, or SEQ ID NO: 71, and VHH-CDR3 as shown in SEQ ID NO: 48 or SEQ ID NO: 69.

In a more preferred embodiment of the present disclosure, the VHH-CDR1 comprises the amino acid sequence as shown in SEQ ID NO: 46, the VHH-CDR2 comprises the amino acid sequence as shown in SEQ ID NO: 47, and the VHH-CDR3 comprises the amino acid sequence as shown in SEQ ID NO: 48;

or, the VHH-CDR1 comprises the amino acid sequence as shown in SEQ ID NO: 67, the VHH-CDR2 comprises the amino acid sequence as shown in SEQ ID NO: 68, and the VHH-CDR3 comprises the amino acid sequence as shown in SEQ ID NO: 69;

or, the VHH-CDR1 comprises the amino acid sequence as shown in SEQ ID NO: 70, the VHH-CDR2 comprises the amino acid sequence as shown in SEQ ID NO: 71, and the VHH-CDR3 comprises the amino acid sequence as shown in SEQ ID NO: 48.

In some embodiments, the VHH comprises:
VHH-CDR1 with the sequence as shown in SEQ ID NO: 46, VHH-CDR2 with the sequence as shown in SEQ ID NO: 47, and VHH-CDR3 with the sequence as shown in SEQ ID NO: 48;
VHH-CDR1 with the sequence as shown in SEQ ID NO: 67, VHH-CDR2 with the sequence as shown in SEQ ID NO: 68, and VHH-CDR3 with the sequence as shown in SEQ ID NO: 69,
or, VHH-CDR1 with the sequence as shown in SEQ ID NO: 70, VHH-CDR2 with the sequence as shown in SEQ ID NO: 71, and VHH-CDR3 with the sequence as shown in SEQ ID NO: 48.

In some embodiments, the amino acid sequence of the VHH is as shown in SEQ ID NO: 49, SEQ ID NO: 72, SEQ ID NO: 73, or SEQ ID NO: 74, or has at least 90%, at least 95%, or at least 99% sequence identity with the amino acid sequence as shown in SEQ ID NO: 49, SEQ ID NO: 72, SEQ ID NO: 73, or SEQ ID NO: 74.

In some embodiments, the VHH is a humanized VHH.

In some embodiments, the VHH comprises framework regions FR1, FR2, FR3, and FR4, wherein the FR1 comprises an amino acid sequence as shown in SEQ ID NO: 85, the FR2 comprises an amino acid sequence as shown in SEQ ID NO: 79, the FR3 comprises an amino acid sequence as shown in SEQ ID NO: 86, and the FR4 comprises an amino acid sequence as shown in SEQ ID NO: 84.

In some embodiments, the FR1 comprises an amino acid sequence as shown in SEQ ID NO: 82 or SEQ ID NO: 78, the FR2 comprises the amino acid sequence as shown in SEQ ID NO: 79, the FR3 comprises an amino acid sequence as shown in SEQ ID NO: 83 or SEQ ID NO: 80, and the FR4 comprises the amino acid sequence as shown in SEQ ID NO: 84.

In some embodiments, the FR1 comprises the amino acid sequence as shown in SEQ ID NO: 82, the FR2 comprises the amino acid sequence as shown in SEQ ID NO: 79, the FR3 comprises the amino acid sequence as shown in SEQ ID NO: 83, and the FR4 comprises the amino acid sequence as shown in SEQ ID NO: 84;
or, the FR1 comprises the amino acid sequence as shown in SEQ ID NO: 78, the FR2 comprises the amino acid sequence as shown in SEQ ID NO: 79, the FR3 comprises the amino acid sequence as shown in SEQ ID NO: 80, and the FR4 comprises the amino acid sequence as shown in SEQ ID NO: 84;
or, the FR1 comprises the amino acid sequence as shown in SEQ ID NO: 82, the FR2 comprises the amino acid sequence as shown in SEQ ID NO: 79, the FR3 comprises the amino acid sequence as shown in SEQ ID NO: 80, and the FR4 comprises the amino acid sequence as shown in SEQ ID NO: 84.

In some embodiments, the amino acid sequence of the VHH is as shown in SEQ ID NO: 49, SEQ ID NO: 72, SEQ ID NO: 73, or SEQ ID NO: 74, or has at least 90% sequence identity with the amino acid sequence as shown in SEQ ID NO: 49, SEQ ID NO: 72, SEQ ID NO: 73, or SEQ ID NO: 74.

In some embodiments, the amino acid sequence of the VHH is as shown in SEQ ID NO: 49, SEQ ID NO: 72, SEQ ID NO: 73, or SEQ ID NO: 74.

In some embodiments, the second antigen-binding domain further comprises a heavy chain constant region.

Preferably, the heavy chain constant region is selected from the heavy chain constant region of IgG1, IgG2, IgG3, or IgG4; the heavy chain constant region is preferably the Fc region of human IgG1; the heavy chain constant region more preferably comprises an amino acid sequence as shown in SEQ ID NO: 88.

More preferably, the VHH is linked to the heavy chain constant region via a linker; the linker is preferably a linker having an amino acid sequence as shown in (G4S)x, wherein x is independently selected from an integer of 1 to 20, and more preferably is a linker as shown in SEQ ID NO: 89.

Further more preferably, the amino acid sequence of the second antigen-binding domain is as shown in SEQ ID NO: 93, SEQ ID NO: 90, SEQ ID NO: 91, or SEQ ID NO: 92, or has at least 90% sequence identity with the amino acid sequence as shown in SEQ ID NO: 93, SEQ ID NO: 90, SEQ ID NO: 91, or SEQ ID NO: 92;

Most preferably, the second antigen-binding domain has two amino acid sequences as shown in SEQ ID NO: 93.

In the present disclosure, "Fab" consists of a light chain and the CH1 and variable region of a heavy chain. "Fab'" contains a light chain and a portion containing the VH and CH1 domains along with the region between the CH1 and CH2 domains. An inter-chain disulfide bond can be formed between the two heavy chains of two Fab' fragments to form an F(ab')₂ molecule. The F(ab')₂ fragment consists of two Fab' fragments held together by a disulfide bond between the two heavy chains. The term "Fv" refers to an antibody fragment consisting of the VL and VH domains of a single arm of an antibody.

In the present disclosure, scFv (single-chain antibody fragment) refers to a polypeptide chain formed by linking the VH and VL domains via a linker (also known as a spacer). The VL and VH domains are paired to form a monovalent molecule via a linker that enables them to produce a single polypeptide chain [see, *e.g.,* Bird et al., Science 242: 423-426 (1988) and Huston et al., Proc. Natl. Acad. Sci. USA 85: 5879-5883 (1988)]. Such scFv molecules may have the general structure: NH2-VL-linker-VH-COOH or NH2-VH-linker-VL-COOH. Suitable linkers in the prior art consist of repeated G₄S amino acid sequences or variants thereof. For example, linkers having the amino acid sequence (G₄S)₄ or (G₄S)₃ may be used, but variants thereof may also be used.

The term "multispecific antibody" is used in its broadest sense to encompass antibodies with specificity for two or more epitopes, such as bispecific antibodies. These multispecific antibodies include, but are not limited to: antibodies comprising a heavy chain variable region (VH) and a light chain variable region (VL), wherein the VH-VL unit has specificity for two or more epitopes; antibodies having two or more VL and VH regions, wherein each VH-VL unit binds to a different target or a different epitope of the same target; antibodies having two or more single variable regions (*e.g.,* VHH), wherein each single variable region binds to a different target or a different epitope of the same target.

The term "epitope" refers to an area or region on an antigen that can specifically bind to an antibody. Epitopes may be formed from a string of continuous amino acids (linear epitopes) or contain non-contiguous amino acids (conformational epitopes) that come into spatial proximity, for example, due to the folding of an antigen (*i.e*., by the tertiary folding of a proteinaceous antigen). The difference between conformational and linear epitopes is the loss of antibody binding to conformational epitopes in the presence of denaturing solvents. An epitope contains at least 3, at least 4, at least 5, at least 6, at least 7, or 8 to 10 amino acids in a unique spatial conformation. Screening for antibodies that bind to a specific epitope (*i.e.,* those that bind to the same epitope) can be performed using routine methods in the art, such as, but not limited to, alanine scanning or peptide mapping (see Meth. Mol. Biol. 248 (2004) 443-463).

The term "specific binding" means that an antibody binds to an antigen or an epitope within the antigen with higher affinity than against other antigens or epitopes. Typically, an antibody binds to an antigen or an epitope within the antigen with an equilibrium dissociation constant (K_{D}) of approximately 1 × 10⁻⁷ M or less (*e.g.,* approximately 1 × 10⁻⁸ M or less, approximately 1 × 10⁻⁹ M or less, approximately 1 × 10⁻¹⁰ M or less, approximately 1 × 10⁻¹¹ M or less, or approximately 1 × 10⁻¹² M or less). In some embodiments, the K_{D} of an antibody binding to an antigen is 10% or 1% of the K_{D} of the antibody binding to a non-specific antigen *(e.g.,* BSA or casein). K_{D} can be measured using standard procedures, such as BIACORE^{®} surface plasmon resonance assay. However, antibodies that specifically bind to an antigen or an epitope within the antigen may exhibit cross-reactivity with other related antigens, such as the same antigens from other species (homologous) (*e.g.,* humans or monkeys, such as cynomolgus monkeys (Macaca fascicularis), chimpanzees (Pan troglodytes), or common marmosets (Callithrix jacchus)).

The term "affinity" refers to the overall strength of the non-covalent interaction between a single binding site of a molecule *(e.g.,* an antibody) and its binding ligand *(e.g.,* an antigen). Unless otherwise indicated, as used herein, "affinity" refers to intrinsic binding affinity, which reflects a 1:1 interaction between members of a binding pair (*e.g.,* antibody and antigen). The affinity of molecule X for its ligand Y can typically be represented by the dissociation constant (K_{D}). Affinity can be measured by conventional methods known in the art, including those described herein. The term "k_{assoc}" or "kₐ" refers to the association rate of a specific antibody-antigen interaction, while the term "k_{dis}" or "k_{d}" as used herein refers to the dissociation rate of a specific antibody-antigen interaction. As used herein, the term "K_{D}" refers to the dissociation constant, which is obtained from the ratio of kₐ to kₐ (*i.e.,* k_{d}/kₐ) and is expressed in molar concentration (M). The K_{D} value of an antibody can be determined using methods well established in the art. Methods for determining the K_{D} of an antibody include measuring surface plasmon resonance using a biosensor system such as a system, or measuring affinity in solution by solution equilibrium titration (SET).

The term "anti-CD40 antibody" or "antibody that specifically binds to CD40" refers to an antibody that is capable of binding to CD40 with sufficient affinity such that the antibody can be used as a diagnostic and/or therapeutic agent targeting CD40. In some examples, antibodies that bind to CD40 have a dissociation constant (K_{D}) of less than approximately 1 µM, less than approximately 100 nM, less than approximately 10 nM, less than approximately 1 nM, less than approximately 0.1 nM, less than approximately 0.01 nM, or less than approximately 0.001 nM (such as 10⁻⁸ M or less, such as 10⁻⁸ M to 10⁻¹² M, such as 10⁻⁹ M to 10⁻¹⁰ M). In some examples, anti-CD40 antibodies bind to conserved antigenic epitopes within CD40 from different species.

The term "anti-PD-L1 antibody" or "antibody that specifically binds to PD-L1" refers to an antibody that is capable of binding to PD-L1 with sufficient affinity such that the antibody can be used as a diagnostic and/or therapeutic agent targeting PD-L1. In some examples, antibodies that bind to PD-L1 have a dissociation constant (K_{D}) of less than approximately 1 µM, less than approximately 100 nM, less than approximately 10 nM, less than approximately 1 nM, less than approximately 0.1 nM, less than approximately 0.01 nM, or less than approximately 0.001 nM (such as 10⁻⁸ M or less, such as 10⁻⁸ M to 10⁻¹² M, such as 10⁻⁹ M to 10⁻¹⁰ M). In some examples, anti-PD-L1 antibodies bind to conserved antigenic epitopes within PD-L1 from different species.

In some preferred examples, the first antigen-binding domain is operably linked to the second antigen-binding domain directly or via a linker.

Preferably, the second antigen-binding domain is linked to N-terminus of a light chain variable region or heavy chain variable region of the first antigen-binding domain, or to C-terminus of the light chain constant region, or to C-terminus of the IgG.

The linker is preferably a peptide sequence, more preferably comprising or consisting of (G₄S)ₙG, wherein n is an integer of 1 to 10, for example, n is 3.

The bispecific antibody according to the present disclosure comprises two first polypeptide chains and two second polypeptide chains.

In some preferred examples, the amino acid sequence of the first polypeptide chain is as shown in SEQ ID NO: 50 or has at least 99%, at least 95%, or at least 90% sequence identity with SEQ ID NO: 50; and/or, the amino acid sequence of the second polypeptide chain is as shown in SEQ ID NO: 51 or has at least 99%, at least 95%, or at least 90% sequence identity with SEQ ID NO: 51.

In some preferred examples, the amino acid sequence of the first polypeptide chain is as shown in SEQ ID NO: 52 or has at least 99%, at least 95%, or at least 90% sequence identity with SEQ ID NO: 52; and/or, the amino acid sequence of the second polypeptide chain is as shown in SEQ ID NO: 53 or SEQ ID NO: 60 or has at least 99%, at least 95%, or at least 90% sequence identity with SEQ ID NO: 53 or SEQ ID NO: 60.

In some preferred examples, the amino acid sequence of the first polypeptide chain is as shown in SEQ ID NO: 54 or has at least 99%, at least 95%, or at least 90% sequence identity with SEQ ID NO: 54; and/or, the amino acid sequence of the second polypeptide chain is as shown in SEQ ID NO: 55 or SEQ ID NO: 61 or has at least 99%, at least 95%, or at least 90% sequence identity with SEQ ID NO: 55 or SEQ ID NO: 61.

In some preferred examples, the amino acid sequence of the first polypeptide chain is as shown in SEQ ID NO: 56 or has at least 99%, at least 95%, or at least 90% sequence identity with SEQ ID NO: 56; and/or, the amino acid sequence of the second polypeptide chain is as shown in SEQ ID NO: 57 or SEQ ID NO: 62 or has at least 99%, at least 95%, or at least 90% sequence identity with SEQ ID NO: 62.

In some preferred examples, the amino acid sequence of the first polypeptide chain is as shown in SEQ ID NO: 58 or has at least 99%, at least 95%, or at least 90% sequence identity with SEQ ID NO: 58; and/or, the amino acid sequence of the second polypeptide chain is as shown in SEQ ID NO: 59 or SEQ ID NO: 63 or has at least 99%, at least 95%, or at least 90% sequence identity with SEQ ID NO: 59 or SEQ ID NO: 63.

A third aspect of the present disclosure provides a bispecific antibody comprising a first antigen-binding domain that specifically binds to human CD40 and a second antigen-binding domain that specifically binds to human PD-L1, wherein the second antigen-binding domain comprises at least one VHH, and the VHH has a sequence as defined in the bispecific antibody according to the second aspect of the present disclosure.

In some preferred examples, the first antigen-binding domain is as defined in the anti-CD40 antibody according to the first aspect of the present disclosure.

In some preferred examples, the first antigen-binding domain is operably linked to the second antigen-binding domain directly or via a linker.

Preferably, the second antigen-binding domain is linked to N-terminus of a light chain variable region or heavy chain variable region of the first antigen-binding domain, or to C-terminus of the light chain constant region, or to C-terminus of the IgG.

The linker is preferably a peptide sequence, more preferably comprising or consisting of (G₄S)ₙG, wherein n is an integer of 1 to 10, for example, n is 3.

The bispecific antibody according to the present disclosure comprises two first polypeptide chains and two second polypeptide chains.

In some preferred examples, the amino acid sequence of the first polypeptide chain is as shown in SEQ ID NO: 50, and/or, the amino acid sequence of the second polypeptide chain is as shown in SEQ ID NO: 51.

In some preferred examples, the amino acid sequence of the first polypeptide chain is as shown in SEQ ID NO: 52, and/or, the amino acid sequence of the second polypeptide chain is as shown in SEQ ID NO: 53 or SEQ ID NO: 60.

In some preferred examples, the amino acid sequence of the first polypeptide chain is as shown in SEQ ID NO: 54, and/or, the amino acid sequence of the second polypeptide chain is as shown in SEQ ID NO: 55 or SEQ ID NO: 61.

In some preferred examples, the amino acid sequence of the first polypeptide chain is as shown in SEQ ID NO: 56, and/or, the amino acid sequence of the second polypeptide chain is as shown in SEQ ID NO: 57 or SEQ ID NO: 62.

In some preferred examples, the amino acid sequence of the first polypeptide chain is as shown in SEQ ID NO: 58, and/or, the amino acid sequence of the second polypeptide chain is as shown in SEQ ID NO: 59 or SEQ ID NO: 63.

A fourth aspect of the present disclosure provides an isolated nucleic acid encoding the anti-CD40 antibody according to the first aspect of the present disclosure or the bispecific antibody according to the second aspect or the third aspect of the present disclosure.

As known in the art, "nucleic acid" in the present disclosure refers to a nucleotide chain of any length and includes DNA and RNA. Nucleotides may be deoxyribonucleotides, ribonucleotides, modified nucleotides or bases, and/or analogs thereof, or any substrate that can be incorporated into a strand by DNA or RNA polymerase.

A fifth aspect of the present disclosure provides a recombinant expression vector comprising the isolated nucleic acid according to the fourth aspect of the present disclosure.

Preferably, the recombinant expression vector is a plasmid, cosmid, phage, or viral vector.

For example, the plasmid has a backbone of pcDNA3.4.

The term "recombinant expression vector" means a genetically modified oligonucleotide or polynucleotide construct that permits the expression of an mRNA, protein, polypeptide, or peptide by a host cell, when the construct comprises a nucleotide sequence encoding the mRNA, protein, polypeptide, or peptide, and the vector is contacted with the cell under conditions sufficient to have the mRNA, protein, polypeptide, or peptide expressed within the cell. The vectors of the present disclosure are not naturally occurring as a whole. However, parts of the vectors may be naturally occurring. The recombinant expression vector of the present disclosure may comprise any type of nucleotide, including, but not limited to, DNA and RNA, which may be single-stranded or double-stranded, synthesized or partially obtained from natural sources, and which may contain natural, non-natural, or altered nucleotides. The recombinant expression vector may comprise naturally occurring or non-naturally occurring internucleotide linkages, or both types of linkages. In exemplary aspects, the altered nucleotides or non-naturally occurring internucleotide linkages do not hinder the transcription or replication of the vector.

The recombinant expression vector of the present disclosure may be any suitable recombinant expression vector that can be used for transformation or transfection to deliver one or more genes or sequences of interest into any suitable host cell and preferably to express the genes or sequences in the host cell. Suitable vectors include those designed for expansion and amplification or for expression or both. Examples of vectors include, but are not limited to, viral vectors, naked DNA or RNA expression vectors, plasmid, cosmid, or phage vectors, DNA or RNA expression vectors associated with cationic coagulants, DNA or RNA expression vectors encapsulated in liposomes, and certain eukaryotic cells, such as production cells.

A sixth aspect of the present disclosure provides a transformant comprising the recombinant expression vector according to the fifth aspect of the present disclosure.

Preferably, the transformant has a host cell that is either a prokaryotic cell or a eukaryotic cell.

More preferably, the eukaryotic cell is a yeast cell or a mammalian cell.

The mammalian cell is, for example, an EXPI-293 cell or a CHO cell.

As used herein, the term "host cell" refers to any type of cell that may contain the nucleic acid or vector described herein. The host cell may be a eukaryotic cell, such as a plant, animal, fungus, or algae; or may be a prokaryotic cell, such as a bacterium or protozoan. As described herein, the host cell may be a cell derived or obtained from a subject. The host cell can be derived or obtained from a mammal. As used herein, the term "mammal" refers to any mammal, including, but not limited to, mammals of the order Rodentia, such as mice and hamsters; and mammals of the order Lagomorpha, such as rabbits. Preferably, the mammal is from the order Carnivora, including felids (cats) and canids (dogs). More preferably, the mammal is from the order Artiodactyla, including bovids (cattle) and suids (pigs) or of the order Perssodactyla, including equids (horses). Most preferably, the mammal is of the order Primates, Ceboids, or Simoids (monkeys) or of the order Anthropoids (humans and apes). A particularly preferred mammal is a human.

The expression vector can be transfected or introduced into suitable host cells. A variety of techniques can be used to achieve this purpose, including protoplast fusion, calcium phosphate precipitation, electroporation, retroviral transduction, viral transfection, gene editing (CRISPR-Cas system, ZFN system, or TALEN system), transposons (Sleeping Beauty or PiggyBAC), gene guns, lipid-based transfection, or other conventional techniques. In the case of protoplast fusion, cells are cultured in medium and screened for appropriate activity. The methods and conditions for culturing the resulting transfected cells and for recovering the resulting antibody molecules are known to those skilled in the art and can be varied or optimized based on the specification and methods known in the prior art, depending on the specific expression vectors and mammalian host cells used. Alternatively, cells that have stably incorporated DNA into their chromosomes can be selected by introducing one or more markers that allow selection of transfected host cells. The marker may, for example, provide prototrophy, biocidal resistance (*e.g.,* antibiotics), or heavy metal (*e.g.,* copper) resistance to an auxotrophic host. The selectable marker gene can be linked directly to the DNA sequence to be expressed or be introduced into the same cell by co-transformation. Additional elements may also be required for optimal synthesis of mRNA. These elements may include splicing signals, as well as transcriptional promoters, enhancers, and termination signals.

A seventh aspect of the present disclosure provides a method for preparing an anti-CD40 antibody or a bispecific antibody, comprising steps of: culturing the transformant according to the sixth aspect of the present disclosure, and obtaining an anti-CD40 antibody or a bispecific antibody from the culture.

An eighth aspect of the present disclosure provides a pharmaceutical composition comprising the anti-CD40 antibody according to the first aspect of the present disclosure, the bispecific antibody according to the second aspect or the third aspect of the present disclosure, and a pharmaceutically acceptable carrier.

Preferably, the pharmaceutical composition further comprises an other agent. In some embodiments, the other agent is selected from one or more of the group consisting of a hormone preparation, a targeted small molecule preparation, a proteasome inhibitor, an imaging agent, a diagnostic agent, a chemotherapeutic agent, an oncolytic drug, a cytotoxic agent, a cytokine, an activator of co-stimulatory molecules, an inhibitor of inhibitory molecules, and a vaccine.

The "pharmaceutically acceptable carrier" is any of those conventionally used and is limited only by physico-chemical considerations (*e.g.,* solubility and lack of reactivity with CD40-targeting antibodies), and by the route of administration. The pharmaceutically acceptable carriers described herein, such as vehicles, adjuvants, excipients, and diluents, are well known to those skilled in the art and are readily available to the public. In one aspect, the pharmaceutically acceptable carrier is one that is chemically inert to the active ingredients of the pharmaceutical composition, and one which has no detrimental side effects or toxicity under the conditions of use. In some examples, the carrier does not produce adverse, allergic, or other untoward reactions when administered to animals or humans. In some aspects, the pharmaceutical composition is free of pyrogens and other impurities that may be harmful to humans or animals. Pharmaceutically acceptable carriers include any and all solvents, dispersion media, coatings, antibacterial and antifungal agents, isotonic and absorption delaying agents, *etc*.; the use of which are well known in the art.

Therapeutic formulations of compositions suitable for implementing the methods disclosed herein, such as polypeptides, polynucleotides, or antibodies, can be prepared for storage by mixing the selected composition having the desired purity with an optional physiologically and pharmaceutically acceptable carrier, excipient, or stabilizer in the form of a lyophilized cake or an aqueous solution *(*Remington's Pharmaceutical Sciences, 18th edition, edited by A. R. Gennaro, Mack Publishing Company (1990)). Pharmaceutical compositions can be manufactured by admixing with one or more suitable carriers or adjuvants such as water, mineral oil, polyethylene glycol, starch, talc, lactose, thickeners, stabilizers, suspending agents, *etc.* Such compositions may be in the form of solutions, suspensions, tablets, capsules, creams, salves, ointments, or other conventional forms.

The composition to be used for *in vivo* administration should be sterile. This is readily accomplished by filtration through a sterile filtration membrane, prior to or following lyophilization and reconstitution. Therapeutic compositions are generally placed in a container having a sterile access port, such as an intravenous solution bag or vial having a stopper pierceable by a hypodermic needle. Pharmaceutical forms suitable for injectable use include sterile aqueous solutions or dispersions and sterile powders for the extemporaneous preparation of sterile injectable solutions or dispersions. In some cases, the form should be sterile and should be fluid to the extent that it can be easily injected. It should be stable under the conditions of manufacture and storage and should be preserved against the contaminating action of microorganisms, such as bacteria and fungi. The composition for parenteral administration will typically be stored in lyophilized form or in solution.

The carrier may be a solvent or dispersion medium containing, for example, water or a suitable mixture thereof and vegetable oil. The proper fluidity can be maintained, for example, by the use of coatings, such as lecithin, by the maintenance of a desired particle size in the case of dispersions, and by the use of surfactants. The choice of carrier will be determined, in part, by the particular type of pharmaceutical composition as well as the route of administration of the pharmaceutical composition. Accordingly, a variety of suitable formulations of the pharmaceutical composition may be formulated.

The pharmaceutical composition of the present disclosure may comprise any pharmaceutically acceptable ingredient, including, for example, acidifying agents, additives, adsorbents, aerosol propellants, air displacement agents, alkalizing agents, anticaking agents, anticoagulants, antimicrobial preservatives, antioxidants, antiseptics, bases, binders, buffers, chelating agents, coating agents, colorants, desiccants, detergents, diluents, disinfectants, disintegrants, dispersants, dissolution enhancers, dyes, emollients, emulsifiers, emulsion stabilizers, fillers, film-forming agents, flavor enhancers, flavoring agents, flow enhancers, gelling agents, granulating agents, thermal insulators, lubricants, mucoadhesives, ointment bases, ointments, oleaginous vehicles, organic bases, pastille bases, pigments, plasticizers, polishing agents, preservatives, sequestering agents, skin penetrants, solubilizers, solvents, stabilizers, suppository bases, surface active agents, surfactants, suspending agents, sweetening agents, therapeutic agents, thickeners, tonicity agents, toxicants, tackifiers, water absorbents, water-miscible cosolvents, water softeners, or wetting agents.

In some examples, the pharmaceutical composition comprising the bispecific antibody described herein is formulated for parenteral administration, subcutaneous administration, intravenous administration, intramuscular administration, intraarterial administration, intrathecal administration, or intraperitoneal administration. In other examples, the pharmaceutical composition is administered via nasal, spray, oral, aerosol, rectal, or vaginal administration. The composition can be administered by infusion, bolus injection, or via an implantable device.

Topical formulations are well known to those skilled in the art. Such formulations are particularly suitable for application to the skin in the context of the present disclosure.

In some examples, the pharmaceutical composition described herein is formulated for parenteral administration. For purposes herein, parenteral administration includes, but is not limited to, intravenous, intraarterial, intramuscular, intracerebral, intracerebroventricular, intracardiac, subcutaneous, intraosseous, intradermal, intrathecal, intraperitoneal, retrobulbar, intrapulmonary, intravesical, and intracavernosal injections or infusions. Administration by surgical implantation at a specific site is also contemplated.

Injectable formulations are in accordance with the present disclosure. The requirements for effective pharmaceutical carriers for injectable compositions are well known to those of ordinary skill in the art (see, *e.g.,* Pharmaceutics and Pharmacy Practice, J. B. Lippincott Company, Philadelphia, PA, Banker and Chalmers, eds., pp. 238-250 (1982); and ASHP Handbook on Injectable Drugs, Toissel, 4th ed., pp. 622-630 (1986)).

It should be appreciated by those skilled in the art that, in addition to the pharmaceutical compositions described above, the compositions of the present disclosure can be formulated as inclusion complexes, such as cyclodextrin inclusion complexes, or liposomes.

A ninth aspect of the present disclosure provides a use of the anti-CD40 antibody according to the first aspect of the present disclosure, the bispecific antibody according to the second aspect or the third aspect of the present disclosure, and/or the pharmaceutical composition according to the eighth aspect of the present disclosure in the manufacture of a medicament for preventing and/or treating a tumor.

The tumor is preferably a PD-L1 positive and/or CD40 positive tumor.

In a preferred embodiment of the present disclosure, the tumor is lymphoma, breast cancer, ovarian cancer, prostate cancer, pancreatic cancer, kidney cancer, lung cancer, liver cancer, gastric cancer, colorectal cancer, bladder cancer, rhabdomyosarcoma, esophageal cancer, cervical cancer, multiple myeloma, leukemia, gallbladder cancer, glioblastoma, or melanoma, but not limited thereto.

A tenth aspect of the present disclosure provides a kit comprising the anti-CD40 antibody according to the first aspect of the present disclosure, the bispecific antibody according to the second aspect or the third aspect of the present disclosure, or the pharmaceutical composition according to the eighth aspect of the present disclosure.

Preferably, the kit further comprises (i) a device for administering the antibody or pharmaceutical composition; and/or (ii) instructions for use.

An eleventh aspect of the present disclosure provides a drug box kit comprising a drug box A and a drug box B, wherein
the drug box A comprises the anti-CD40 antibody according to the first aspect of the present disclosure, the bispecific antibody according to the second aspect or the third aspect of the present disclosure, and/or the pharmaceutical composition according to the eighth aspect of the present disclosure;
the drug box B comprises an other anti-tumor antibody or a pharmaceutical composition comprising the other anti-tumor antibody, and/or one or more of the group consisting of a hormone preparation, a targeted small molecule preparation, a proteasome inhibitor, an imaging agent, a diagnostic agent, a chemotherapeutic agent, an oncolytic drug, a cytotoxic agent, a cytokine, an activator of co-stimulatory molecules, an inhibitor of inhibitory molecules, and a vaccine.

A twelfth aspect of the present disclosure provides an immunoassay, or a method for determining CD40 and/or PD-L1, comprising using the anti-CD40 antibody according to the first aspect of the present disclosure, the bispecific antibody according to the second aspect or the third aspect of the present disclosure, and/or the pharmaceutical composition according to the eighth aspect of the present disclosure.

In a preferred embodiment of the present disclosure, the immunoassay is an assay for non-diagnostic purposes and is only suitable for scientific research purposes.

A thirteenth aspect of the present disclosure provides a method for preventing and/or treating a tumor, comprising administering to a patient in need thereof a therapeutically effective amount of the anti-CD40 antibody according to the first aspect of the present disclosure, the bispecific antibody according to the second aspect or the third aspect of the present disclosure, and/or the pharmaceutical composition according to the eighth aspect of the present disclosure, or the drug box kit according to the eleventh aspect of the present disclosure.

For example, the tumor is lymphoma, breast cancer, ovarian cancer, prostate cancer, pancreatic cancer, kidney cancer, lung cancer, liver cancer, gastric cancer, colorectal cancer, bladder cancer, rhabdomyosarcoma, esophageal cancer, cervical cancer, multiple myeloma, leukemia, gallbladder cancer, glioblastoma, or melanoma, but not limited thereto.

As used in the present disclosure, the term "effective amount" refers to an amount of a drug or agent that elicits a biological or pharmacological response in a tissue, system, animal, or human, for example, that is sought by a researcher or clinician. Additionally, the term "therapeutically effective amount" refers to an amount that results in an improvement in the treatment, cure, prevention, or alleviation of a disease, condition, or side effect, or a reduction in the rate of progression of a disease or condition, as compared to a corresponding subject that does not receive the amount. The term also includes within its scope an amount effective to enhance normal physiological function.

A fourteenth aspect of the present disclosure provides a combination therapy comprising administering to a patient in need thereof the anti-CD40 antibody according to the first aspect of the present disclosure, the bispecific antibody according to the second aspect or the third aspect of the present disclosure, and/or the pharmaceutical composition according to the eighth aspect of the present disclosure, and a second therapeutic agent, respectively;

the second therapeutic agent preferably comprises an other anti-tumor antibody or a pharmaceutical composition comprising the other anti-tumor antibody, and/or one or more of the group consisting of a hormone preparation, a targeted small molecule preparation, a proteasome inhibitor, an imaging agent, a diagnostic agent, a chemotherapeutic agent, an oncolytic drug, a cytotoxic agent, a cytokine, an activator of co-stimulatory molecules, an inhibitor of inhibitory molecules, and a vaccine.

A fifteenth aspect of the present disclosure provides a use of the anti-CD40 antibody according to the first aspect of the present disclosure, the bispecific antibody according to the second aspect or the third aspect of the present disclosure, and/or the pharmaceutical composition according to the eighth aspect of the present disclosure as a medicament; in some technical solutions, the medicament is used to prevent and/or treat a tumor.

Preferably, the tumor is a PD-L1 positive and/or CD40 positive tumor.

For example, the tumor is lymphoma, breast cancer, ovarian cancer, prostate cancer, pancreatic cancer, kidney cancer, lung cancer, liver cancer, gastric cancer, colorectal cancer, bladder cancer, rhabdomyosarcoma, esophageal cancer, cervical cancer, multiple myeloma, leukemia, gallbladder cancer, glioblastoma, or melanoma.

Preferably, the tumor is colon cancer.

The present disclosure also provides an antibody-drug conjugate comprising the anti-CD40 antibody according to the first aspect of the present disclosure, or the bispecific antibody according to the second aspect or the third aspect of the present disclosure conjugated with one or more therapeutic agents or radioisotopes.

Preferably, the therapeutic agent is a cytotoxic agent, a chemotherapeutic agent, a drug, a growth inhibitor, and/or a toxin; and/or, the conjugation is to link the antibody to the therapeutic agent or radioisotope using a linker.

On the basis of common sense in the art, the above preferred conditions can be arbitrarily combined to obtain the preferred examples of the present disclosure.

The novel anti-CD40 agonistic antibody of the present disclosure can effectively regulate the activation of DC cells, with stronger T cell activation but with low toxic and side effects, making it suitable for tumor immunotherapy. The bispecific antibody of the present disclosure acts in a positive feedback loop involving cross-talk between DC cells and T cells by simultaneously activating CD40 and blocking PD-L1/PD-1, thereby maximizing anti-tumor efficacy; it also improves the selectivity of CD40 activation by means of PD-L1-dependent CD40 activation, thereby reducing the toxic and side effects of the CD40 agonistic antibody.

### BRIEF DESCRIPTION OF THE DRAWINGS

FIG. 1 shows the CD40 agonistic activity of the monoclonal antibody as determined by the reporter gene assay in Example 7 of the present disclosure.
FIG. 2 shows the DC regulatory activity of the monoclonal antibody in Example 8 of the present disclosure, using CD83 as an indicator of DC activation.
FIG. 3 shows the T cell regulatory activity of the monoclonal antibody in Example 9 of the present disclosure, using IFN-γ as an indicator of T cell activation.
FIGs. 4A to 4C show the safety of the monoclonal antibody in human CD40 gene knock-in mice in Example 10 of the present disclosure, among which FIG. 4A shows the changes in body weight of mice; FIG. 4B shows the changes in liver function and hematology of mice; FIG. 4C shows the changes in organ coefficients of mice.
FIG. 5 shows the PD-L1/PD-1 blocking activity of the monoclonal antibody as determined by the reporter gene assay in Example 12 of the present disclosure.
FIG. 6 shows the T cell regulatory activity of the monoclonal antibody in Example 13 of the present disclosure.
FIG. 7 shows the structure of the bispecific antibody in Example 4 of the present disclosure.
FIG. 8 shows the bispecific antibody binding to both PD-L1 and CD40 in Example 15 of the present disclosure.
FIG. 9 shows the PD-L1/PD-1 blocking activity of the bispecific antibody as determined by the reporter gene assay in Example 16 of the present disclosure.
FIG. 10 shows the CD40 agonistic activity of the bispecific antibody as determined by the reporter gene assay in Example 17 of the present disclosure, among which FIG. 10A shows the CD40 agonistic activity of 1605, 1606, and 1607; FIG. 10B shows the CD40 agonistic activity of 1608 and 1609; FIG. 10C shows the CD40 agonistic activity of 1652 and 1653; FIG. 10D shows the CD40 agonistic activity of 1654, 1655, and CP-870893.
FIG. 11 shows the DC regulatory activity of the bispecific antibody in Example 19, among which FIG. 11A compares the activity of different bispecific antibodies, using IL-12 p40 as an indicator; FIGs. 11B and 11C compare the activity of the parental monoclonal antibody and the bispecific antibody, with FIG. 11B showing the activity of CD83 and FIG. 11C showing the activity of IL-12 p40.
FIGs. 12A and 12B show the T cell regulatory activity of the bispecific antibody in Example 20 of the present disclosure.
FIG. 13 shows the efficacy of the bispecific antibody against MC38 tumors in mice in Example 21 of the present disclosure.
FIG. 14 shows the *in vivo* efficacy of the anti-PD-L1/CD40 bispecific antibody against MC38/hPD-L1 colon cancer.

### DETAILED DESCRIPTION OF THE PREFERRED EMBODIMENT

The present disclosure is further illustrated below by means of examples, but the present disclosure is not thereby limited to the scope of the examples. The experimental methods for which specific conditions are not indicated in the following examples are selected according to conventional methods and conditions, or according to the product instructions. The reagents whose specific sources are not indicated are conventional reagents that are commercially available.

### Example 1: Establishment of CD40 antibody and PD-L1 antibody phage libraries

Based on the crystal structure of human CD40 protein, a variable region sequence of human CD40 antibody was designed, and a single chain Fv sequence (VL-G4S linker-VH) was obtained by gene synthesis technology. The sequence was inserted into a phage vector (pComb3XSS, purchased from Beijing Zoman Biotechnology Co., Ltd.) using a restriction endonuclease, resulting in a recombinant plasmid library. The phage plasmid carrying the scFv gene was electroporated into SS320 *E. coli* competent cells. Once SS320 *E. coli* (purchased from Lucigen) proliferated to the logarithmic phase, a helper phage (M13K07, purchased from NEB) was added for infection, followed by overnight incubation. The phage was then extracted from the culture supernatant, resulting in a CD40 antibody phage library.

Based on the crystal structure of human PD-L1 protein, a variable region sequence of human PD-L1 nanobody was designed, and a VHH sequence was obtained by gene synthesis technology. The sequence was inserted into a phage vector using a restriction endonuclease, resulting in a recombinant plasmid library. The phage plasmid carrying the VHH gene was electroporated into SS320 *E*. *coli* competent cells. Once SS320 proliferated to the logarithmic phase, a helper phage was added for infection, followed by overnight incubation. The phage was then extracted from the culture supernatant, resulting in a PD-L1 antibody phage library.

### Example 2: Screening of CD40 and PD-L1 antibodies from antibody phage libraries

CD40 antibody was screened from the CD40 antibody phage library and PD-L1 antibody was screened from the PD-L1 antibody phage library.

Three rounds of panning of phage display libraries were performed using protein-based phage antibody panning technology. The first round of screening was performed as follows: human CD40-mFc antigen or human PD-L1-mFc antigen (purchased from ACROBiosystems) was coated on an enzyme-linked immunosorbent assay (ELISA) plate. The corresponding phage library was premixed with an equal volume of 2% skim milk for incubation, and the premix was added to the coated wells for reaction. After reaction, the wells were washed with sterile PBST to remove the premix. The adsorbed phages in the well plate were eluted using 75 mM sodium citrate buffer. After neutralization of the phage library, the screened phage library was amplified 100-fold using M13K07 helper phage. The second and third rounds of screening were then performed in a similar manner to the first round of screening. After three rounds of screening, an enriched phage library was obtained. The enrichment of phages was monitored by the initial phage amount of each round of screening and the phage titer collected after screening.

SS320 *E. coli* was infected with the enriched CD40 phage library and PD-L1 phage library, respectively, and then spread on an agarose plate for incubation. The monoclonal colonies were picked and placed in a 96-well deep-well plate with 2YT medium containing ampicillin and kanamycin, and incubated with shaking at 37°C to obtain a supernatant containing monoclonal phages. The monoclonal phage supernatant was incubated with the ELISA plate coated with human CD40-mFc antigen or human PD-L1-mFc antigen for 1 hour, followed by washing with sterile PBST. Anti-M13-HRP (purchased from Sino Biological) was then added, and the mixture was incubated at 4°C for 30 minutes. Subsequently, the binding of phage to antigen was detected using a microplate reader, and CD40 monoclonal phage and PD-L1 monoclonal phage with high binding to the antigen were screened.

### Example 3: Preparation of recombinant CD40 monoclonal antibody and recombinant PD-L1 monoclonal antibody

The cDNA sequences of the heavy and light chain variable regions of the screened CD40 monoclonal phages were cloned into the pcDNA3.4 vector (Invitrogen) which already contained the antibody constant region, resulting in several anti-CD40 monoclonal antibodies, named 1605CD, 1606CD, 1607CD, 1608CD, 1609CD, 1652CD, 1653CD, 1654CD, and 1655CD. The heavy and light chain recombinant plasmids were transiently co-transfected into EXPI-293 cells (Invitrogen) using the PEI method for 7 to 10 days, followed by centrifugation to collect the supernatant. The supernatant was purified by protein A to obtain purified anti-CD40 monoclonal antibodies. The CDR sequences of the CD40 antibodies are referenced in Table 7 (determined by the Kabat CDR system); the amino acid sequences of the heavy and light chain variable regions are referenced in Table 8.

**Exemplarily, the full-length amino acid sequences of 1654CD are as follows:**
Amino acid sequence of the heavy chain of 1654CD (SEQ ID NO: 94): 
EVQLVESGGGLVQPGGSLRLSCAASGFTFSNYYMSWVRQAPGKGLEWVGFI
RNKANAYTTEYAASVKGRFTISRDNSKSTLYLQMNRLRAEDTAVYYCARYGGLKVG
WYFDLWGQGTLVTVSSASTKGPSVFPLAPCSRSTSESTAALGCLVKDYFPEPVTVSW
NSGALTSGVHTFPAVLQSSGLYSLSSVVTVPSSNFGTQTYTCNVDHKPSNTKVDKTVE
RKCCVECPPCPAPPVAGPSVFLFPPKPKDTLMISRTPEVTCVVVDVSHEDPEVQFNWY
VDGVEVHNAKTKPREEQFNSTFRVVSVLTVVHQDWLNGKEYKCKVSNKGLPAPIEK
TISKTKGQPREPQVYTLPPSREEMTKNQVSLTCLVKGFYPSDIAVEWESNGQPENNYK
TTPPMLDSDGSFFLYSKLTVDKSRWQQGNVFSCSVMHEALHNHYTQKSLSLSPG
Amino acid sequence of the light chain of 1654CD (SEQ ID NO: 95):

DIQMTQSPSSLSASVGDRVTITCRASQSISNYLAWYQQKPGKAPKLLIYAASS
LQSGVPSRFSGSGSGTDYTLTISSLQPEDFATYYCQQGSSYPWTFGGGTKVEIKRTVAA
PSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC

**The full-length amino acid sequences of 1606CD are as follows:**
Amino acid sequence of the heavy chain of 1606CD (SEQ ID NO: 96): 
EVQLVESGGGLVQPGGSLRLSCAASGFTFSDYYMSWVRQAPGKGLEWVGFI
RNKANGYTTEYAASVKGRFTISRDNSKNTLYLQMNSLRAEDTAVYYCARYGGLRQG
WYFDVWGQGTLVTVSSASTKGPSVFPLAPCSRSTSESTAALGCLVKDYFPEPVTVSW
NSGALTSGVHTFPAVLQSSGLYSLSSVVTVPSSNFGTQTYTCNVDHKPSNTKVDKTVE
RKCCVECPPCPAPPVAGPSVFLFPPKPKDTLMISRTPEVTCVVVDVSHEDPEVQFNWY
VDGVEVHNAKTKPREEQFNSTFRVVSVLTVVHQDWLNGKEYKCKVSNKGLPAPIEK
TISKTKGQPREPQVYTLPPSREEMTKNQVSLTCLVKGFYPSDIAVEWESNGQPENNYK
TTPPMLDSDGSFFLYSKLTVDKSRWQQGNVFSCSVMHEALHNHYTQKSLSLSPG
Amino acid sequence of the light chain of 1606CD (SEQ ID NO: 81):

DIQMTQSPSSLSASVGDRVTITCRASQDIRNYLNWYQQKPGKAPKLLIYYTS
RRDSGVPSRFSGSGSGTDYTLTISSLQPEDFATYFCQQGKTLPWTFGGGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

**The full-length amino acid sequences of 1652CD are as follows:**
Amino acid sequence of the heavy chain of 1652CD (SEQ ID NO: 87): 
EVQLVESGGGLVQPGGSLRLSCAASGFTFSDYYMSWVRQAPGKGLEWVGFI
RNKANAYTTEYAASVKGRFTISRDNSKNTLYLQMNSLRAEDTAVYYCARYGGLRQG
WYFDVWGQGTLVTVSSASTKGPSVFPLAPCSRSTSESTAALGCLVKDYFPEPVTVSW
NSGALTSGVHTFPAVLQSSGLYSLSSVVTVPSSNFGTQTYTCNVDHKPSNTKVDKTVE
RKCCVECPPCPAPPVAGPSVFLFPPKPKDTLMISRTPEVTCVVVDVSHEDPEVQFNWY
VDGVEVHNAKTKPREEQFNSTFRVVSVLTVVHQDWLNGKEYKCKVSNKGLPAPIEK
TISKTKGQPREPQVYTLPPSREEMTKNQVSLTCLVKGFYPSDIAVEWESNGQPENNYK
TTPPMLDSDGSFFLYSKLTVDKSRWQQGNVFSCSVMHEALHNHYTQKSLSLSPG
Amino acid sequence of the light chain of 1652CD (SEQ ID NO: 81):

DIQMTQSPSSLSASVGDRVTITCRASQDIRNYLNWYQQKPGKAPKLLIYYTS
RRDSGVPSRFSGSGSGTDYTLTISSLQPEDFATYFCQQGKTLPWTFGGGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

The cDNA sequences (VHH) of the variable regions of the screened PD-L1 monoclonal phages were cloned into the pcDNA3.4 vector (Invitrogen) which already contained the antibody constant region, resulting in several anti-PD-L1 monoclonal antibodies, named 1029, 1031, 1102, and 1541. The complementary determining region (CDR) sequences of the PD-L1 monoclonal antibodies are shown in Table 2 (determined by the Kabat CDR system), the framework region (FR) sequences of the monoclonal antibodies are shown in Table 3, the amino acid sequences of the variable regions (VHH) are shown in Table 4, the amino acid sequences of the linker and constant region of the monoclonal antibodies are shown in Table 5, and the full-length amino acid sequences of the monoclonal antibodies are shown in Table 6. The plasmids were transiently co-transfected into EXPI-293 cells (Invitrogen) using the PEI method for 7 to 10 days, followed by centrifugation to collect the supernatant. The supernatant was purified by protein A to obtain purified anti-PD-L1 monoclonal antibodies.

**Table 2: CDR sequences of PD-L1 monoclonal antibodies**

| | HCDR1 | HCDR2 | HCDR3 |
|---|---|---|---|
| 1029 | DYYSKC | CIGSSDGSTY | RNGGPLTIENFFDY |
| SEQ ID NO: | 67 | 68 | 69 |
| 1031 | **E**YY**T**KC | CI**ST**S**E**GSTY | R**K**GGPLTIENFFDY |
| SEQ ID NO: | 70 | 71 | 48 |
| 1102 | DYY**TQ**C | CI**S**SS**E**GSTY | R**K**GGPLTIENFFDY |
| SEQ ID NO: | 46 | 47 | 48 |
| 1541 | DYY**TQ**C | CI**S**SS**E**GSTY | R**K**GGPLTIENFFDY |
| SEQ ID NO: | 46 | 47 | 48 |
| Superordinate sequence | X₄₁YYX₄₂X₄₃C | CIX₄₄X₄₅SX₄₆GSTY | RX₄₇GGPLTIENFFDY |
| SEQ ID NO: | / | 76 | 77 |

Note: The bolded and underlined portions in the table are the amino acids different from those of 1029, wherein X₄₁ is D or E; X₄₂ is S or T; X₄₃ is K or Q; X₄₄ is G or S; X₄₅ is S or T; X₄₆ is D or E; X₄₇ is K or N.

**Table 3: FR sequences of PD-L1 monoclonal antibodies**

| | FR1 | FR2 | FR3 | FR4 |
|---|---|---|---|---|
| 1029 | EVQLVESGGGLVQ PGGSLRLSCAASGF TL | WFRQAPGKER EWVS | YADSVKGRFTISRDN AKNTVYLQMNSLR AEDTAVYYCAA | WGQGTQVT VSS |
| SEQ ID NO: | 78 | 79 | 80 | 84 |
| 1031 | EVQLVESGGGLVQ PGGSLRLSCAASGF TF | WFRQAPGKER EWVS | YADSVKGRFTISRDN AKNTVYLQMNSLR AEDTAVYYCAA | WGQGTQVT VSS |
| SEQ ID NO: | 82 | 79 | 80 | 84 |
| 1102 | EVQLVESGGGLVQ PGGSLRLSCAASGF TF | WFRQAPGKER EWVS | YADSVKGRFTISRDN AKNTVYLQMNSLR AEDTAVYYCAA | WGQGTQVT VSS |
| SEQ ID NO: | 82 | 79 | 80 | 84 |
| 1541 | EVQLVESGGGLVQ PGGSLRLSCAASGF TF | WFRQAPGKER EWVS | YADSVKGRFTISRDN SKNTVYLQMNSLKT EDTAVYYCAA | WGQGTQVT VSS |
| SEQ ID NO: | 82 | 79 | 83 | 84 |
| Superordinate sequence | EVQLVESGGGLVQ PGGSLRLSCAASGF TX48 | WFRQAPGKER EWVS | YADSVKGRFTISRDN X49KNTVYLQMNSL X50X51EDTAVYYCAA | WGQGTQVT VSS |
| SEQ ID NO: | 85 | 79 | 86 | 84 |

Note: The bolded and underlined portions in the table are the amino acids different from those of 1029, wherein X₄₁ is L or F; X₄₉ is A or S; X₅₀ is K or R; X₅₁ is A or T.

**Table 4: Amino acid sequences of VHH of PD-L1 monoclonal antibodies**

| | Amino acid sequence SEQ ID NO: | Amino acid sequence |
|---|---|---|
| 1029 sequence | 72 | EVQLVESGGGLVQPGGSLRLSCAASGFTLDYYSKCWFRQAPGK EREWVSCIGSSDGSTYYADSVKGRFTISRDNAKNTVYLQMNSL RAEDTAVYYCAARNGGPLTIENFFDYWGQGTQVTVSS |
| 1031 sequence | 73 | EVQLVESGGGLVQPGGSLRLSCAASGFTFEYYTKCWFRQAPGK EREWVSCISTSEGSTYYADSVKGRFTISRDNAKNTVYLQMNSL RAEDTAVYYCAARKGGPLTIENFFDYWGQGTQVTVSS |
| 1102 sequence | 74 | EVQLVESGGGLVQPGGSLRLSCAASGFTFDYYTQCWFRQAPG KEREWVSCISSSEGSTYYADSVKGRFTISRDNAKNTVYLQMNS LRAEDTAVYYCAARKGGPLTIENFFDYWGQGTQVTVSS |
| 1541 sequence | 49 | EVQLVESGGGLVQPGGSLRLSCAASGFTFDYYTQCWFRQAPG KEREWVSCISSSEGSTYYADSVKGRFTISRDNSKNTVYLQMNSL KTEDTAVYYCAARKGGPLTIENFFDYWGQGTQVTVSS |

Note: The italic and underlined portions in the table are the CDR sequences described above; the bold and underlined portions are the amino acids different from those of 1029.

Amino acid sequence of the general formula of VHH (SEQ ID NO: 66):
EVQLVESGGGLVQPGGSLRLSCAASGFTX₄₈X₄₁YYX₄₂X₄₃CWFRQAPGKERE WVSCIX₄₄X₄₅SX₄₆GSTYYADSVKGRFTISRDNX₄₉KNTVYLQMNSLX₅₀X₅₁EDTAVYYC AARX₄₇GGPLTIENFFDYWGQGTQVTVSS
wherein X₄₁ is D or E; X₄₂ is S or T; X₄₃ is K or Q; X₄₄ is G or S; X₄₅ is S or T; X₄₆ is D or E; X₄₇ is K or N; X₄₈ is L or F; X₄₉ is A or S; X₅₀ is K or R; X₅₁ is A or T.

**Table 5: Amino acid sequences of linker and constant region of PD-L1 monoclonal antibodies**

| | Amino acid sequence SEQ ID NO: | Amino acid sequence |
|---|---|---|
| Heavy chain constant region sequence | 88 | EPKSCDKTHTCPPCPAPELLGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTIS KAKGQPREPQVYTLPPSREEMTKNQVSLTCLVKGFYPSDIA VEWESNGQPENNYKTTPPVLDSDGSFFLYSKLTVDKSRWQ QGNVFSCSVMHEALHNHYTQKSLSLSPG |
| Linker sequence | 89 | GGGGSGGGGSGGGGSG |

**Table 6: Full-length amino acid sequences of PD-L1 monoclonal antibodies**

| | Amino acid sequence SEQ ID NO: | Amino acid sequence |
|---|---|---|
| 1029 sequence | 90 | EVQLVESGGGLVQPGGSLRLSCAASGFTLDYYSKCWFRQAPGKE REWVSCIGSSDGSTYYADSVKGRFTISRDNAKNTVYLQMNSLRA EDTAVYYCAARNGGPLTIENFFDYWGQGTQVTVSSGGGGSGGGG SGGGGSGEPKSCDKTHTCPPCPAPELLGGPSVFLFPPKPKDTLMIS RTPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNGQ PENNYKTTPPVLDSDGSFFLYSKLTVDKSRWQQGNVFSCSVMHE ALHNHYTQKSLSLSPG |
| 1031 sequence | 91 | EVQLVESGGGLVQPGGSLRLSCAASGFTFEYYTKCWFRQAPGKE REWVSCISTSEGSTYYADSVKGRFTISRDNAKNTVYLQMNSLRAE DTAVYYCAARKGGPLTIENFFDYWGQGTQVTVSSGGGGSGGGGS GGGGSGEPKSCDKTHTCPPCPAPELLGGPSVFLFPPKPKDTLMISR TPEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSREEMTKNQVSLTCLVKGFYPSDIAVEWESNGQP ENNYKTTPPVLDSDGSFFLYSKLTVDKSRWQQGNVFSCSVMHEA LHNHYTQKSLSLSPG |
| 1102 sequence | 92 | EVQLVESGGGLVQPGGSLRLSCAASGFTFDYYTQCWFRQAPGKE REWVSCISSSEGSTYYADSVKGRFTISRDNAKNTVYLQMNSLRAE DTAVYYCAARKGGPLTIENFFDYWGQGTQVTVSSGGGGSGGGGS GGGGSGEPKSCDKTHTCPPCPAPELLGGPSVFLFPPKPKDTLMISR TPEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSREEMTKNQVSLTCLVKGFYPSDIAVEWESNGQP ENNYKTTPPVLDSDGSFFLYSKLTVDKSRWQQGNVFSCSVMHEA LHNHYTQKSLSLSPG |
| 1541 sequence | 93 | EVQLVESGGGLVQPGGSLRLSCAASGFTFDYYTQCWFRQAPGKE REWVSCISSSEGSTYYADSVKGRFTISRDNSKNTVYLQMNSLKTE DTAVYYCAARKGGPLTIENFFDYWGQGTQVTVSSGGGGSGGGGS GGGGSGEPKSCDKTHTCPPCPAPELLGGPSVFLFPPKPKDTLMISR TPEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSREEMTKNQVSLTCLVKGFYPSDIAVEWESNGQP ENNYKTTPPVLDSDGSFFLYSKLTVDKSRWQQGNVFSCSVMHEA LHNHYTQKSLSLSPG |

### Example 4: Preparation of recombinant anti-PD-L1/CD40 bispecific antibody

The recombinant anti-PD-L1/CD40 bispecific antibody was prepared using PD-L1 monoclonal antibody 1541 and CD40 monoclonal antibodies 1605CD, 1606CD, 1607CD, 1608CD, 1609CD, 1652CD, 1653CD, 1654CD, and 1655CD.

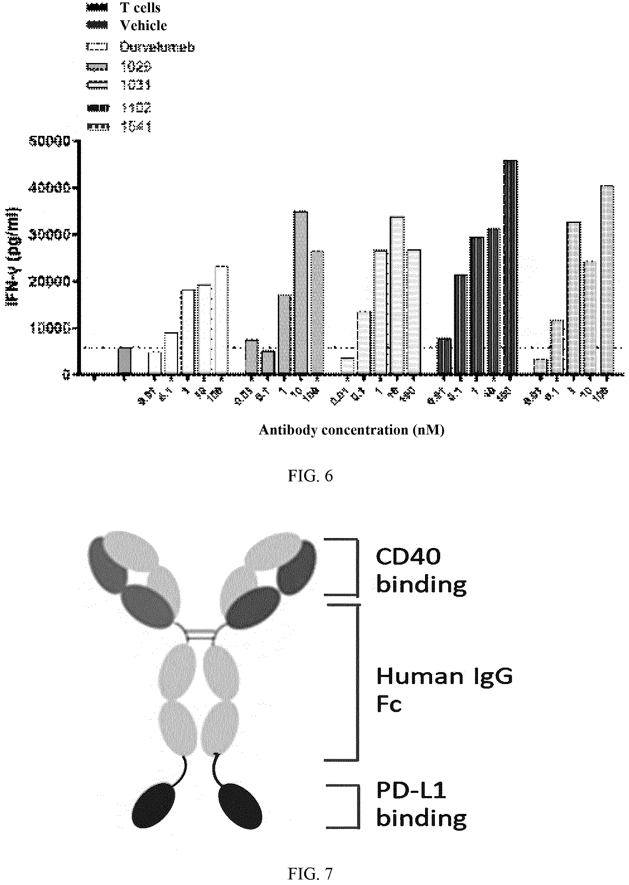

The cDNA sequences of the heavy and light chain variable regions of the screened CD40 monoclonal phages were cloned into the pcDNA3.4 vector (Invitrogen) which already contained the antibody constant region. The cDNA sequences (VHH) of the variable regions of the screened PD-L1 monoclonal phages were cloned into the pcDNA3.4 vector (Invitrogen) which already contained CD40-VH and the antibody constant region. The VHH sequence was inserted into the C-terminus of the antibody constant region, with a (G4S) linker introduced between the constant region and VHH, resulting in several anti-PD-L1/CD40 bispecific antibodies (structure as shown in FIG. 7), named 1605, 1606, 1607, 1608, 1609, 1652, 1653, 1654, and 1655. The CDR sequences of the bispecific antibodies are shown in Table 7 (determined by the Kabat CDR system); the amino acid sequences of the heavy and light chain variable regions are shown in Table 8; the amino acid sequences of the heavy and light chains of the bispecific antibodies are shown in Table 9. The heavy and light chain recombinant plasmids were transiently co-transfected into EXPI-293 cells (Invitrogen) using the PEI method for 7 to 10 days, followed by centrifugation to collect the supernatant. The supernatant was purified by protein A to obtain purified anti-PD-L1/CD40 bispecific antibodies.

**Table 7: CDR sequences of bispecific antibodies**

| | Anti-CD40 | | | | | | Anti-PD-L1 | | |
|---|---|---|---|---|---|---|---|---|---|
| | LCDR1 | LCDR2 | LCDR3 | HCDR1 | HCDR2 | HCDR3 | VHH-CDR1 | VHH-CDR2 | VHH-CDR3 |
| Shared sequence | RASQX1IX2 X3YLX4 | X5X6SX7X8 X9S | QQGX10X11 X12PW | SX13YY MS | FIRNKAN X14YT | YGGX15X16X17 GWYFDX18 | DYYTQC **(SEQ ID NO: 46)** | CISSSE GSTY (SEQ ID NO: 47) | RKGGP LTIENF FDY (SEQ ID NO: 48) |
| Shared sequence SEQ ID NO: | 1 | / | 3 | 4 | 5 | 6 | | | |
| 1605 | RASQDIRN YLN | YTSRLQS | QQGQSFP W | SNYYMS | FIRNKAN AYT | YGGIRVGWYF DL | | | |
| 1605 SEQ ID NO: | 7 | 11 | 16 | 21 | 23 | 25 | | | |
| 1606 | RASQDIRN YLN | YTSRRDS | QQGKTLP W | SDYYMS | FIRNKAN GYT | YGGLRQGWY FDV | | | |
| 1606 SEQ ID NO: | 7 | 12 | 17 | 22 | 24 | 26 | | | |
| 1607 | RASQGIRN YLN | YTSSLQS | QQGINLPW | SDYYMS | FIRNKAN GYT | YGGLKKGWY FDL | | | |
| 1607 SEQ ID NO: | 8 | 13 | 18 | 22 | 24 | 27 | | | |
| 1608 | RASQSISN YLA | AASSLQS | QQGSSYP W | SNYYMS | FIRNKAN GYT | YGGLKVGWY FDL | | | |
| 1608 SEQ ID NO: | 9 | 14 | 19 | 21 | 24 | 28 | | | |
| 1609 | RASQTISS YLN | YTSTLQS | QQGNAYP W | SNYYMS | FIRNKAN GYT | YGGLKIGWYF DL | | | |
| 1609CD SEQ ID NO: | 10 | 15 | 20 | 21 | 24 | 29 | | | |
| 1652 | RASQDIRN YLN | YTSRRDS | QQGKTLP W | SDYYMS | FIRNKAN AYT | YGGLRQGWY FDV | | | |
| 1652 SEQ ID NO: | 7 | 12 | 17 | 22 | 23 | 26 | | | |
| 1653 | RASQGIRN YLN | YTSSLQS | QQGINLPW | SDYYMS | FIRNKAN AYT | YGGLKKGWY FDL | | | |
| 1653 SEQ ID NO: | 8 | 13 | 18 | 22 | 23 | 27 | | | |
| 1654 | RASQSISN YLA | AASSLQS | QQGSSYP W | SNYYMS | FIRNKAN AYT | YGGLKVGWY FDL | | | |
| 1654 SEQ ID NO: | 9 | 14 | 19 | 21 | 23 | 28 | | | |
| 1655 | RASQTISS YLN | YTSTLQS | QQGNAYP W | SNYYMS | FIRNKAN AYT | YGGLKIGWYF DL | | | |
| 1655 SEQ ID NO: | 10 | 15 | 20 | 21 | 23 | 29 | | | |

Note: In RASQX₁IX₂X₃YLX₄ (SEQ ID NO: 1), X₁ is D, G, S, or T; X₂ is R or S; X₃ is N or S; X₄ is N or A. In X₅X₆SX₇X₈X₉S, X₅ is Y or A; X₆ is T or A; X₇ is S, R, or T; X₈ is L or R; X₉ is Q or D. In QQGX₁₀X₁₁X₁₂PW (SEQ ID NO: 3), X₁₀ is K, I, N, Q, or S; X₁₁ is S, A, N, or T; X₁₂ is L, Y, or F. In SX₁₃YYMS (SEQ ID NO: 4), X₁₃ is N or D. In FIRNKANX₁₄YT (SEQ ID NO: 5), X₁₄ is A or G. In YGGX₁₅X₁₆X₁₇GWYFDX₁₈ (SEQ ID NO: 6), X₁₅ is L or I; X₁₆ is K or R; X₁₇ is V, K, I, or Q; X₁₈ is L or V

**Table 8: Amino acid sequences of heavy and light chain variable regions of bispecific antibodies**

| Anti-CD40 | Amino acid sequence SEQ ID NO: | Amino acid sequence |
|---|---|---|
| 1605 light chain variable region | 30 | DIQMTQSPSSLSASVGDRVTITCRASQDIRNYLNWYQQKPGK APKLLIYYTSRLQSGVPSRFSGSGSGTDYTLTISSLQPEDFATY YCQQGQSFPWTFGGGTKVEIK |
| 1605 heavy chain variable region | 31 | EVQLVESGGGLVQPGGSLRLSCAASGFTFSNYYMSWVRQAP GKGLEWVAFIRNKANAYTTEYAASVKGRFTISRDNSKSTLYL QMNSLRAEDTAVYYCARYGGIRVGWYFDLWGQGTLVTVSS |
| 1606 light chain variable region | 32 | DIQMTQSPSSLSASVGDRVTITCRASQDIRNYLNWYQQKPGK APKLLIYYTSRRDSGVPSRFSGSGSGTDYTLTISSLQPEDFATY FCQQGKTLPWTFGGGTKVEIK |
| 1606 heavy chain variable region | 33 | EVQLVESGGGLVQPGGSLRLSCAASGFTFSDYYMSWVRQAP GKGLEWVGFIRNKANGYTTEYAASVKGRFTISRDNSKNTLY LQMNSLRAEDTAVYYCARYGGLRQGWYFDVWGQGTLVTV SS |
| 1607 light chain variable region | 35 | DIQMTQSPSSLSASVGDRVTITCRASQGIRNYLNWYQQKPGK APKLLIYYTSSLQSGVPSRFSGSGSGTDYTLTISSLQPEDFATY YCQQGINLPWTFGGGTKVEIK |
| 1607 heavy chain variable region | 36 | EVQLVESGGGLVQPGGSLRLSCAASGFTFSDYYMSWVRQAP GKGLEWVGFIRNKANGYTTEYAASVKGRFTISRDNSKSTLYL QMNSLRAEDTAVYYCARYGGLKKGWYFDLWGQGTLVTVSS |
| 1608 light chain variable region | 38 | DIQMTQSPSSLSASVGDRVTITCRASQSISNYLAWYQQKPGK APKLLIYAASSLQSGVPSRFSGSGSGTDYTLTISSLQPEDFATY YCQQGSSYPWTFGGGTKVEIK |
| 1608 heavy chain variable region | 39 | EVQLVESGGGLVQPGGSLRLSCAASGFTFSNYYMSWVRQAP GKGLEWVGFIRNKANGYTTEYAASVKGRFTISRDNSKSTLYL QMNRLRAEDTAVYYCARYGGLKVGWYFDLWGQGTLVTVS S |
| 1609 light chain variable region | 41 | DIQMTQSPSSLSASVGDRVTITCRASQTISSYLNWYQQKPGK APKLLIYYTSTLQSGVPSRFSGSGSGTDYTLTISSLQPEDFATY YCQQGNAYPWTFGGGTKVEIK |
| 1609 heavy chain variable region | 42 | EVQLVESGGGLVQPGGSLRLSCAASGFTFSNYYMSWVRQAP GKGLEWVGFIRNKANGYTTEYAASVKGRFTISRDNSKSTLYL QMNSLRAEDTAVYYCARYGGLKIGWYFDLWGQGTLVTVSS |
| 1652 light chain variable region | 32 | DIQMTQSPSSLSASVGDRVTITCRASQDIRNYLNWYQQKPGK APKLLIYYTSRRDSGVPSRFSGSGSGTDYTLTISSLQPEDFATY FCQQGKTLPWTFGGGTKVEIK |
| 1652 heavy chain variable region | 34 | EVQLVESGGGLVQPGGSLRLSCAASGFTFSDYYMSWVRQAP |
| | | GKGLEWVGFIRNKANAYTTEYAASVKGRFTISRDNSKNTLY LQMNSLRAEDTAVYYCARYGGLRQGWYFDVWGQGTLVTV SS |
| 1653 light chain variable region | 35 | DIQMTQSPSSLSASVGDRVTITCRASQGIRNYLNWYQQKPGK APKLLIYYTSSLQSGVPSRFSGSGSGTDYTLTISSLQPEDFATY YCQQGINLPWTFGGGTKVEIK |
| 1653 heavy chain variable region | 37 | EVQLVESGGGLVQPGGSLRLSCAASGFTFSDYYMSWVRQAP GKGLEWVGFIRNKANAYTTEYAASVKGRFTISRDNSKSTLYL QMNSLRAEDTAVYYCARYGGLKKGWYFDLWGQGTLVTVSS |
| 1654 light chain variable region | 38 | DIQMTQSPSSLSASVGDRVTITCRASQSISNYLAWYQQKPGK APKLLIYAASSLQSGVPSRFSGSGSGTDYTLTISSLQPEDFATY YCQQGSSYPWTFGGGTKVEIK |
| 1654 heavy chain variable region | 40 | EVQLVESGGGLVQPGGSLRLSCAASGFTFSNYYMSWVRQAP GKGLEWVGFIRNKANAYTTEYAASVKGRFTISRDNSKSTLYL QMNRLRAEDTAVYYCARYGGLKVGWYFDLWGQGTLVTVS S |
| 1655 light chain variable region | 41 | DIQMTQSPSSLSASVGDRVTITCRASQTISSYLNWYQQKPGK APKLLIYYTSTLQSGVPSRFSGSGSGTDYTLTISSLQPEDFATY YCQQGNAYPWTFGGGTKVEIK |
| 1655 heavy chain variable region | 43 | EVQLVESGGGLVQPGGSLRLSCAASGFTFSNYYMSWVRQAP GKGLEWVGFIRNKANAYTTEYAASVKGRFTISRDNSKSTLYL QMNSLRAEDTAVYYCARYGGLKIGWYFDLWGQGTLVTVSS |
| Anti-CD40 light chain constant region sequence | 44 | |
| Anti-CD40 heavy chain constant region sequence | 45 | ASTKGPSVFPLAPCSRSTSESTAALGCLVKDYFPEPVTVSWNS GALTSGVHTFPAVLQSSGLYSLSSVVTVPSSNFGTQTYTCNV DHKPSNTKVDKTVERKCCVECPPCPAPPVAGPSVFLFPPKPK DTLMISRTPEVTCVVVDVSHEDPEVQFNWYVDGVEVHNAK TKPREEQFNSTFRVVSVLTVVHQDWLNGKEYKCKVSNKGLP APIEKTISKTKGQPREPQVYTLPPSREEMTKNQVSLTCLVKGF YPSDIAVEWESNGQPENNYKTTPPMLDSDGSFFLYSKLTVDK SRWQQGNVFSCSVMHEALHNHYTQKSLSLSPG |
| VHH variable region in bispecific antibodies (1541) | 49 | EVQLVESGGGLVQPGGSLRLSCAASGFTFDYYTQCWFRQAP GKEREWVSCISSSEGSTYYADSVKGRFTISRDNSKNTVYLQM NSLKTEDTAVYYCAARKGGPLTIENFFDYWGQGTQVTVSS |

The shared sequence of the heavy chain variable region of Anti-CD40 is:
EVQLVESGGGLVQPGGSLRLSCAASGFTFSX₁₉YYMSWVRQAPGKGLEWVX₂ oFIRNKANX₂₁YTTEYAASVKGRFTISRDNSKX₂₂TLYLQMNX₂₃LRAEDTAVYYCARYG GX₂₄X₂₅X₂₆GWYFDX₂₇WGQGTLVTVSS (SEQ ID NO: 64): X₁₉ is N or D; X₂₀ is G or A; X₂₁ is A or G; X₂₂ is N or S; X₂₃ is R or S; X₂₄ is L or I; X₂₅ is K or R; X₂₆ is V, K, I, or Q; X₂₇ is L or V.

The shared sequence of the light chain variable region of Anti-CD40 is (SEQ ID NO: 65):
DIQMTQSPSSLSASVGDRVTITCRASQX₂₈IX₂₉X₃₀YLX₃₁WYQQKPGKAPKLLI YX₃₂X₃₃SX₃₄X₃₅X₃₆SGVPSRFSGSGSGTDYTLTISSLQPEDFATYX₃₇CQQGX₃₈X₃₉X₄₀PW TFGGGTKVEIK: X₂₈ is D, G, S, or T; X₂₉ is R or S; X₃₀ is N or S; X₃₁ is N or A; X₃₂ is Y or A; X₃₃ is T or A; X₃₄ is S, R, or T; X₃₅ is L or R; X₃₆ is Q or D; X₃₇ is Y or F; X₃₈ is K, I, N, Q, or S; X₃₉ is S, A, N, or T; X₄₀ is L, Y, or F.

**Table 9: Amino acid sequences of heavy and light chains of bispecific antibodies**

| Anti-CD40 and Anti-PD-L1 | Amino acid sequence SEQ ID NO: | Amino acid sequence |
|---|---|---|
| 1605 first polypeptide chain | 50 | DIQMTQSPSSLSASVGDRVTITCRASQDIRNYLNWYQQKPGKAPKLLIYY TSRLQSGVPSRFSGSGSGTDYTLTISSLQPEDFATYYCQQGQSFPWTFGGG TKVEIKRTVAAPSVFIFPPSDEQLKSGTASVVCLLNNFYPREAKVQWKVD NALQSGNSQESVTEQDSKDSTYSLSSTLTLSKADYEKHKVYACEVTHQGL SSPVTKSFNRGEC |
| 1605 second polypeptide chain d | 51 | EVQLVESGGGLVQPGGSLRLSCAASGFTFSNYYMSWVRQAPGKGLEWVA FIRNKANAYTTEYAASVKGRFTISRDNSKSTLYLQMNSLRAEDTAVYYCA RYGGIRVGWYFDLWGQGTLVTVSSASTKGPSVFPLAPCSRSTSESTAALG CLVKDYFPEPVTVSWNSGALTSGVHTFPAVLQSSGLYSLSSVVTVPSSNFG TQTYTCNVDHKPSNTKVDKTVERKCCVECPPCPAPPVAGPSVFLFPPKPK DTLMISRTPEVTCVVVDVSHEDPEVQFNWYVDGVEVHNAKTKPREEQFN STFRVVSVLTVVHQDWLNGKEYKCKVSNKGLPAPIEKTISKTKGQPREPQ VYTLPPSREEMTKNQVSLTCLVKGFYPSDIAVEWESNGQPENNYKTTPPM LDSDGSFFLYSKLTVDKSRWQQGNVFSCSVMHEALHNHYTQKSLSLSPG GGGGSGGGGSGGGGSGEVQLVESGGGLVQPGGSLRLSCAASGFTFDYYT QCWFRQAPGKEREWVSCISSSEGSTYYADSVKGRFTISRDNSKNTVYLQ MNSLKTEDTAVYYCAARKGGPLTIENFFDYWGQGTQVTVSS |
| 1606 first polypeptide chain | 52 | DIQMTQSPSSLSASVGDRVTITCRASQDIRNYLNWYQQKPGKAPKLLIYY TSRRDSGVPSRFSGSGSGTDYTLTISSLQPEDFATYFCQQGKTLPWTFGGG TKVEIKRTVAAPSVFIFPPSDEQLKSGTASVVCLLNNFYPREAKVQWKVD NALQSGNSQESVTEQDSKDSTYSLSSTLTLSKADYEKHKVYACEVTHQGL SSPVTKSFNRGEC |
| 1606 second polypeptide chain | 53 | EVQLVESGGGLVQPGGSLRLSCAASGFTFSDYYMSWVRQAPGKGLEWV GFIRNKANGYTTEYAASVKGRFTISRDNSKNTLYLQMNSLRAEDTAVYYC ARYGGLRQGWYFDVWGQGTLVTVSSASTKGPSVFPLAPCSRSTSESTAAL GCLVKDYFPEPVTVSWNSGALTSGVHTFPAVLQSSGLYSLSSVVTVPSSNF GTQTYTCNVDHKPSNTKVDKTVERKCCVECPPCPAPPVAGPSVFLFPPKP KDTLMISRTPEVTCVVVDVSHEDPEVQFNWYVDGVEVHNAKTKPREEQF NSTFRVVSVLTVVHQDWLNGKEYKCKVSNKGLPAPIEKTISKTKGQPREP QVYTLPPSREEMTKNQVSLTCLVKGFYPSDIAVEWESNGQPENNYKTTPP MLDSDGSFFLYSKLTVDKSRWQQGNVFSCSVMHEALHNHYTQKSLSLSP GGGGGSGGGGSGGGGSGEVQLVESGGGLVQPGGSLRLSCAASGFTFDYY TQCWFRQAPGKEREWVSCISSSEGSTYYADSVKGRFTISRDNSKNTVYLQ MNSLKTEDTAVYYCAARKGGPLTIENFFDYWGQGTQVTVSS |
| 1607 first polypeptide chain | 54 | DIQMTQSPSSLSASVGDRVTITCRASQGIRNYLNWYQQKPGKAPKLLIYY TSSLQSGVPSRFSGSGSGTDYTLTISSLQPEDFATYYCQQGINLPWTFGGGT KVEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGLS SPVTKSFNRGEC |
| 1607 second polypeptide chain | 55 | EVQLVESGGGLVQPGGSLRLSCAASGFTFSDYYMSWVRQAPGKGLEWV GFIRNKANGYTTEYAASVKGRFTISRDNSKSTLYLQMNSLRAEDTAVYYC ARYGGLKKGWYFDLWGQGTLVTVSSASTKGPSVFPLAPCSRSTSESTAAL GCLVKDYFPEPVTVSWNSGALTSGVHTFPAVLQSSGLYSLSSVVTVPSSNF GTQTYTCNVDHKPSNTKVDKTVERKCCVECPPCPAPPVAGPSVFLFPPKP KDTLMISRTPEVTCVVVDVSHEDPEVQFNWYVDGVEVHNAKTKPREEQF NSTFRVVSVLTVVHQDWLNGKEYKCKVSNKGLPAPIEKTISKTKGQPREP QVYTLPPSREEMTKNQVSLTCLVKGFYPSDIAVEWESNGQPENNYKTTPP MLDSDGSFFLYSKLTVDKSRWQQGNVFSCSVMHEALHNHYTQKSLSLSP GGGGGSGGGGSGGGGSGEVQLVESGGGLVQPGGSLRLSCAASGFTFDYY TQCWFRQAPGKEREWVSCISSSEGSTYYADSVKGRFTISRDNSKNTVYLQ MNSLKTEDTAVYYCAARKGGPLTIENFFDYWGQGTQVTVSS |
| 1608 first polypeptide chain | 56 | DIQMTQSPSSLSASVGDRVTITCRASQSISNYLAWYQQKPGKAPKLLIYAA SSLQSGVPSRFSGSGSGTDYTLTISSLQPEDFATYYCQQGSSYPWTFGGGT KVEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGLS SPVTKSFNRGEC |
| 1608 second polypeptide chain | 57 | EVQLVESGGGLVQPGGSLRLSCAASGFTFSNYYMSWVRQAPGKGLEWV GFIRNKANGYTTEYAASVKGRFTISRDNSKSTLYLQMNRLRAEDTAVYYC ARYGGLKVGWYFDLWGQGTLVTVSSASTKGPSVFPLAPCSRSTSESTAAL GCLVKDYFPEPVTVSWNSGALTSGVHTFPAVLQSSGLYSLSSVVTVPSSNF GTQTYTCNVDHKPSNTKVDKTVERKCCVECPPCPAPPVAGPSVFLFPPKP KDTLMISRTPEVTCVVVDVSHEDPEVQFNWYVDGVEVHNAKTKPREEQF NSTFRVVSVLTVVHQDWLNGKEYKCKVSNKGLPAPIEKTISKTKGQPREP QVYTLPPSREEMTKNQVSLTCLVKGFYPSDIAVEWESNGQPENNYKTTPP MLDSDGSFFLYSKLTVDKSRWQQGNVFSCSVMHEALHNHYTQKSLSLSP GGGGGSGGGGSGGGGSGEVQLVESGGGLVQPGGSLRLSCAASGFTFDYY |
| | | TQCWFRQAPGKEREWVSCISSSEGSTYYADSVKGRFTISRDNSKNTVYLQ MNSLKTEDTAVYYCAARKGGPLTIENFFDYWGQGTQVTVSS |
| 1609 first polypeptide chain | 58 | DIQMTQSPSSLSASVGDRVTITCRASQTISSYLNWYQQKPGKAPKLLIYYT STLQSGVPSRFSGSGSGTDYTLTISSLQPEDFATYYCQQGNAYPWTFGGGT KVEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGLS SPVTKSFNRGEC |
| 1609 second polypeptide chain | 59 | EVQLVESGGGLVQPGGSLRLSCAASGFTFSNYYMSWVRQAPGKGLEWV GFIRNKANGYTTEYAASVKGRFTISRDNSKSTLYLQMNSLRAEDTAVYYC ARYGGLKIGWYFDLWGQGTLVTVSSASTKGPSVFPLAPCSRSTSESTAAL GCLVKDYFPEPVTVSWNSGALTSGVHTFPAVLQSSGLYSLSSVVTVPSSNF GTQTYTCNVDHKPSNTKVDKTVERKCCVECPPCPAPPVAGPSVFLFPPKP KDTLMISRTPEVTCVVVDVSHEDPEVQFNWYVDGVEVHNAKTKPREEQF NSTFRVVSVLTVVHQDWLNGKEYKCKVSNKGLPAPIEKTISKTKGQPREP QVYTLPPSREEMTKNQVSLTCLVKGFYPSDIAVEWESNGQPENNYKTTPP MLDSDGSFFLYSKLTVDKSRWQQGNVFSCSVMHEALHNHYTQKSLSLSP GGGGGSGGGGSGGGGSGEVQLVESGGGLVQPGGSLRLSCAASGFTFDYY TQCWFRQAPGKEREWVSCISSSEGSTYYADSVKGRFTISRDNSKNTVYLQ MNSLKTEDTAVYYCAARKGGPLTIENFFDYWGQGTQVTVSS |
| 1652 first polypeptide chain | 52 | DIQMTQSPSSLSASVGDRVTITCRASQDIRNYLNWYQQKPGKAPKLLIYY TSRRDSGVPSRFSGSGSGTDYTLTISSLQPEDFATYFCQQGKTLPWTFGGG TKVEIKRTVAAPSVFIFPPSDEQLKSGTASVVCLLNNFYPREAKVQWKVD NALQSGNSQESVTEQDSKDSTYSLSSTLTLSKADYEKHKVYACEVTHQGL SSPVTKSFNRGEC |
| 1652 second polypeptide chain | 60 | EVQLVESGGGLVQPGGSLRLSCAASGFTFSDYYMSWVRQAPGKGLEWV GFIRNKANAYTTEYAASVKGRFTISRDNSKNTLYLQMNSLRAEDTAVYYC ARYGGLRQGWYFDVWGQGTLVTVSSASTKGPSVFPLAPCSRSTSESTAAL GCLVKDYFPEPVTVSWNSGALTSGVHTFPAVLQSSGLYSLSSVVTVPSSNF GTQTYTCNVDHKPSNTKVDKTVERKCCVECPPCPAPPVAGPSVFLFPPKP KDTLMISRTPEVTCVVVDVSHEDPEVQFNWYVDGVEVHNAKTKPREEQF NSTFRVVSVLTVVHQDWLNGKEYKCKVSNKGLPAPIEKTISKTKGQPREP QVYTLPPSREEMTKNQVSLTCLVKGFYPSDIAVEWESNGQPENNYKTTPP MLDSDGSFFLYSKLTVDKSRWQQGNVFSCSVMHEALHNHYTQKSLSLSP GGGGGSGGGGSGGGGSGEVQLVESGGGLVQPGGSLRLSCAASGFTFDYY TQCWFRQAPGKEREWVSCISSSEGSTYYADSVKGRFTISRDNSKNTVYLQ MNSLKTEDTAVYYCAARKGGPLTIENFFDYWGQGTQVTVSS |
| 1653 first polypeptide chain | 54 | DIQMTQSPSSLSASVGDRVTITCRASQGIRNYLNWYQQKPGKAPKLLIYY TSSLQSGVPSRFSGSGSGTDYTLTISSLQPEDFATYYCQQGINLPWTFGGGT KVEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGLS SPVTKSFNRGEC |
| 1653 second polypeptide chain | 61 | EVQLVESGGGLVQPGGSLRLSCAASGFTFSDYYMSWVRQAPGKGLEWV GFIRNKANAYTTEYAASVKGRFTISRDNSKSTLYLQMNSLRAEDTAVYYC ARYGGLKKGWYFDLWGQGTLVTVSSASTKGPSVFPLAPCSRSTSESTAAL |
| | | GCLVKDYFPEPVTVSWNSGALTSGVHTFPAVLQSSGLYSLSSVVTVPSSNF GTQTYTCNVDHKPSNTKVDKTVERKCCVECPPCPAPPVAGPSVFLFPPKP KDTLMISRTPEVTCVVVDVSHEDPEVQFNWYVDGVEVHNAKTKPREEQF NSTFRVVSVLTVVHQDWLNGKEYKCKVSNKGLPAPIEKTISKTKGQPREP QVYTLPPSREEMTKNQVSLTCLVKGFYPSDIAVEWESNGQPENNYKTTPP MLDSDGSFFLYSKLTVDKSRWQQGNVFSCSVMHEALHNHYTQKSLSLSP GGGGGSGGGGSGGGGSGEVQLVESGGGLVQPGGSLRLSCAASGFTFDYY TQCWFRQAPGKEREWVSCISSSEGSTYYADSVKGRFTISRDNSKNTVYLQ MNSLKTEDTAVYYCAARKGGPLTIENFFDYWGQGTQVTVSS |
| 1654 first polypeptide chain | 56 | DIQMTQSPSSLSASVGDRVTITCRASQSISNYLAWYQQKPGKAPKLLIYAA SSLQSGVPSRFSGSGSGTDYTLTISSLQPEDFATYYCQQGSSYPWTFGGGT KVEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGLS SPVTKSFNRGEC |
| 1654 second polypeptide chain | 62 | EVQLVESGGGLVQPGGSLRLSCAASGFTFSNYYMSWVRQAPGKGLEWV GFIRNKANAYTTEYAASVKGRFTISRDNSKSTLYLQMNRLRAEDTAVYYC ARYGGLKVGWYFDLWGQGTLVTVSSASTKGPSVFPLAPCSRSTSESTAAL GCLVKDYFPEPVTVSWNSGALTSGVHTFPAVLQSSGLYSLSSVVTVPSSNF GTQTYTCNVDHKPSNTKVDKTVERKCCVECPPCPAPPVAGPSVFLFPPKP KDTLMISRTPEVTCVVVDVSHEDPEVQFNWYVDGVEVHNAKTKPREEQF NSTFRVVSVLTVVHQDWLNGKEYKCKVSNKGLPAPIEKTISKTKGQPREP QVYTLPPSREEMTKNQVSLTCLVKGFYPSDIAVEWESNGQPENNYKTTPP MLDSDGSFFLYSKLTVDKSRWQQGNVFSCSVMHEALHNHYTQKSLSLSP GGGGGSGGGGSGGGGSGEVQLVESGGGLVQPGGSLRLSCAASGFTFDYY TQCWFRQAPGKEREWVSCISSSEGSTYYADSVKGRFTISRDNSKNTVYLQ MNSLKTEDTAVYYCAARKGGPLTIENFFDYWGQGTQVTVSS |
| 1655 first polypeptide chain | 58 | DIQMTQSPSSLSASVGDRVTITCRASQTISSYLNWYQQKPGKAPKLLIYYT STLQSGVPSRFSGSGSGTDYTLTISSLQPEDFATYYCQQGNAYPWTFGGGT KVEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGLS SPVTKSFNRGEC |
| 1655 second polypeptide chain | 63 | EVQLVESGGGLVQPGGSLRLSCAASGFTFSNYYMSWVRQAPGKGLEWV GFIRNKANAYTTEYAASVKGRFTISRDNSKSTLYLQMNSLRAEDTAVYYC ARYGGLKIGWYFDLWGQGTLVTVSSASTKGPSVFPLAPCSRSTSESTAAL GCLVKDYFPEPVTVSWNSGALTSGVHTFPAVLQSSGLYSLSSVVTVPSSNF GTQTYTCNVDHKPSNTKVDKTVERKCCVECPPCPAPPVAGPSVFLFPPKP KDTLMISRTPEVTCVVVDVSHEDPEVQFNWYVDGVEVHNAKTKPREEQF NSTFRVVSVLTVVHQDWLNGKEYKCKVSNKGLPAPIEKTISKTKGQPREP QVYTLPPSREEMTKNQVSLTCLVKGFYPSDIAVEWESNGQPENNYKTTPP MLDSDGSFFLYSKLTVDKSRWQQGNVFSCSVMHEALHNHYTQKSLSLSP GGGGGSGGGGSGGGGSGEVQLVESGGGLVQPGGSLRLSCAASGFTFDYY TQCWFRQAPGKEREWVSCISSSEGSTYYADSVKGRFTISRDNSKNTVYLQ MNSLKTEDTAVYYCAARKGGPLTIENFFDYWGQGTQVTVSS |

### Example 5: Binding assay of recombinant CD40 monoclonal antibody to CD40

The binding ability of monoclonal antibody to human CD40, as well as the species specificity, was detected using an enzyme-linked immunosorbent assay (ELISA). The specific method is as follows: human CD40, cynomolgus CD40, rat CD40, and mouse CD40 antigens (all purchased from ACROBiosystems) were coated with 1 µg/mL carbonate buffer (pH = 9.6) in an ELISA plate at 100 µL per well, followed by incubation at 4°C overnight. The wells were washed 5 times with PBST. 300 µL/well of PBST containing 1% BSA was added to block the wells, and the plate was incubated at room temperature for 1 hour. The wells were washed 5 times with PBST. Monoclonal antibody serially diluted with PBST containing 1% BSA, along with control CD40 monoclonal antibody CP-870893 (IMGT database ID 10523), were added at 100 µL per well, and the plate was incubated at room temperature for 1 hour. The wells were washed 5 times with PBST. HRP-labeled anti-human IgG antibody (Jackson ImmunoResearch, Cat. No. 109-035-088) diluted with PBST containing 1% BSA was added at 100 µL per well, and the plate was incubated at room temperature for 1 hour. The wells were washed 5 times with PBST. Colorimetric substrate TMB was added at 100 µL per well and allowed to develop color at room temperature for 10 minutes, followed by the addition of 1 M sulfuric acid to terminate the reaction. The OD_{450 nm} was read on a microplate reader to analyze the results, and the EC₅₀ was calculated using a 4-parameter fit binding curve.

The results are shown in Table 10, indicating that monoclonal antibodies 1605CD, 1606CD, 1607CD, 1608CD, 1609CD, 1652CD, 1653CD, 1654CD, and 1655CD are able to bind to both human CD40 and cynomolgus CD40, and their binding ability to human and cynomolgus monkey antigens is comparable. These monoclonal antibodies are not able to bind to rat CD40 or mouse CD40.

### Example 10: Binding of monoclonal antibodies to antigens

| EC₅₀ (nM) | | | | |
|---|---|---|---|---|
| CD40 | | | | |
| Antibody name | Human | Cynomolgus | Rat | Mouse |
| CP-870893 | 0.018 | 0.018 | NB | NB |
| 1605CD | 0.022 | 0.021 | NB | NB |
| 1606CD | 0.024 | 0.020 | NB | NB |
| 1607CD | 0.021 | 0.017 | NB | NB |
| 1608CD | 0.022 | 0.021 | NB | NB |
| 1609CD | 0.022 | 0.020 | NB | NB |
| 1652CD | 0.022 | 0.018 | NB | NB |
| 1653CD | 0.020 | 0.018 | NB | NB |
| 1654CD | 0.014 | 0.015 | NB | NB |
| 1655CD | 0.024 | 0.009 | NB | NB |

### Example 6: Effect of anti-CD40 monoclonal antibody on CD40/CD40L binding

The blocking effect of monoclonal antibodies on CD40/CD40L binding was tested using HEK-Blue CD40L cells (purchased from InvivoGen) that highly express CD40. The specific method is as follows: HEK-Blue CD40L cells were collected and resuspended in pre-cooled PBS containing 2% FBS at a density of 1 × 10⁷ cells/mL, and then added to a 96-well plate at 50 µL per well, *i.e.,* 5 × 10⁵ cells/well. Biotin-CD40L (FutureGen Biopharmaceutical) and serially diluted CD40 monoclonal antibody were then added at 50 µL per well, with a final concentration of Biotin-CD40L of 10 nM, and the plate was incubated at 4°C for 1 hour. The wells were washed twice with pre-cooled PBS. Streptavidin-PE (BioLegend, Cat. No. 405203) diluted in pre-cooled PBS containing 2% FBS was added, and the plate was incubated at 4°C for 30 minutes. The wells were washed twice with pre-cooled PBS. The cells were then resuspended in pre-cooled PBS containing 2% FBS and assayed by flow cytometry.

The results are shown in Table 11, indicating that monoclonal antibodies 1605CD, 1606CD, 1607CD, 1608CD, 1609CD, 1652CD, 1653CD, 1654CD, and 1655CD hinder the CD40/CD40L interaction, while the control CD40 monoclonal antibody CP-870893 has no effect on the CD40/CD40L interaction.

### Example 11: Effect of monoclonal antibodies on CD40/CD40L binding

| Antibody name | CD40/CD40L interaction blocking |
|---|---|
| CP-870893 | No |
| 1605CD | Yes |
| 1606CD | Yes |
| 1607CD | Yes |
| 1608CD | Yes |
| 1609CD | Yes |
| 1652CD | Yes |
| 1653CD | Yes |
| 1654CD | Yes |
| 1655CD | Yes |

### Example 7: Detection of CD40 agonistic activity of anti-CD40 monoclonal antibody by reporter gene assay

The CD40 agonistic activity of monoclonal antibodies was tested using HEK-Blue CD40L cells. HEK-Blue CD40L cells, purchased from InvivoGen, highly express CD40 and contain the SEAP reporter gene under the control of NF-κB response elements. When CD40 on HEK-Blue CD40L cells is activated, it induces the activation of downstream NF-κB signaling, which in turn induces the production of SEAP. CD40 activation can be monitored by detecting the amount of secreted SEAP using QUANTI-Blue reagent (InvivoGen). The specific method is as follows: HEK-Blue CD40L cells were collected and resuspended in complete medium (RPMI 1640 containing 10% FBS) at 3 × 10⁵ cells/mL, and then evenly spread in a 96-well plate at 100 µL/well, *i.e*., 3 × 10⁴ cells per well. Anti-CD40 monoclonal antibody samples serially diluted in complete medium and the control antibody (CP-870893) were added at 100 µL/well, and the plate was incubated in a 5% CO₂ incubator at 37°C for 20 to 24 hours. After incubation, the 96-well plate was removed and centrifuged at 300 g for 5 minutes, and 40 µL/well of the supernatant was aspirated and transferred to a new 96-well plate. 160 µL/well of QUANTI-Blue reagent was added to the supernatant, and the plate was incubated in a 5% CO₂ incubator at 37°C for 20 to 30 minutes. The OD_{655 nm} was then read on a microplate reader to analyze the results.

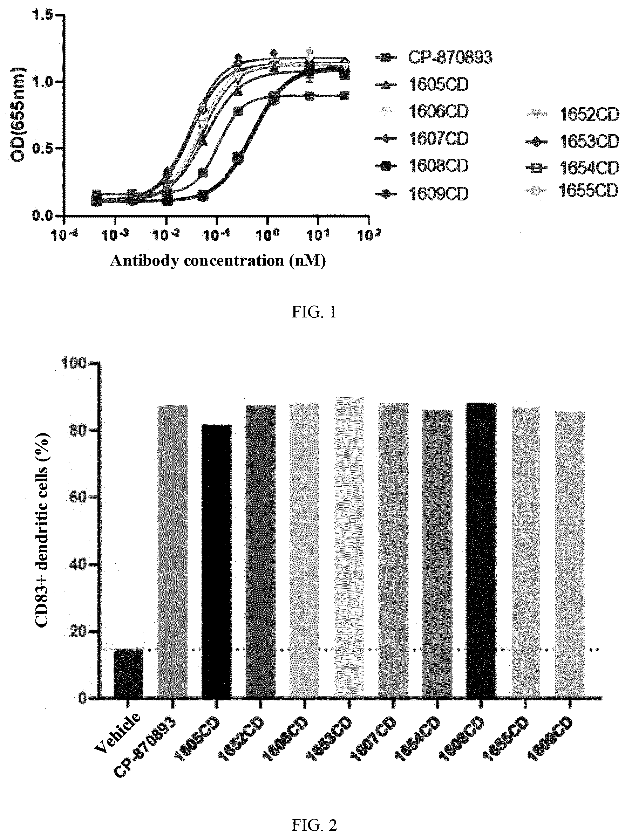

The results are shown in FIG. 1, indicating that the CD40 agonistic activity of monoclonal antibodies can be roughly divided into two categories. One category includes antibodies 1608CD, 1609CD, 1654CD, and 1655CD, which exhibit weak CD40 agonistic activity, weaker than that of the control CD40 monoclonal antibody CP-870893. The other category includes antibodies 1605CD, 1606CD, 1607CD, 1652CD, and 1653CD, which exhibit strong activity, stronger than that of the control CD40 monoclonal antibody CP-870893.

### Example 8: DC regulatory activity of anti-CD40 monoclonal antibody

The dendritic cell (DC) regulatory activity of CD40 monoclonal antibody was tested.

Donor human PBMCs were resuspended in complete medium (RPMI 1640 containing 10% FBS) and inoculated in a 10 cm cell culture dish, which was incubated in a CO₂ incubator at 37°C for 2 hours. The culture supernatant and suspended cells were discarded, leaving adherent monocytes. The monocytes were incubated in complete medium containing 100 ng/mL GM-CSF (PeproTech, Cat. No. 300-03) and 100 ng/mL IL-4 (PeproTech, Cat. No. 200-04) for 6 days, with medium exchanged every 2 days to obtain immature dendritic cells (imDCs). The imDCs were collected, resuspended in complete medium, and inoculated in a 24-well plate, followed by the addition of 100 nM antibody samples and the control antibody (CP-870893). The culture plate was incubated in a CO₂ incubator at 37°C for 2 days. After incubation, the supernatant in the wells was removed for assay of the cytokines IL-12/IL-23 p40 (R&D, Cat. No. DY1240) according to the kit instructions. Simultaneously, the cells in the well plate were collected and incubated with the detection antibody (APC anti-human CD83 antibody, Biolegend, 305312), followed by assay of CD83 expression on DC cells by flow cytometry.

FIG. 2 compares the DC regulatory activity of the anti-CD40 monoclonal antibodies of the present disclosure, showing that different monoclonal antibodies can significantly upregulate CD83 expression on DC cells.

### Example 9: T cell regulatory activity of anti-CD40 monoclonal antibody

CD40 agonistic antibodies show their most important anti-tumor biological effect through activation and empowerment of DC cells followed by T cell activation. The T cell regulatory activity of CD40 monoclonal antibodies was tested using the MLR assay system incubated with allogeneic DC cells and T cells.

DC cells were obtained using the same method as in Example 8. Allogeneic T cells were isolated from human PBMCs, following the instructions provided in the Pan T Cell Isolation Kit (Miltenyi Biotech, Cat. No. 130-096-535). Briefly, PBMCs were first washed once with PBS, and then resuspended in isolation buffer (PBS containing 2 mM EDTA and 0.5% BSA, pH = 7.2) at a concentration of 1E7 cells per 40 µL (all the following amounts were calculated based on 1E7 cells). 10 µL of Pan T Cell Biotin Antibody Cocktail was added, and the mixture was incubated at 4°C for 5 minutes. 30 µL of isolation buffer and 20 µL of Pan T Cell MicroBead Cocktail were added, and the mixture was incubated at 4°C for 10 minutes. The mixture was then passed through a MACS separation column to obtain T cells.

The obtained human DC cells and human T cells were collected, resuspended in complete medium (RPMI 1640 containing 10% FBS), inoculated in a 96-well plate at a density of 1E4 cells per well for DC cells and 1E5 cells per well for T cells, and co-cultured. Monoclonal antibody samples serially diluted in complete medium and the control antibody were added. The culture plate was incubated in a CO₂ incubator at 37°C for 5 days. After incubation, the supernatant in the wells was removed for assay of the cytokine IFN-γ (Biolegend, Cat. No. 430101) according to the kit instructions.

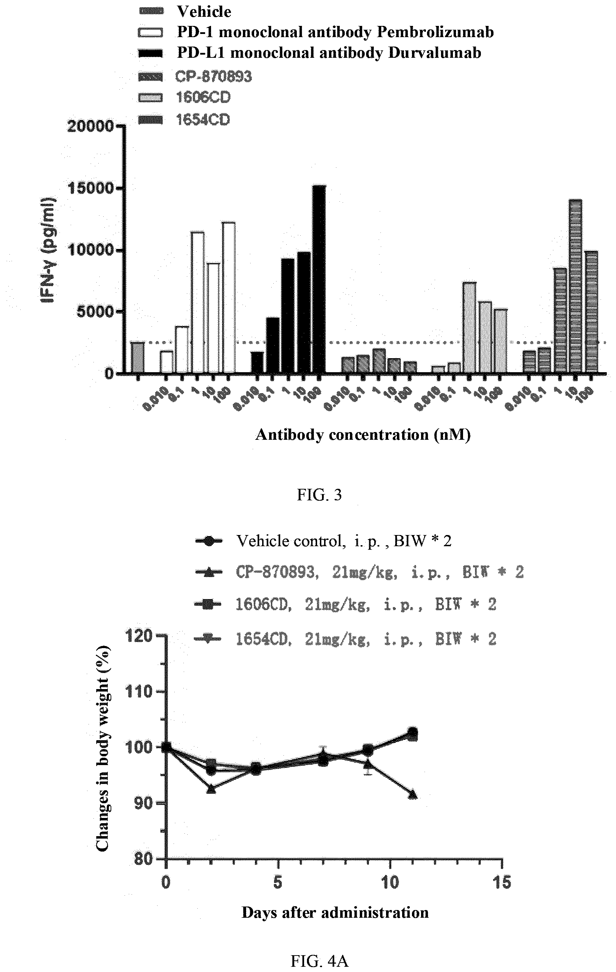

The results are shown in FIG. 3, indicating that both monoclonal antibody 1606CD with strong CD40 agonistic activity and monoclonal antibody 1654CD with weak CD40 agonistic activity of the present disclosure can enhance T cell activation under the condition of low percentage of DCs, while control monoclonal antibody CP-870893 has little effect.

### Example 10: Preliminary safety evaluation of anti-CD40 monoclonal antibody

The preliminary safety evaluation of anti-CD40 monoclonal antibody was conducted in human CD40 gene knock-in mice. Experimental animals were purchased from Biocytogen and randomly divided into 4 groups (5 mice per group), receiving vehicle control, 21 mg/kg of monoclonal antibody 1606CD, 21 mg/kg of monoclonal antibody 1654CD, and 21 mg/kg of control CD40 monoclonal antibody CP-870893, respectively. The mice were administered via intraperitoneal injection, twice a week for two consecutive weeks, for a total of 4 doses. During the trial period, the animals were monitored for clinical symptoms, body weight, liver function biochemistry, and blood cell counts. At the end of the administration period, all animals were euthanized the day following the last administration as planned, and dissected to check for abnormalities and to weigh the organs.

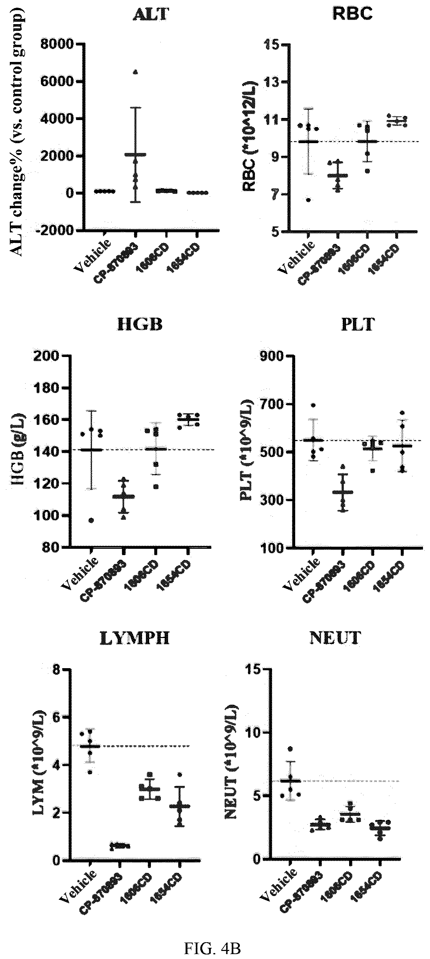

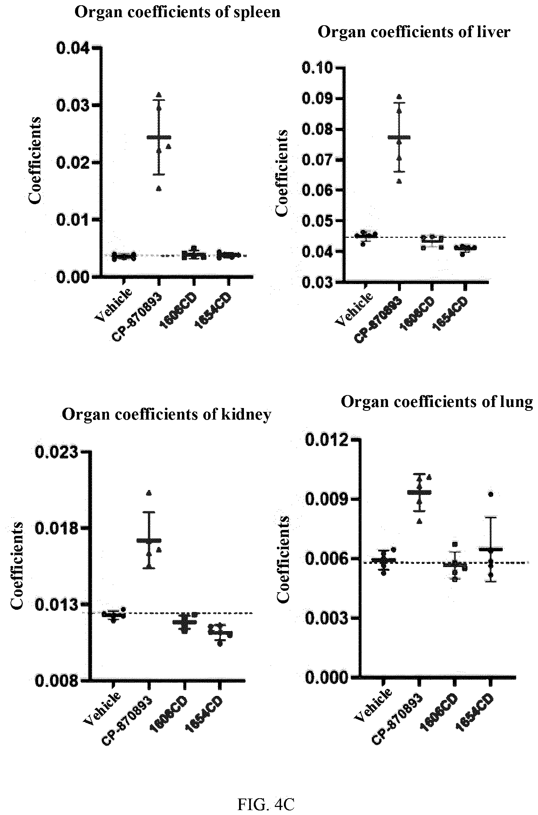

As shown in FIGs. 4A to 4C, mice repeatedly injected with monoclonal antibody 1606CD with strong CD40 agonistic activity and monoclonal antibody 1654CD with weak CD40 agonistic activity showed no significant abnormalities, with only a decrease in lymphocytes and granulocytes observed in hematology. In contrast, mice repeatedly injected with monoclonal antibody CP-870893 showed weight loss, elevated ALT levels, and significant decreases in RBC, HGB, and PLT. During dissection, significant organ necrosis and enlargement were observed. The results suggest that monoclonal antibodies 1606CD and 1654CD have a better safety profile compared to monoclonal antibody CP-870893.

### Example 11: Binding assay of recombinant PD-L1 monoclonal antibody to PD-L1

The binding ability of monoclonal antibody to human PD-L1, as well as the species specificity, was detected using an enzyme-linked immunosorbent assay (ELISA). The specific method is as follows: human PD-L1, cynomolgus PD-L1, rat PD-L1, and mouse PD-L1 antigens (all purchased from ACROBiosystems) were coated with 1 µg/mL carbonate buffer (pH = 9.6) in an ELISA plate at 100 µL per well, followed by incubation at 4°C overnight. The wells were washed 5 times with PBST. 300 µL/well of PBST containing 1% BSA was added to block the wells, and the plate was incubated at room temperature for 1 hour. The wells were washed 5 times with PBST. Monoclonal antibody serially diluted with PBST containing 1% BSA, along with control PD-L1 monoclonal antibody Durvalumab (IMGT database ID 10010), were added at 100 µL per well, and the plate was incubated at room temperature for 1 hour. The wells were washed 5 times with PBST. HRP-labeled anti-human IgG antibody (Jackson ImmunoResearch, Cat. No. 109-035-088) diluted with PBST containing 1% BSA was added at 100 µL per well, and the plate was incubated at room temperature for 1 hour. The wells were washed 5 times with PBST. Colorimetric substrate TMB was added at 100 µL per well and allowed to develop color at room temperature for 10 minutes, followed by the addition of 1 M sulfuric acid to terminate the reaction. The OD_{450 nm} was read on a microplate reader to analyze the results, and the EC₅₀ was calculated using a 4-parameter fit binding curve.

The results are shown in Table 12, indicating that monoclonal antibodies 1029, 1031, 1102, and 1541 are able to bind to both human PD-L1 and cynomolgus PD-L1, and their binding ability to human and cynomolgus monkey antigens is comparable to that of the control antibody. These monoclonal antibodies are not able to bind to rat PD-L1 or mouse PD-L1.

**Table 12: Binding of monoclonal antibodies to antigens**

| Antibody name | EC₅₀ (nM) | | | |
|---|---|---|---|---|
| | PD-L1 | | | |
| | Human | Cynomolgus | Rat | Mouse |
| Durvalumab | 0.014 | 0.017 | NB | NB |
| 1029 | 0.012 | 0.013 | NB | NB |
| 1031 | 0.020 | 0.018 | NB | NB |
| 1102 | 0.013 | 0.013 | NB | NB |
| 1541 | 0.014 | 0.018 | NB | NB |

| | | | | |
|---|---|---|---|---|
| NB: No binding | | | | |

The interaction between monoclonal antibodies and antigens was detected using Gator, a label-free biomolecular interaction analyzer based on the principle of biolayer interferometry (BLI). The specific method is as follows: monoclonal antibody was diluted to 50 nM and added to a probe plate using a PA probe, allowing the monoclonal antibody to be captured by the PA probe. Human PD-L1, an antigen serially diluted starting from 200 nM, was then added for interaction with the bispecific antibody captured by the PA probe. The interaction was analyzed by detecting the signal change of the reflected interference spectrum on the surface of the probe, and the binding kinetic constants of the antibody were finally calculated.

The results are shown in Table 13, indicating that the binding kinetic constants of monoclonal antibodies 1029, 1031, 1102, and 1541 to human PD-L1 range from 4.37 to 8.30 nM.

**Table 13: Binding kinetics of monoclonal antibodies to antigens**

| Antibody name | Human PD-L1 | | |
|---|---|---|---|
| | k_{off} (1/s) | kₒₙ (1/Ms) | K_{D} (M) |
| Durvalumab | 2.65 × 10⁻³ | 4.92 × 10⁶ | 5.39 × 10⁻⁹ |
| 1029 | 4.22 × 10⁻³ | 9.67 × 10⁵ | 4.37 × 10⁻⁹ |
| 1031 | 9.13 × 10⁻³ | 1.10 × 10⁶ | 8.30 × 10⁻⁹ |
| 1102 | 4.51 × 10⁻³ | 1.02 × 10⁶ | 4.41 × 10⁻⁹ |
| 1541 | 2.93 × 10⁻³ | 6.70 × 10⁵ | 4.38 × 10⁻⁹ |

### Example 12: Detection of PD-L1/PD-1 blocking activity of PD-L1 monoclonal antibody by reporter gene assay

A reporter gene assay for PD-L1/PD-1 blocking activity was established using Jurkat/PD-1-NFAT-luciferase cells (which highly express PD-1 and contain the luciferase reporter gene under the control of NFAT response elements) and WIL2S/PD-L1 cells (which highly express PD-L1) constructed by FutureGen Biopharmaceutical, along with anti-CD20/CD3 bispecific antibody. The specific method is as follows: WIL2S/PD-L1 cells were collected and resuspended in complete medium (RPMI 1640 containing 10% FBS) at a density of 4 × 10⁶ cells/mL, and then evenly spread in a 96-well plate at 50 µL/well, *i.e.,* 2 × 10⁵ cells per well. Jurkat-PD-1-NFAT-luciferase cells were collected and resuspended in complete medium (RPMI 1640 containing 10% FBS) at a density of 4 × 10⁶ cells/mL, and then evenly spread in the 96-well plate at 50 µL/well, *i.e.,* 2 × 10⁵ cells per well. The anti-CD20/CD3 bispecific antibody diluted in complete medium was added to the 96-well plate at 25 µL/well. Anti-PD-L1 monoclonal antibody samples serially diluted in complete medium and the control antibody were added to the 96-well plate at 25 µL/well.The plate was then incubated in a 5% CO₂ incubator at 37°C for 6 hours. After incubation, 50 µL/well of ONE-Glo reagent (Promega, Cat. No. E6120) was added to the 96-well plate, which was shaken on a plate shaker for 5 minutes and allowed to stand for 10 minutes. The relative chemiluminescence units (RLU) were read on a microplate reader (MD, SpectraMax iD3) with a chemiluminescence module to analyze the results.

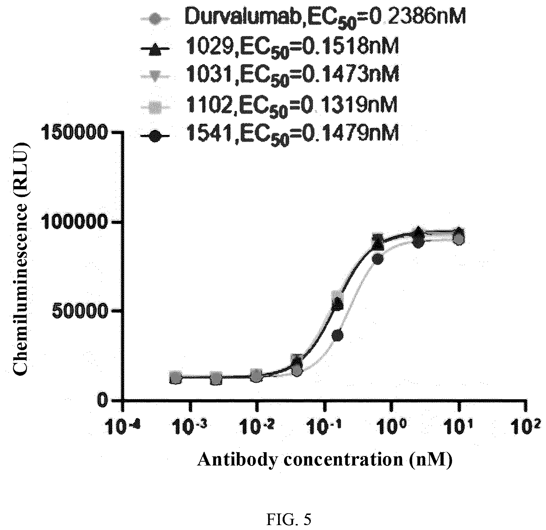

The results are shown in FIG. 5, indicating that monoclonal antibodies 1029, 1031, 1102, and 1541 can block the negative signaling from PD-L1 to PD-1. The activity of these antibodies is similar, but slightly stronger than that of the PD-L1 control monoclonal antibody Durvalumab.

### Example 13: T cell regulatory activity of PD-L1 monoclonal antibody

The T cell regulatory activity of monoclonal antibodies was tested using the MLR assay system incubated with allogeneic DC cells and T cells.

Donor human PBMCs were resuspended in complete medium (RPMI 1640 containing 10% FBS) and inoculated in a 10 cm cell culture dish, which was incubated in a CO₂ incubator at 37°C for 2 hours. The culture supernatant and suspended cells were discarded, leaving adherent monocytes. The monocytes were incubated in complete medium containing 100 ng/mL GM-CSF (PeproTech, Cat. No. 300-03) and 100 ng/mL IL-4 (PeproTech, Cat. No. 200-04) for 6 days, with medium exchanged every 2 days, followed by the addition of TNFα and IL-1β (both purchased from PeproTech) and incubation for 2 days to obtain DC cells.

Allogeneic T cells were isolated from donor human PBMCs, following the instructions provided in the Pan T Cell Isolation Kit (Miltenyi Biotech, Cat. No. 130-096-535). Briefly, PBMCs were first washed once with PBS, and then resuspended in isolation buffer (PBS containing 2 mM EDTA and 0.5% BSA, pH = 7.2) at a concentration of 1 × 10⁷ cells per 40 µL (all the following amounts were calculated based on 1 × 10⁷ cells). 10 µL of Pan T Cell Biotin Antibody Cocktail was added, and the mixture was incubated at 4°C for 5 minutes. 30 µL of isolation buffer and 20 µL of Pan T Cell MicroBead Cocktail were added, and the mixture was incubated at 4°C for 10 minutes. The mixture was then passed through a MACS separation column to obtain T cells.

The obtained human DC cells and human T cells were collected, resuspended in complete medium (RPMI 1640 containing 10% FBS), inoculated in a 96-well plate at a density of 1 × 10⁴ cells per well for DC cells and 1 × 10⁵ cells per well for T cells, and co-cultured. Antibody samples serially diluted in complete medium were added. The culture plate was incubated in a CO₂ incubator at 37°C for 5 days. After incubation, the supernatant in the wells was removed for assay of the cytokine IFN-γ (Biolegend, Cat. No. 430101) according to the kit instructions.

The results are shown in FIG. 6, indicating that the monoclonal antibodies significantly enhance T cell activation.

### Example 14: Binding assay of recombinant anti-PD-L1/CD40 bispecific antibody to PD-L1 and CD40

The binding ability of bispecific antibody to human PD-L1 and human CD40, as well as the species specificity, was detected using an enzyme-linked immunosorbent assay (ELISA). The specific method is as follows: human PD-L1, cynomolgus PD-L1, rat PD-L1, mouse PD-L1, human CD40, cynomolgus CD40, rat CD40, and mouse CD40 antigens (all purchased from ACROBiosystems) were coated with 1 µg/mL carbonate buffer (pH = 9.6) in an ELISA plate at 100 µL per well, followed by incubation at 4°C overnight. The wells were washed 5 times with PBST. 300 µL/well of PBST containing 1% BSA was added to block the wells, and the plate was incubated at room temperature for 1 hour. The wells were washed 5 times with PBST. Bispecific antibody serially diluted with PBST containing 1% BSA, along with control PD-L1 monoclonal antibody Durvalumab (IMGT database ID 10010) and CD40 monoclonal antibody CP-870893 (IMGT database ID 10523), were added at 100 µL per well, and the plate was incubated at room temperature for 1 hour. The wells were washed 5 times with PBST. HRP-labeled anti-human IgG antibody (Jackson ImmunoResearch, Cat. No. 109-035-088) diluted with PBST containing 1% BSA was added at 100 µL per well, and the plate was incubated at room temperature for 1 hour. The wells were washed 5 times with PBST. Colorimetric substrate TMB was added at 100 µL per well and allowed to develop color at room temperature for 10 minutes, followed by the addition of 1 M sulfuric acid to terminate the reaction. The OD_{450 nm} was read on a microplate reader to analyze the results, and the EC₅₀ was calculated using a 4-parameter fit binding curve.

The results are shown in Table 14, indicating that bispecific antibodies 1605, 1606, 1607, 1608, 1609, 1652, 1653, 1654, and 1655 are able to bind to human PD-L1 and CD40 as well as cynomolgus PD-L1 and CD40, and their binding ability to human and cynomolgus monkey antigens is comparable. These bispecific antibodies are not able to bind to rat PD-L1 and CD40 or mouse PD-L1 and CD40.

**Table 14: Binding of bispecific antibodies to antigens**

| | EC₅₀ (nM) | | | | | | | |
|---|---|---|---|---|---|---|---|---|
| | PD-L1 | | | | CD40 | | | |
| | Human | Cynomolgus | Rat | Mouse | Human | Cynomolgus | Rat | Mouse |
| Durvalumab | 0.012 | 0.017 | NB | NB | NB | NB | NB | NB |
| CP-870893 | NB | NB | NB | NB | 0.016 | 0.016 | NB | NB |
| 1605 | 0.050 | 0.025 | NB | NB | 0.024 | 0.020 | NB | NB |
| 1606 | 0.039 | 0.022 | NB | NB | 0.024 | 0.020 | NB | NB |
| 1607 | 0.036 | 0.019 | NB | NB | 0.020 | 0.016 | NB | NB |
| 1608 | 0.038 | 0.019 | NB | NB | 0.022 | 0.020 | NB | NB |
| 1609 | 0.034 | 0.016 | NB | NB | 0.021 | 0.019 | NB | NB |
| 1652 | 0.035 | 0.018 | NB | NB | 0.021 | 0.017 | NB | NB |
| 1653 | 0.039 | 0.021 | NB | NB | 0.020 | 0.018 | NB | NB |
| 1654 | 0.031 | 0.045 | NB | NB | 0.013 | 0.013 | NB | NB |
| 1655 | 0.039 | 0.023 | NB | NB | 0.025 | 0.006 | NB | NB |

| | | | | | | | | |
|---|---|---|---|---|---|---|---|---|
| NB: No binding | | | | | | | | |

### Example 15: Simultaneous binding assay of recombinant anti-PD-L1/CD40 bispecific antibody to PD-L1 and CD40

Antigen human CD40 (purchased from ACROBiosystems) was coated with 1 µg/mL carbonate buffer (pH = 9.6) in an ELISA plate at 100 µL per well, followed by incubation at 4°C overnight. The wells were washed 5 times with PBST. 300 µL/well of PBST containing 1% BSA was added to block the wells, and the plate was incubated at room temperature for 1 hour. The wells were washed 5 times with PBST. Bispecific antibody serially diluted with PBST containing 1% BSA, along with control PD-L1 monoclonal antibody Durvalumab and CD40 monoclonal antibody CP-870893, were added at 100 µL per well, and the plate was incubated at room temperature for 1 hour. The wells were washed 5 times with PBST. Biotin-labeled human PD-L1 (FutureGen Biopharmaceutical) diluted with PBST containing 1% BSAwas added at 100 µL per well, and the plate was incubated at room temperature for 1 hour. The wells were washed 5 times with PBST. Streptavidin-HRP (BioLegend, Cat. No. 405210) diluted with PBST containing 1% BSA was added at 100 µL per well, and the plate was incubated at room temperature for 30 minutes. Colorimetric substrate TMB was added at 100 µL per well and allowed to develop color at room temperature for 10 minutes, followed by the addition of 1 M sulfuric acid to terminate the reaction. The OD_{450 nm} was read on a microplate reader to analyze the results, and the EC₅₀ was calculated using a 4-parameter fit binding curve.

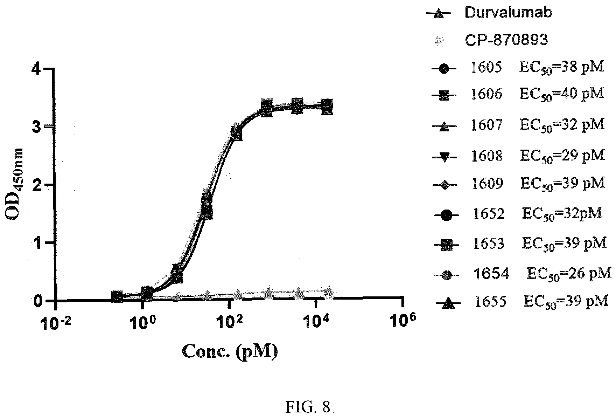

The results are shown in FIG. 8, indicating that bispecific antibodies 1605, 1606, 1607, 1608, 1609, 1652, 1653, 1654, and 1655 can bind to both human PD-L1 and human CD40, while the control monoclonal antibody has no signal and cannot bind to both antigens.

### Example 16: Detection of PD-L1/PD-1 blocking activity of anti-PD-L1/CD40 bispecific antibody by reporter gene assay

Jurkat/PD-1-NFAT-luciferase cells (which highly express PD-1 and contain the luciferase reporter gene under the control of NFAT response elements) and WIL2S/PD-L1 cells (which highly express PD-L1) were constructed by lentiviral transfection using methods referenced in the literature (Xiaoyin Wang et al., J Vis Exp. 2009; (32): 1499; Jonathan Elegheert et al., Nat Protoc. 2018 Dec; 13(12): 2991-3017; Andreas Rinne et al., J Physiol. 2010 Sep 1; 588(Pt 17): 3211-3216). A reporter gene assay for PD-L1/PD-1 blocking activity was established using the Jurkat and WIL2S cells, along with anti-CD20/CD3 bispecific antibody. The specific method is as follows: WIL2S/PD-L1 cells were collected and resuspended in complete medium (RPMI 1640 containing 10% FBS) at a density of 4E6 cells/mL, and then evenly spread in a 96-well plate at 50 µL/well, *i.e.,* 2E5 cells per well. Jurkat-PD-1-NFAT-luciferase cells were collected and resuspended in complete medium (RPMI 1640 containing 10% FBS) at a density of 4E6 cells/mL, and then evenly spread in the 96-well plate at 50 µL/well, *i.e.,* 2E5 cells per well. The anti-CD20/CD3 bispecific antibody diluted in complete medium was added to the 96-well plate at 25 µL/well. Anti-PD-L1/CD40 bispecific antibody samples serially diluted in complete medium and the control antibody were added to the 96-well plate at 25 µL/well. The plate was then incubated in a 5% CO₂ incubator at 37°C for 6 hours. After incubation, 50µl/well of ONE-Glo reagent (Promega, Cat. No. E6120) was added to the 96-well plate, which was shaken on a plate shaker for 5 minutes and allowed to stand for 10 minutes. The relative chemiluminescence units (RLU) were read on a microplate reader (MD, SpectraMax iD3) with a chemiluminescence module to analyze the results.

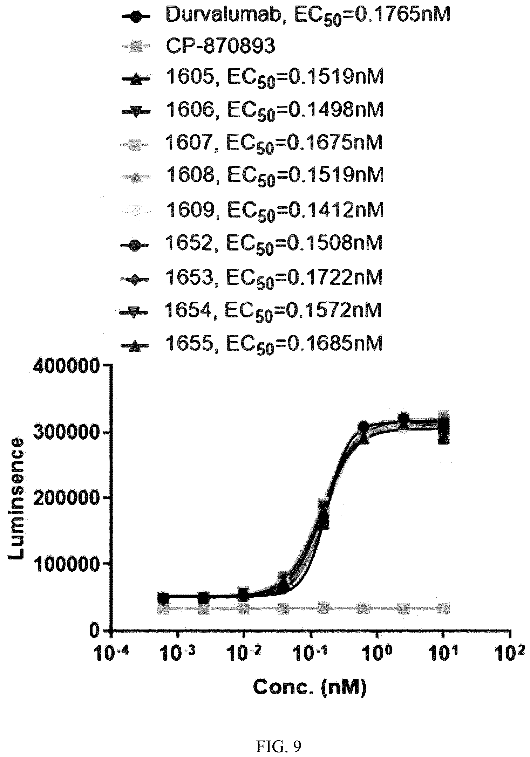

The results are shown in FIG. 9, indicating that bispecific antibodies 1605, 1606, 1607, 1608, 1609, 1652, 1653, 1654, and 1655 can block the negative signaling from PD-L1 to PD-1. The activity of different bispecific antibodies is similar, which is also similar to that of the PD-L1 control monoclonal antibody Durvalumab.

### Example 17: Detection of CD40 agonistic activity of anti-PD-L1/CD40 bispecific antibody by reporter gene assay

The CD40 agonistic activity of bispecific antibodies was tested using HEK-Blue CD40L cells. HEK-Blue CD40L cells, purchased from InvivoGen, highly express CD40 and contain the SEAP reporter gene under the control of NF-κB response elements. When CD40 on HEK-Blue CD40L cells is activated, it induces the activation of downstream NF-κB signaling, which in turn induces the production of SEAP. CD40 activation can be monitored by detecting the amount of secreted SEAP using QUANTI-Blue reagent (InvivoGen). The CD40 agonistic activity of bispecific antibodies in the presence of PD-L1 was tested by co-incubation of CHO/PD-L1 cells with HEK-Blue CD40L cells. The specific method is as follows: HEK-Blue CD40L cells were collected and resuspended in complete medium (RPMI 1640 containing 10% FBS) at 3E5 cells/mL, and then evenly spread in a 96-well plate at 100 µL/well, *i.e.,* 3E4 cells per well. CHO/PD-L1 cells were collected and resuspended in complete medium (RPMI 1640 containing 10% FBS) at 6E5 cells/mL, and then added to the 96-well plate at 50 µL/well, *i.e.,* 3E4 cells per well. If CHO/PD-L1 cells were not required, 50 µL/well of complete medium was added instead. Anti-PD-L1/CD40 bispecific antibody samples serially diluted in complete medium and the control antibody were added at 50 µL/well, and the plate was incubated in a 5% CO₂ incubator at 37°C for 20 to 24 hours. After incubation, the 96-well plate was removed and centrifuged at 300 g for 5 minutes, and 40 µL/well of the supernatant was aspirated and transferred to a new 96-well plate. 160 µL/well of QUANTI-Blue reagent was added to the supernatant, and the plate was incubated in a 5% CO₂ incubator at 37°C for 20 to 30 minutes. The OD_{655 nm} was then read on a microplate reader to analyze the results.

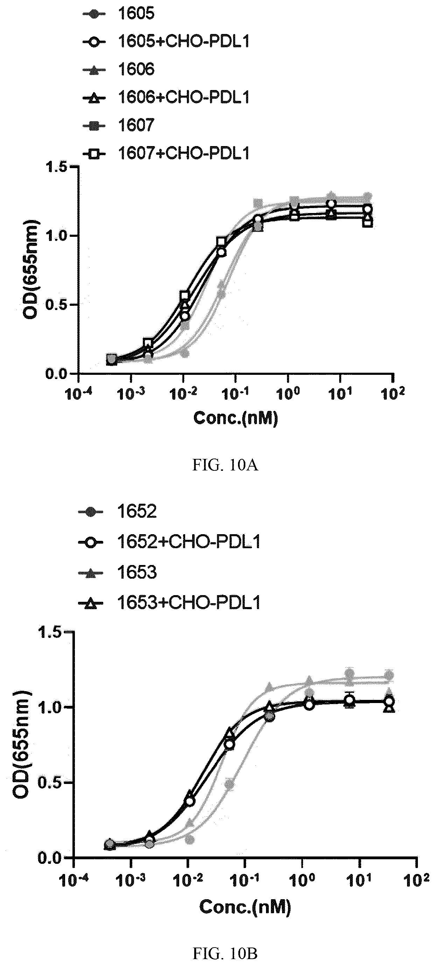

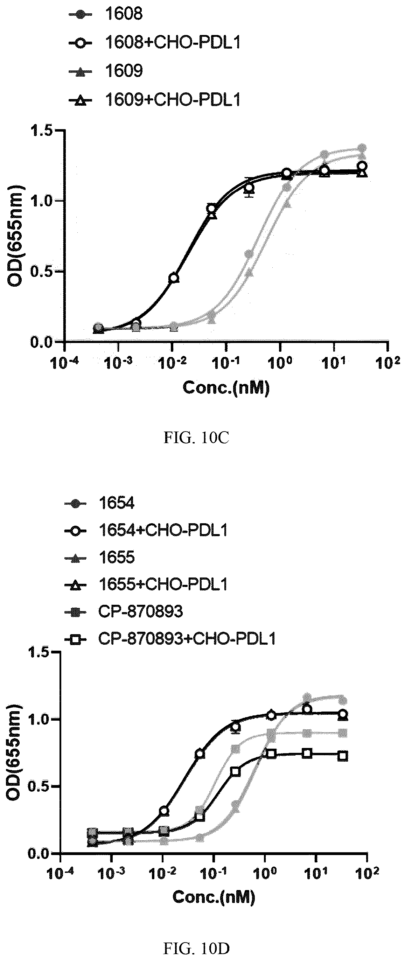

The results are shown in FIGs. 10A to 10D and Table 15, indicating that the CD40 agonistic activity of bispecific antibodies 1608, 1609, 1654, and 1655 is significantly enhanced by the PD-L1 cross-linking signal provided by CHO/PD-L1. This suggests that the activity of these bispecific antibodies is selective: their activity is low in environments with low PD-L1 expression (*e.g.,* in blood environments) and high in environments with high PD-L1 expression (*e.g.,* in tumor environments), leading to enhanced efficacy and controlled side effects in treatment. In contrast, the CD40 agonistic activity of bispecific antibodies 1605, 1606, 1607, 1652, and 1653 is less enhanced by CHO/PD-L1, while the CD40 agonistic activity of the control CD40 monoclonal antibody CP-870893 is not enhanced by CHO/PD-L1.

**Table 15: CD40 agonistic activity of bispecific antibodies**

| | EC₅₀ (nM) | | |
|---|---|---|---|
| | without CHO/PD-L1 (A) | with CHO/PD-L1 (B) | (A)/(B) Ratio |
| 1605 | 0.075 | 0.024 | 3.2 |
| 1606 | 0.061 | 0.017 | 3.7 |
| 1607 | 0.029 | 0.012 | 2.4 |
| 1652 | 0.092 | 0.022 | 4.1 |
| 1653 | 0.039 | 0.018 | 2.2 |
| 1608 | 0.396 | 0.020 | 20.2 |
| 1609 | 0.556 | 0.020 | 28.0 |
| 1654 | 0.633 | 0.027 | 23.2 |
| 1655 | 0.686 | 0.027 | 25.3 |
| CP-870893 | 0.109 | 0.123 | 0.9 |

### Example 18: Effect of anti-PD-L1/CD40 bispecific antibody on CD40/CD40L binding

The blocking effect of bispecific antibodies on CD40/CD40L binding was tested using HEK-Blue CD40L cells that highly express CD40. The specific method is as follows: HEK-Blue CD40L cells were collected and resuspended in pre-cooled PBS containing 2% FBS at a density of 1E7 cells/mL, and then added to a 96-well plate at 50 µL per well, *i.e*., 5E5 cells/well. Biotin-CD40L (FutureGen Biopharmaceutical) and serially diluted bispecific antibody were then added at 50 µL per well, with a final concentration of Biotin-CD40L of 10 nM, and the plate was incubated at 4°C for 1 hour. The wells were washed twice with pre-cooled PBS. Streptavidin-PE (BioLegend, Cat. No. 405203) diluted in pre-cooled PBS containing 2% FBS was added, and the plate was incubated at 4°C for 30 minutes. The wells were washed twice with pre-cooled PBS. The cells were then resuspended in pre-cooled PBS containing 2% FBS and assayed by flow cytometry.

The results are shown in Table 16, indicating that bispecific antibodies 1605, 1606, 1607, 1608, 1609, 1652, 1653, 1654, and 1655 hinder the CD40/CD40L interaction, while the control CD40 monoclonal antibody CP-870893 has no effect on the CD40/CD40L interaction.

**Table 16: Effect of bispecific antibodies on CD40/CD40L binding**

| CD40/CD40L interaction blocking | | IC₅₀ (nM) |
|---|---|---|
| Durvalumab | No | NA |
| CP-870893 | No | NA |
| 1605 | Yes | 1.088 |
| 1606 | Yes | 0.780 |
| 1607 | Yes | 0.703 |
| 1608 | Yes | 4.542 |
| 1609 | Yes | 3.156 |
| 1652 | Yes | 0.852 |
| 1653 | Yes | 0.807 |
| 1654 | Yes | 7.699 |
| 1655 | Yes | 8.626 |

### Example 19: DC regulatory activity of anti-PD-L1/CD40 bispecific antibody

The dendritic cell (DC) regulatory activity of bispecific antibodies was tested.

Human PBMCs were resuspended in complete medium (RPMI 1640 containing 10% FBS) and inoculated in a 10 cm cell culture dish, which was incubated in a CO₂ incubator at 37°C for 2 hours. The culture supernatant and suspended cells were discarded, leaving adherent monocytes. The monocytes were incubated in complete medium containing 100 ng/mL GM-CSF (PeproTech, Cat. No. 300-03) and 100 ng/mL IL-4 (PeproTech, Cat. No. 200-04) for 6 days, with medium exchanged every 2 days to obtain imDCs. The imDCs were collected, resuspended in complete medium, and inoculated in a 24-well plate, followed by the addition of serially diluted bispecific antibody samples and the control antibody. The culture plate was incubated in a CO₂ incubator at 37°C for 2 days. After incubation, the supernatant in the wells was removed for assay of the cytokines IL-12/IL-23 p40 (R&D, Cat. No. DY1240) according to the kit instructions. In some experiments, the cells in the well plate were simultaneously collected and incubated with the detection antibodies (APC anti-human CD83 antibody, Biolegend, 305312; PE/Cyanine7 anti-human CD86 antibody, Biolegend, 374210), followed by assay of CD83 and CD86 expression on DC cells by flow cytometry.

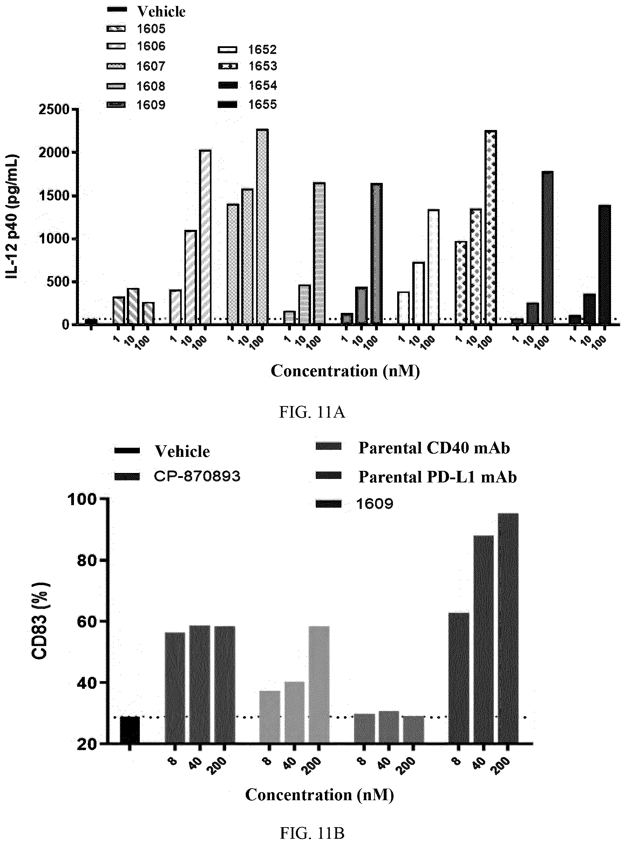

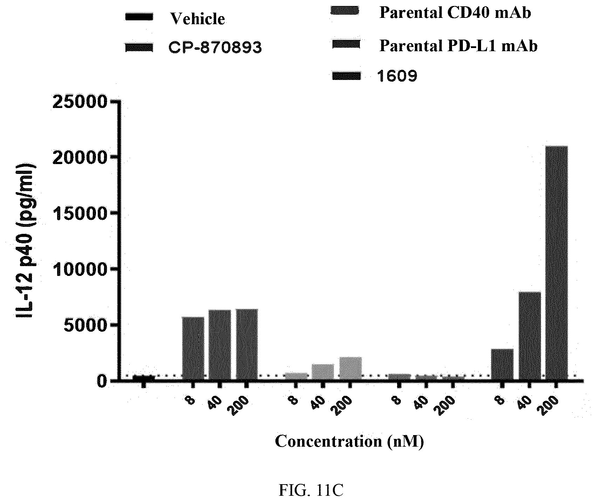

FIG. 11A compares the DC regulatory activity of different bispecific antibodies, using IL-12/IL-23 p40 as an indicator. The bispecific antibodies stimulate DCs to secrete IL-12 p40 in a concentration-dependent manner, with large differences in activity observed among different bispecific antibodies. FIGs. 11B and 11C compare the DC regulatory activity of the parental PD-L1 monoclonal antibody, the parental CD40 monoclonal antibody, the bispecific antibody (1609), and the control CD40 monoclonal antibody CP-870893, using CD83 and IL-12/IL-23 p40 as indicators. The data show that the parental PD-L1 monoclonal antibody has no regulatory effect on DCs, while the parental CD40 monoclonal antibody exhibits weak activity. However, when constructed as a bispecific antibody, the DC activity is significantly enhanced.

### Example 20: T cell regulatory activity of anti-PD-L1/CD40 bispecific antibody

The T cell regulatory activity of bispecific antibodies was tested using the MLR assay system incubated with allogeneic DC cells and T cells.

DC cells were obtained using the same method as in Example 19. Allogeneic T cells were isolated from human PBMCs, following the instructions provided in the Pan T Cell Isolation Kit (Miltenyi Biotech, Cat. No. 130-096-535). Briefly, PBMCs were first washed once with PBS, and then resuspended in isolation buffer (PBS containing 2 mM EDTA and 0.5% BSA, pH = 7.2) at a concentration of 1E7 cells per 40 µL (all the following amounts were calculated based on 1E7 cells). 10 µL of Pan T Cell Biotin Antibody Cocktail was added, and the mixture was incubated at 4°C for 5 minutes. 30 µL of isolation buffer and 20 µL of Pan T Cell MicroBead Cocktail were added, and the mixture was incubated at 4°C for 10 minutes. The mixture was then passed through a MACS separation column to obtain T cells.

The obtained human DC cells and human T cells were collected, resuspended in complete medium (RPMI 1640 containing 10% FBS), inoculated in a 96-well plate at a density of 1E4 cells per well for DC cells and 1E5 cells per well for T cells, and co-cultured. Bispecific antibody samples serially diluted in complete medium and the control antibody were added. The culture plate was incubated in a CO₂ incubator at 37°C for 5 days. After incubation, the supernatant in the wells was removed for assay of the cytokine IFN-γ (Biolegend, Cat. No. 430101) according to the kit instructions.

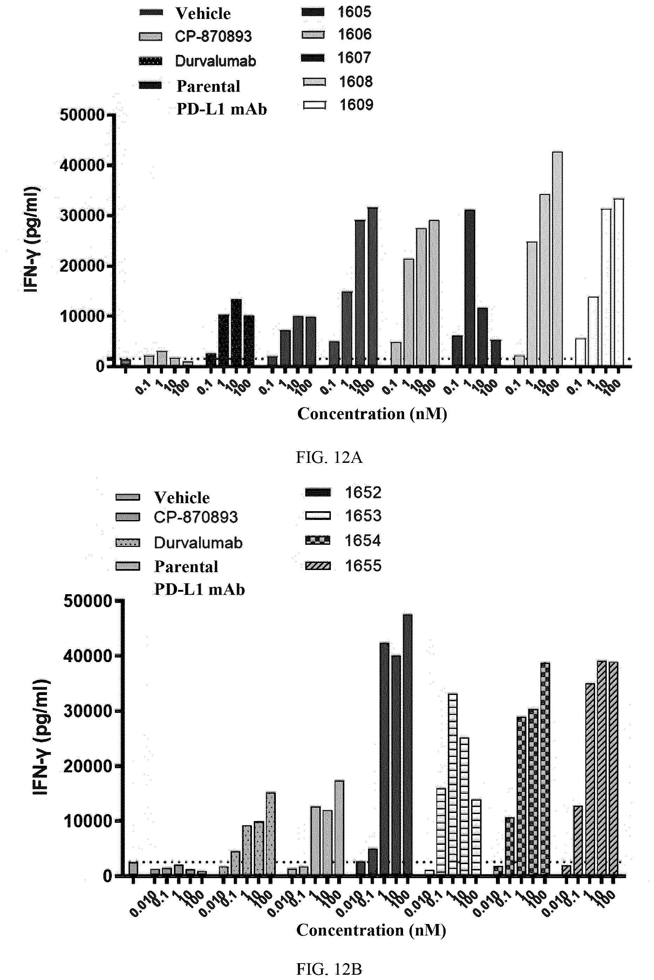

The results are shown in FIGs. 12A and 12B, indicating that the bispecific antibodies enhance T cell activation, and their T cell regulatory activity is significantly stronger than that of the parental PD-L1 monoclonal antibody, the parental CD40 monoclonal antibody, and the PD-L1 control monoclonal antibody Durvalumab.

### Example 21: In vivo anti-tumor efficacy of anti-mouse PD-L1/mouse CD40 bispecific antibody

The *in vivo* anti-tumor efficacy of bispecific antibodies in mice was tested in this example. To facilitate the evaluation of the *in vivo* efficacy of the anti-PD-L1/CD40 bispecific antibody, anti-mouse PD-L1/mouse CD40 bispecific antibody 1058, an alternative expressing the bispecific antibody, was constructed (the anti-PD-L1 sequence is derived from IMGT database ID 9814; the anti-CD40 sequences are No. 33_VH and No. 34_VL in WO2018185045A1). The purpose of using the alternative is to evaluate the *in vivo* effect of a bispecific antibody formed by an antibody targeting PD-L1 and an antibody targeting CD40 in wild-type mice, solely for validating the efficacy of such bispecific antibodies.

Female C57BL/6 mice aged 6 to 8 weeks were purchased from Beijing Vital River Laboratory Animal Technology Co., Ltd. After acclimatization for one week, each mouse was inoculated with 3E5 MC38 mouse colon cancer cells (purchased from the Cell Center of Basic Medicine, Institute of Basic Medical Sciences, Chinese Academy of Medical Sciences). Once the tumor volume reached approximately 100 mm³, the mice were grouped according to tumor volume, with 8 mice per group, set as the vehicle control group, anti-mouse PD-L1 monoclonal antibody treatment group, anti-mouse CD40 monoclonal antibody treatment group, and anti-mouse PD-L1/mouse CD40 bispecific antibody treatment group. The mice were administered via intraperitoneal injection at a dose of 35 nmol/kg, once a week for two consecutive weeks. From the date of administration, the tumor volume was measured three times a week, measuring the long diameter (a) and short diameter (b) to calculate tumor volume (mm³) = (a × b²) / 2.

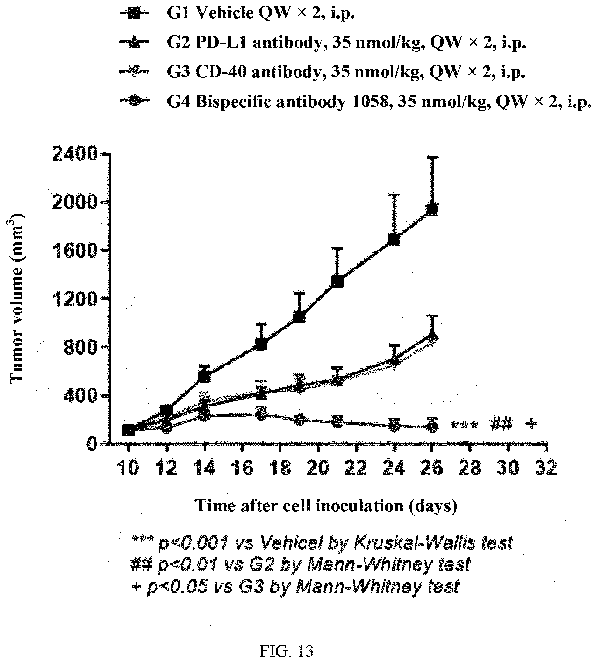

The results are shown in FIG. 13, indicating that the anti-mouse PD-L1/mouse CD40 bispecific antibody 1058, an alternative for bispecific antibodies, significantly inhibited the growth of MC38 colon cancer xenografts in mice, demonstrating good anti-tumor efficacy, which was stronger than that of the PD-L1 monoclonal antibody and the CD40 monoclonal antibody.

### Example 22: In vivo anti-tumor efficacy of anti-PD-L1/CD40 bispecific antibody

The *in vivo* anti-tumor efficacy of anti-PD-L1/CD40 bispecific antibodies in PD-L1/CD40 humanized mice was evaluated in this example. The experiment was conducted in cooperation with Beijing University of Agriculture. Female PD-L1/CD40 humanized mice aged 6 to 8 weeks were purchased from Biocytogen. After acclimatization for one week, each mouse was inoculated with 5E6 MC38/hPD-L1 mouse colon cancer cells. Once the tumor volume reached approximately 100 mm³, the mice were grouped according to tumor volume, with 6 mice per group, receiving vehicle, anti-PD-L1 monoclonal antibody, anti-CD40 monoclonal antibody, and anti-PD-L1/anti-CD40 bispecific antibody 1654 via intraperitoneal injection, twice a week for two consecutive weeks. From the date of administration, the mice were monitored for clinical performance, body weight, and tumor volume. The long diameter (a) and short diameter (b) were measured to calculate tumor volume (mm³) = (a × b²) / 2.

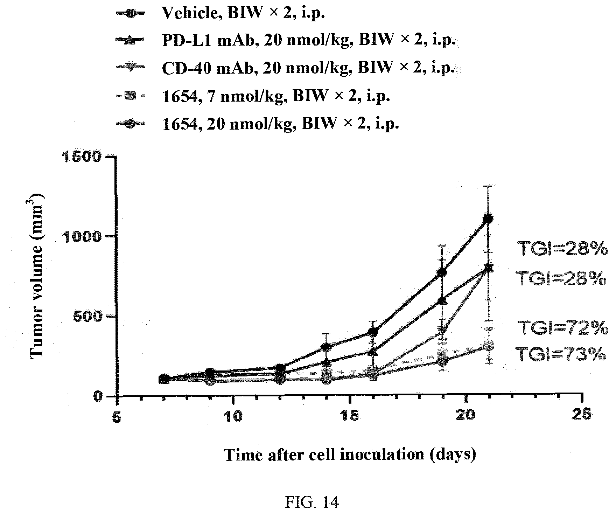

The results are shown in FIG. 14, indicating that 1654 significantly inhibited the growth of MC38/hPD-L1 xenografts in PD-L1/CD40 humanized mice. At a dose as low as 7 nmol/kg, the tumor growth inhibition (TGI) reached 72%, which was stronger than that of the PD-L1 monoclonal antibody or the CD40 monoclonal antibody at a dose of 20 nmol/kg, both of which had a TGI of 28%. During the experiment, one mouse in the vehicle control group died, but no abnormalities were observed in the other mice. There were no abnormalities in body weight of the mice.

### Example 23: Preliminary safety evaluation of anti-PD-L1/CD40 bispecific antibody

The preliminary safety evaluation of anti-PD-L1/CD40 bispecific antibody was commissioned to JOINN (Suzhou) New Drug Research Center Co., Ltd.

The experimental animals used were male cynomolgus monkeys, which were randomly divided into 5 groups (2 mice per group), receiving vehicle control, 12 mg/kg of bispecific antibodies 1607, 1608, and 1609, and 10 mg/kg of control CD40 monoclonal antibody CP-870893, respectively. The animals were administered via subcutaneous intravenous infusion in the forelimbs or hindlimbs using a syringe pump, with a volume of 10 mL/kg and a rate of 0.5 mL/kg/min. The animals were administered once a week for two consecutive weeks, for a total of 3 doses. During the trial period, the animals were regularly monitored for clinical symptoms, body weight, food intake, body temperature, blood cell counts, coagulation function, blood biochemistry and urinalysis, immune cell phenotypes, and cytokines, and tested for blood drug concentrations and anti-drug antibodies. At the end of the administration period, all surviving animals in groups 2 to 5 were euthanized the day following the last administration as planned, and gross dissection was performed to check for abnormalities and to weigh the organs.

For the animals in the groups receiving bispecific antibodies 1607, 1608, and 1609 at a dose of 12 mg/kg, clinical observations, body weight, weight gain, food intake, body temperature, blood cell counts, coagulation function, blood biochemistry, urinalysis, organ weight, and gross pathological anatomy showed no abnormalities related to the test samples. Only an increase was observed in immune cell phenotypes and some cytokines, which was related to the pharmacological mechanism of action. For the animals in the group receiving the CD40 monoclonal antibody CP-870893 at a dose of 10 mg/kg, a small amount of soft/loose stools was observed on D7-D8, D10-D11, and D7-D13, respectively; a slight decrease in weight gain was observed on day 14 compared to baseline; a significant decrease in food intake was observed starting from D5; a decreasing trend was observed in RBC, HGB, HCT, and PLT; phenotypic alteration of immune cells and an increase in some cytokines were observed; during the dissection of cynomolgus monkeys, the thymus showed a decrease in organ weight, organ-to-body weight ratio, and organ-to-brain ratio, while the spleen showed an increase in organ weight, organ-to-body weight ratio, and organ-to-brain ratio, with significant variation.

The results suggest that the anti-PD-L1/CD40 bispecific antibodies have a better safety profile compared to the CD40 monoclonal antibody.

## Claims

1. An anti-CD40 antibody comprising a heavy chain variable region and a light chain variable region, wherein the heavy chain variable region comprises HCDR1, HCDR2, and HCDR3 of SEQ ID NO: 64, and the light chain variable region comprises LCDR1, LCDR2, and LCDR3 of SEQ ID NO: 65;
preferably, the LCDR1 comprises an amino acid sequence as shown in SEQ ID NO: 1; the LCDR2 comprises an amino acid sequence as shown in X₅X₆SX₇X₈X₉S, wherein X₅ is Y or A; X₆ is T or A; X₇ is S, R, or T; X₈ is L or R; X₉ is Q or D; the LCDR3 comprises an amino acid sequence as shown in SEQ ID NO: 3; the HCDR1 comprises an amino acid sequence as shown in SEQ ID NO: 4; the HCDR2 comprises an amino acid sequence as shown in SEQ ID NO: 5; and the HCDR3 comprises an amino acid sequence as shown in SEQ ID NO: 6.

2. The antibody according to claim 1, wherein the LCDR1 comprises an amino acid sequence as shown in SEQ ID NO: 9, SEQ ID NO: 8, SEQ ID NO: 7, or SEQ ID NO: 10; the LCDR2 comprises an amino acid sequence as shown in SEQ ID NO: 14, SEQ ID NO: 12, SEQ ID NO: 13, SEQ ID NO: 11, or SEQ ID NO: 15; the LCDR3 comprises an amino acid sequence as shown in SEQ ID NO: 19, SEQ ID NO: 17, SEQ ID NO: 18, SEQ ID NO: 16, or SEQ ID NO: 20; the HCDR1 comprises an amino acid sequence as shown in SEQ ID NO: 21 or SEQ ID NO: 22; the HCDR2 comprises an amino acid sequence as shown in SEQ ID NO: 23 or SEQ ID NO: 24; and the HCDR3 comprises an amino acid sequence as shown in SEQ ID NO: 28, SEQ ID NO: 26, SEQ ID NO: 27, SEQ ID NO: 25, or SEQ ID NO: 29;
preferably, the LCDR1 comprises the amino acid sequence as shown in SEQ ID NO: 9; the LCDR2 comprises the amino acid sequence as shown in SEQ ID NO: 14; the LCDR3 comprises the amino acid sequence as shown in SEQ ID NO: 19; the HCDR1 comprises the amino acid sequence as shown in SEQ ID NO: 21; the HCDR2 comprises the amino acid sequence as shown in SEQ ID NO: 23; and the HCDR3 comprises the amino acid sequence as shown in SEQ ID NO: 28;
the LCDR1 comprises the amino acid sequence as shown in SEQ ID NO: 9; the LCDR2 comprises the amino acid sequence as shown in SEQ ID NO: 14; the LCDR3 comprises the amino acid sequence as shown in SEQ ID NO: 19; the HCDR1 comprises the amino acid sequence as shown in SEQ ID NO: 21; the HCDR2 comprises the amino acid sequence as shown in SEQ ID NO: 24; and the HCDR3 comprises the amino acid sequence as shown in SEQ ID NO: 28;
the LCDR1 comprises the amino acid sequence as shown in SEQ ID NO: 7; the LCDR2 comprises the amino acid sequence as shown in SEQ ID NO: 11; the LCDR3 comprises the amino acid sequence as shown in SEQ ID NO: 16; the HCDR1 comprises the amino acid sequence as shown in SEQ ID NO: 21; the HCDR2 comprises the amino acid sequence as shown in SEQ ID NO: 23; and the HCDR3 comprises the amino acid sequence as shown in SEQ ID NO: 25;
the LCDR1 comprises the amino acid sequence as shown in SEQ ID NO: 7; the LCDR2 comprises the amino acid sequence as shown in SEQ ID NO: 12; the LCDR3 comprises the amino acid sequence as shown in SEQ ID NO: 17; the HCDR1 comprises the amino acid sequence as shown in SEQ ID NO: 22; the HCDR2 comprises the amino acid sequence as shown in SEQ ID NO: 24; and the HCDR3 comprises the amino acid sequence as shown in SEQ ID NO: 26;
the LCDR1 comprises the amino acid sequence as shown in SEQ ID NO: 7; the LCDR2 comprises the amino acid sequence as shown in SEQ ID NO: 12; the LCDR3 comprises the amino acid sequence as shown in SEQ ID NO: 17; the HCDR1 comprises the amino acid sequence as shown in SEQ ID NO: 22; the HCDR2 comprises the amino acid sequence as shown in SEQ ID NO: 23; and the HCDR3 comprises the amino acid sequence as shown in SEQ ID NO: 26;
the LCDR1 comprises the amino acid sequence as shown in SEQ ID NO: 8; the LCDR2 comprises the amino acid sequence as shown in SEQ ID NO: 13; the LCDR3 comprises the amino acid sequence as shown in SEQ ID NO: 18; the HCDR1 comprises the amino acid sequence as shown in SEQ ID NO: 22; the HCDR2 comprises the amino acid sequence as shown in SEQ ID NO: 24; and the HCDR3 comprises the amino acid sequence as shown in SEQ ID NO: 27;
the LCDR1 comprises the amino acid sequence as shown in SEQ ID NO: 8; the LCDR2 comprises the amino acid sequence as shown in SEQ ID NO: 13; the LCDR3 comprises the amino acid sequence as shown in SEQ ID NO: 18; the HCDR1 comprises the amino acid sequence as shown in SEQ ID NO: 22; the HCDR2 comprises the amino acid sequence as shown in SEQ ID NO: 23; and the HCDR3 comprises the amino acid sequence as shown in SEQ ID NO: 27;
the LCDR1 comprises the amino acid sequence as shown in SEQ ID NO: 10; the LCDR2 comprises the amino acid sequence as shown in SEQ ID NO: 15; the LCDR3 comprises the amino acid sequence as shown in SEQ ID NO: 20; the HCDR1 comprises the amino acid sequence as shown in SEQ ID NO: 21; the HCDR2 comprises the amino acid sequence as shown in SEQ ID NO: 24; and the HCDR3 comprises the amino acid sequence as shown in SEQ ID NO: 29; or
the LCDR1 comprises the amino acid sequence as shown in SEQ ID NO: 10; the LCDR2 comprises the amino acid sequence as shown in SEQ ID NO: 15; the LCDR3 comprises the amino acid sequence as shown in SEQ ID NO: 20; the HCDR1 comprises the amino acid sequence as shown in SEQ ID NO: 21; the HCDR2 comprises the amino acid sequence as shown in SEQ ID NO: 23; and the HCDR3 comprises the amino acid sequence as shown in SEQ ID NO: 29;
more preferably, the light chain variable region comprises LCDR1, LCDR2, and LCDR3 as shown in SEQ ID NO: 9, SEQ ID NO: 14, and SEQ ID NO: 19, respectively, and the heavy chain variable region comprises HCDR1, HCDR2, and HCDR3 as shown in SEQ ID NO: 21, SEQ ID NO: 23, and SEQ ID NO: 28, respectively;
the light chain variable region comprises LCDR1, LCDR2, and LCDR3 as shown in SEQ ID NO: 9, SEQ ID NO: 14, and SEQ ID NO: 19, respectively, and the heavy chain variable region comprises HCDR1, HCDR2, and HCDR3 as shown in SEQ ID NO: 21, SEQ ID NO: 24, and SEQ ID NO: 28, respectively;
the light chain variable region comprises LCDR1, LCDR2, and LCDR3 as shown in SEQ ID NO: 7, SEQ ID NO: 11, and SEQ ID NO: 16, respectively, and the heavy chain variable region comprises HCDR1, HCDR2, and HCDR3 as shown in SEQ ID NO: 21, SEQ ID NO: 23, and SEQ ID NO: 25, respectively;
the light chain variable region comprises LCDR1, LCDR2, and LCDR3 as shown in SEQ ID NO: 7, SEQ ID NO: 12, and SEQ ID NO: 17, respectively, and the heavy chain variable region comprises HCDR1, HCDR2, and HCDR3 as shown in SEQ ID NO: 22, SEQ ID NO: 24, and SEQ ID NO: 26, respectively;
the light chain variable region comprises LCDR1, LCDR2, and LCDR3 as shown in SEQ ID NO: 7, SEQ ID NO: 12, and SEQ ID NO: 17, respectively, and the heavy chain variable region comprises HCDR1, HCDR2, and HCDR3 as shown in SEQ ID NO: 22, SEQ ID NO: 23, and SEQ ID NO: 26, respectively;
the light chain variable region comprises LCDR1, LCDR2, and LCDR3 as shown in SEQ ID NO: 8, SEQ ID NO: 13, and SEQ ID NO: 18, respectively, and the heavy chain variable region comprises HCDR1, HCDR2, and HCDR3 as shown in SEQ ID NO: 22, SEQ ID NO: 24, and SEQ ID NO: 27, respectively;
the light chain variable region comprises LCDR1, LCDR2, and LCDR3 as shown in SEQ ID NO: 8, SEQ ID NO: 13, and SEQ ID NO: 18, respectively, and the heavy chain variable region comprises HCDR1, HCDR2, and HCDR3 as shown in SEQ ID NO: 22, SEQ ID NO: 23, and SEQ ID NO: 27, respectively;
the light chain variable region comprises LCDR1, LCDR2, and LCDR3 as shown in SEQ ID NO: 10, SEQ ID NO: 15, and SEQ ID NO: 20, respectively, and the heavy chain variable region comprises HCDR1, HCDR2, and HCDR3 as shown in SEQ ID NO: 21, SEQ ID NO: 24, and SEQ ID NO: 29, respectively; or
the light chain variable region comprises LCDR1, LCDR2, and LCDR3 as shown in SEQ ID NO: 10, SEQ ID NO: 15, and SEQ ID NO: 20, respectively, and the heavy chain variable region comprises HCDR1, HCDR2, and HCDR3 as shown in SEQ ID NO: 21, SEQ ID NO: 23, and SEQ ID NO: 29, respectively.

3. The antibody according to claim 2, wherein the framework region of the light chain variable region is a human framework region, and the framework region of the heavy chain variable region is a human framework region;
preferably, the light chain variable region comprises an amino acid sequence having at least 90%, at least 95%, or at least 99% sequence identity with SEQ ID NO: 38, and the heavy chain variable region comprises an amino acid sequence having at least 90%, at least 95%, or at least 99% sequence identity with SEQ ID NO: 40 or SEQ ID NO: 39;
the light chain variable region comprises an amino acid sequence having at least 90%, at least 95%, or at least 99% sequence identity with SEQ ID NO: 30, and the heavy chain variable region comprises an amino acid sequence having at least 90%, at least 95%, or at least 99% sequence identity with SEQ ID NO: 31;
the light chain variable region comprises an amino acid sequence having at least 90%, at least 95%, or at least 99% sequence identity with SEQ ID NO: 32, and the heavy chain variable region comprises an amino acid sequence having at least 90%, at least 95%, or at least 99% sequence identity with SEQ ID NO: 33 or SEQ ID NO: 34;
the light chain variable region comprises an amino acid sequence having at least 90%, at least 95%, or at least 99% sequence identity with SEQ ID NO: 35, and the heavy chain variable region comprises an amino acid sequence having at least 90%, at least 95%, or at least 99% sequence identity with SEQ ID NO: 36 or SEQ ID NO: 37;
or,
the light chain variable region comprises an amino acid sequence having at least 90%, at least 95%, or at least 99% sequence identity with SEQ ID NO: 41, and the heavy chain variable region comprises an amino acid sequence having at least 90%, at least 95%, or at least 99% sequence identity with SEQ ID NO: 42 or SEQ ID NO: 43;
more preferably, the light chain variable region comprises an amino acid sequence as shown in SEQ ID NO: 38, and the heavy chain variable region comprises an amino acid sequence as shown in SEQ ID NO: 40 or SEQ ID NO: 39;
the light chain variable region comprises an amino acid sequence as shown in SEQ ID NO: 30, and the heavy chain variable region comprises an amino acid sequence as shown in SEQ ID NO: 31;
the light chain variable region comprises an amino acid sequence as shown in SEQ ID NO: 32, and the heavy chain variable region comprises an amino acid sequence as shown in SEQ ID NO: 33 or SEQ ID NO: 34;
the light chain variable region comprises an amino acid sequence as shown in SEQ ID NO: 35, and the heavy chain variable region comprises an amino acid sequence as shown in SEQ ID NO: 36 or SEQ ID NO: 37; or
the light chain variable region comprises an amino acid sequence as shown in SEQ ID NO: 41, and the heavy chain variable region comprises an amino acid sequence as shown in SEQ ID NO: 42 or SEQ ID NO: 43;
further preferably, the antibody comprises a light chain variable region as shown in SEQ ID NO: 38 and a heavy chain variable region as shown in SEQ ID NO: 40;
preferably, a variable region comprising the amino acid sequence having at least 90%, at least 95%, or at least 99% sequence identity with an original sequence maintains an antigen-binding function that is identical to the original sequence.

4. The antibody according to claim 3, wherein the antibody satisfies one or more of three criteria as follows:
(1) the antibody is a full-length antibody, Fab, Fab', F(ab')₂, or Fv, and the Fv is preferably scFv;
(2) the antibody is a monospecific antibody or a multispecific antibody;
(3) the antibody is a monoclonal antibody or a polyclonal antibody derived from the above antibodies.

5. The antibody according to claim 4, wherein the antibody comprises a heavy chain constant region and/or a light chain constant region;
preferably, the heavy chain constant region of the antibody is derived from heavy chain constant region of human antibody IgG1, IgG2, IgG3, or IgG4, and/or the light chain constant region of the antibody is derived from κ chain of a human antibody;
more preferably, the heavy chain constant region comprises an amino acid sequence as shown in SEQ ID NO: 45, and the light chain constant region comprises an amino acid sequence as shown in SEQ ID NO: 44;
most preferably, a heavy chain of the antibody comprises an amino acid sequence as shown in SEQ ID NO: 94, and a light chain of the antibody comprises an amino acid sequence as shown in SEQ ID NO: 95; or, the heavy chain of the antibody comprises an amino acid sequence as shown in SEQ ID NO: 96, and the light chain of the antibody comprises an amino acid sequence as shown in SEQ ID NO: 81; or, the heavy chain of the antibody comprises an amino acid sequence as shown in SEQ ID NO: 87, and the light chain of the antibody comprises an amino acid sequence as shown in SEQ ID NO: 81.

6. A bispecific antibody comprising a first antigen-binding domain that specifically binds to human CD40 and a second antigen-binding domain that specifically binds to human PD-L1, wherein the first antigen-binding domain is as defined in the anti-CD40 antibody according to any one of claims 1 to 5.

7. The bispecific antibody according to claim 6, wherein the second antigen-binding domain comprises at least one VHH, and the VHH comprises VHH-CDR1, VHH-CDR2, and VHH-CDR3 of SEQ ID NO: 66;
preferably, the VHH-CDR1 comprises an amino acid sequence as shown in X₄₁YYX₄₂X₄₃C, wherein X₄₁ is D or E, X₄₂ is S or T, and X₄₃ is K or Q; the VHH-CDR2 comprises an amino acid sequence as shown in SEQ ID NO: 76; and the VHH-CDR3 comprises an amino acid sequence as shown in SEQ ID NO: 77;
more preferably, the VHH comprises VHH-CDR1, VHH-CDR2, and VHH-CDR3 of SEQ ID NO: 49, SEQ ID NO: 74, SEQ ID NO: 72, or SEQ ID NO: 73;
further preferably, the VHH-CDR1 comprises an amino acid sequence as shown in SEQ ID NO: 46, SEQ ID NO: 67, or SEQ ID NO: 70; the VHH-CDR2 comprises an amino acid sequence as shown in SEQ ID NO: 47, SEQ ID NO: 68, or SEQ ID NO: 71; and the VHH-CDR3 comprises an amino acid sequence as shown in SEQ ID NO: 48 or SEQ ID NO: 69.

8. The bispecific antibody according to claim 7, wherein the VHH-CDR1 comprises the amino acid sequence as shown in SEQ ID NO: 46, the VHH-CDR2 comprises the amino acid sequence as shown in SEQ ID NO: 47, and the VHH-CDR3 comprises the amino acid sequence as shown in SEQ ID NO: 48;
or, the VHH-CDR1 comprises the amino acid sequence as shown in SEQ ID NO: 67, the VHH-CDR2 comprises the amino acid sequence as shown in SEQ ID NO: 68, and the VHH-CDR3 comprises the amino acid sequence as shown in SEQ ID NO: 69;
or, the VHH-CDR1 comprises the amino acid sequence as shown in SEQ ID NO: 70, the VHH-CDR2 comprises the amino acid sequence as shown in SEQ ID NO: 71, and the VHH-CDR3 comprises the amino acid sequence as shown in SEQ ID NO: 48;
preferably, the VHH comprises:
VHH-CDR1 with the sequence as shown in SEQ ID NO: 46, VHH-CDR2 with the sequence as shown in SEQ ID NO: 47, and VHH-CDR3 with the sequence as shown in SEQ ID NO: 48,
VHH-CDR1 with the sequence as shown in SEQ ID NO: 67, VHH-CDR2 with the sequence as shown in SEQ ID NO: 68, and VHH-CDR3 with the sequence as shown in SEQ ID NO: 69,
or, VHH-CDR1 with the sequence as shown in SEQ ID NO: 70, VHH-CDR2 with the sequence as shown in SEQ ID NO: 71, and VHH-CDR3 with the sequence as shown in SEQ ID NO: 48;
further preferably, the amino acid sequence of the VHH is as shown in SEQ ID NO: 49, SEQ ID NO: 72, SEQ ID NO: 73, or SEQ ID NO: 74, or has at least 90% sequence identity with SEQ ID NO: 49, SEQ ID NO: 72, SEQ ID NO: 73, or SEQ ID NO: 74.

9. A bispecific antibody comprising a first antigen-binding domain that specifically binds to human CD40 and a second antigen-binding domain that specifically binds to human PD-L1, wherein the second antigen-binding domain comprises at least one VHH, and the VHH has a sequence as defined in the bispecific antibody according to claim 7 or 8; preferably, the first antigen-binding domain is as defined in the anti-CD40 antibody according to any one of claims 1 to 5.

10. The bispecific antibody according to any one of claims 6 to 9, wherein the first antigen-binding domain is operably linked to the second antigen-binding domain directly or via a linker;
preferably, the second antigen-binding domain is linked to N-terminus of a light chain variable region or heavy chain variable region of the first antigen-binding domain, or to C-terminus of the light chain constant region, or to C-terminus of the IgG; the linker is preferably a peptide sequence, more preferably comprising or consisting of (G₄S)ₙG, wherein n is an integer of 1 to 10, for example, n is 3.

11. The bispecific antibody according to any one of claims 6 to 10, which comprises two first polypeptide chains and two second polypeptide, wherein
the amino acid sequence of the first polypeptide chain is as shown in SEQ ID NO: 56 or has at least 90% sequence identity with SEQ ID NO: 56; and/or, the amino acid sequence of the second polypeptide chain is as shown in SEQ ID NO: 62 or SEQ ID NO: 57 or has at least 90% sequence identity with SEQ ID NO: 62 or SEQ ID NO: 57;
the amino acid sequence of the first polypeptide chain is as shown in SEQ ID NO: 50 or has at least 90% sequence identity with SEQ ID NO: 50; and/or, the amino acid sequence of the second polypeptide chain is as shown in SEQ ID NO: 51 or has at least 90% sequence identity with SEQ ID NO: 51;
the amino acid sequence of the first polypeptide chain is as shown in SEQ ID NO: 52 or has at least 90% sequence identity with SEQ ID NO: 52; and/or, the amino acid sequence of the second polypeptide chain is as shown in SEQ ID NO: 53 or SEQ ID NO: 60 or has at least 90% sequence identity with SEQ ID NO: 53 or SEQ ID NO: 60;
the amino acid sequence of the first polypeptide chain is as shown in SEQ ID NO: 54 or has at least 90% sequence identity with SEQ ID NO: 54; and/or, the amino acid sequence of the second polypeptide chain is as shown in SEQ ID NO: 55 or SEQ ID NO: 61 or has at least 90% sequence identity with SEQ ID NO: 55 or SEQ ID NO: 61; or
the amino acid sequence of the first polypeptide chain is as shown in SEQ ID NO: 58 or has at least 90% sequence identity with SEQ ID NO: 58; and/or, the amino acid sequence of the second polypeptide chain is as shown in SEQ ID NO: 59 or SEQ ID NO: 63 or has at least 90% sequence identity with SEQ ID NO: 59 or SEQ ID NO: 63.

12. An isolated nucleic acid encoding the anti-CD40 antibody according to any one of claims 1 to 5 or the bispecific antibody according to any one of claims 6 to 11.

13. A recombinant expression vector comprising the isolated nucleic acid according to claim 12;
preferably, the recombinant expression vector is a plasmid, cosmid, phage, or viral vector;
more preferably, the plasmid has a backbone of pcDNA3.4.

14. A transformant comprising the recombinant expression vector according to claim 13; preferably, the transformant has a host cell that is either a prokaryotic cell or a eukaryotic cell; more preferably, the eukaryotic cell is a yeast cell or a mammalian cell; wherein the mammalian cell is, for example, an EXPI-293 cell or a CHO cell.

15. A method for preparing an anti-CD40 antibody or a bispecific antibody, comprising steps of:
culturing the transformant according to claim 14, and
obtaining an anti-CD40 antibody or a bispecific antibody from the culture.

16. A pharmaceutical composition comprising the anti-CD40 antibody according to any one of claims 1 to 5 or the bispecific antibody according to any one of claims 6 to 11, and a pharmaceutically acceptable carrier;
preferably, the pharmaceutical composition further comprises an other agent; the other agent is selected from one or more of the group consisting of a hormone preparation, a targeted small molecule preparation, a proteasome inhibitor, an imaging agent, a diagnostic agent, a chemotherapeutic agent, an oncolytic drug, a cytotoxic agent, a cytokine, an activator of co-stimulatory molecules, an inhibitor of inhibitory molecules, and a vaccine.

17. A use of the anti-CD40 antibody according to any one of claims 1 to 5, the bispecific antibody according to any one of claims 6 to 11, and/or the pharmaceutical composition according to claim 16 in the manufacture of a medicament for preventing and/or treating a tumor; preferably, the tumor is lymphoma, breast cancer, ovarian cancer, prostate cancer, pancreatic cancer, kidney cancer, lung cancer, liver cancer, gastric cancer, colorectal cancer, bladder cancer, rhabdomyosarcoma, esophageal cancer, cervical cancer, multiple myeloma, leukemia, gallbladder cancer, glioblastoma, or melanoma.

18. A kit comprising the anti-CD40 antibody according to any one of claims 1 to 5, the bispecific antibody according to any one of claims 6 to 11, or the pharmaceutical composition according to claim 16;
preferably, the kit further comprises (i) a device for administering the antibody or pharmaceutical composition; and/or (ii) instructions for use.

19. A drug box kit comprising a drug box A and a drug box B, wherein
the drug box A comprises the anti-CD40 antibody according to any one of claims 1 to 5, the bispecific antibody according to any one of claims 6 to 11, and/or the pharmaceutical composition according to claim 16;
the drug box B comprises an other anti-tumor antibody or a pharmaceutical composition comprising the other anti-tumor antibody, and/or one or more of the group consisting of a hormone preparation, a targeted small molecule preparation, a proteasome inhibitor, an imaging agent, a diagnostic agent, a chemotherapeutic agent, an oncolytic drug, a cytotoxic agent, a cytokine, an activator of co-stimulatory molecules, an inhibitor of inhibitory molecules, and a vaccine.

20. An immunoassay, or a method for determining CD40 and/or PD-L1, comprising using the anti-CD40 antibody according to any one of claims 1 to 5, the bispecific antibody according to any one of claims 6 to 11, and/or the pharmaceutical composition according to claim 16; preferably, the immunoassay is an assay for non-diagnostic purposes.

21. A method for preventing and/or treating a tumor, comprising administering to a patient in need thereof a therapeutically effective amount of the anti-CD40 antibody according to any one of claims 1 to 5, the bispecific antibody according to any one of claims 6 to 11, and/or the pharmaceutical composition according to claim 16, or the drug box kit according to claim 19; preferably, the tumor is a PD-L1 positive and/or CD40 positive tumor.

22. A combination therapy comprising administering to a patient in need thereof the anti-CD40 antibody according to any one of claims 1 to 5, the bispecific antibody according to any one of claims 6 to 11, and/or the pharmaceutical composition according to claim 16, and a second therapeutic agent, respectively;
the second therapeutic agent preferably comprises an other anti-tumor antibody or a pharmaceutical composition comprising the other anti-tumor antibody, and/or one or more of the group consisting of a hormone preparation, a targeted small molecule preparation, a proteasome inhibitor, an imaging agent, a diagnostic agent, a chemotherapeutic agent, an oncolytic drug, a cytotoxic agent, a cytokine, an activator of co-stimulatory molecules, an inhibitor of inhibitory molecules, and a vaccine.

23. A use of the anti-CD40 antibody according to any one of claims 1 to 5, the bispecific antibody according to any one of claims 6 to 11, and/or the pharmaceutical composition according to claim 16 as a medicament; preferably, the medicament is used to prevent and/or treat a tumor; more preferably, the tumor is lymphoma, breast cancer, ovarian cancer, prostate cancer, pancreatic cancer, kidney cancer, lung cancer, liver cancer, gastric cancer, colorectal cancer, bladder cancer, rhabdomyosarcoma, esophageal cancer, cervical cancer, multiple myeloma, leukemia, gallbladder cancer, glioblastoma, or melanoma.
